# EUROPEAN PATENT SPECIFICATION

(11) **EP 2 174 908 B1**
(45) Date of publication and mention of the grant of the patent: **30.09.2015**
(21) Application number: 09179284.6
(22) Date of filing: 23.12.2005
(51) Int. Cl.: B81C 1/00, G01N 33/68

(54) **Device and use thereof**
Vorrichtung und Verwendung davon
Dispositif et son utilisation

(30) Priority: 23.12.2004 SE 0403139
(43) Date of publication of application: 14.04.2010
(62) Divisional of application: 05819755.9
(73) Proprietor: Nanoxis Consulting AB, 400 16 Göteborg (SE)
(72) Inventor: Karlsson, Anders, 517 36 Bollebygd (SE); Karlsson, Roger, 43146 Möldal (SE); Pihl, Johan, 42470 Olofstorp (SE); Karlsson, Mattias, 43994 Onsala (SE); Orwar, Owe, 43651 Hovås (SE); Lobovkina, Tatiana, 411 39 Göteborg (SE); Jesorka, Aldo, 411 29 Göteborg (SE); Davidson, Max, 414 66 Göteborg (SE)
(74) Representative: Brann AB

(56) References cited:
- US-A1- 2003 013 138
- US-A1- 2003 044 843
- REDEBY T ET AL: "Simple fabrication of a structured matrix-assisted laser desorption/ionization target coating for increased sensitivity in mass spectrometric analysis of membrane proteins" RAPID COMMUNICATIONS IN MASS SPECTROMETRY WILEY UK, vol. 18, no. 10, 23 April 2004 (2004-04-23), pages 1161-1166, XP002574075 ISSN: 0951-4198

## Description

### Field of the invention

The present invention relates methods for identification of a substance using lipid bilayer devices comprising planar and/or topographic substrates. Biological materials and chemicals can be introduced into the lipid bilayer devices for a variety of purposes, e.g. for fractionation and/or analysis by e.g. MALDI-TOF mass spectrometry.

### Background of the invention

Analytical devices for separation and fractionation of biomolecules are often based on chromatography or gel electrophoresis. These rely on the use of separation media and a mobile medium that is chosen in such a way that analytes of interest differentially partition between the phases (because they have different molecular structure) to obtain differential migrational velocities. Thus, depending on the physico-chemical constitution of the analytes (i.e. charge, hydrophobicity, size etc), different analytical platforms might be used. Certain classes of molecules are still after more than hundred years of the analytical sciences difficult to separate, quantitate, and analyze. Most notably, membrane-associated proteins such as ion channels, GPCR's (G-protein coupled receptors), and other receptors belong to this class. These represent some of the most important targets in drug development and play a crucial role in biological systems such as the heart, gastrointestinal tract and brain. Novel devices that would allow their characterization would be of great value.

Membrane proteins in their native form are embedded in lipid bilayer membranes. The complex composite structure defines overall protein function. The lipid bilayer membrane is thus not simply a matrix but an essential part of a functional lipid-protein aggregate. We have previously developed methods for making complex lipid-bilayer membrane devices. These rely on the unique liquid crystalline properties of bilayer membranes and their mechanical properties. We have shown that they are useful for a number of applications such as membrane protein expression, nanofluidics, and reaction devices. The main limitation of the technology has been its manual, and labor-intensive manufacturing, as well as relatively small scale (Microscopic Networks of Containers and Nanotubes, WO 02/26616 or PCT/SE01/02116).

Redeby et al., Rapid Commun Mass Spectrom. 2004;18(10):1161-6., disclose the development of a prestructured target plate for matrix-assisted laser desorption/ionization (MALDI) specifically for hydrophobic integral membrane proteins, containing predefined concentrating sample spots with a focusing effect on droplets with a high content of hexafluoroisopropanol (HFIP).

US2003/013138 discloses systems and methods for the analysis of proteins, e.g. for identifying and characterizing surface membrane proteins. It also discloses methods and systems for arraying and analyzing proteins.

US2003/044843 discloses a proteome analysis method including grouping a proteome into membrane proteins and compounds capable of interacting with the membrane proteins, while retaining their native structure and function, and analyzing both the membrane proteins and the compounds based on biological affinity, and devices therefor.

### Summary of the invention

The present invention relates to a method for identification of a substance, which substance is included in or bound to, connected to or associated with a covering layer in a device, said device comprising:
at least one supporting solid surface comprising at least one membraneophilic region;
a covering layer that is at least partially immobilized to the membraneophilic region, said covering layer consisting of (i) a surfactant membrane, (ii) a lipid mimicking polymer, (iii) a surfactant or emulsion system, (iv) a liquid crystal; and
a substance included in or bound to, connected to or associated with the covering layer,
said method comprising a first step (i) wherein the device is contacted with at least one digestive agent, which carry out controlled digestive cleaving of parts the substance, after which the substance consists of a substantially mobile fraction and an immobile fraction, which is still included in or bound to, connected to or associated with the covering layer;, and a second step (ii) wherein the substantially mobile fraction is transported to be detected. This may be repeated one or several times with different digestive compounds. This method may also comprise a third step (iii) wherein the device is contacted with at least one disruptive agent, which disrupt the structure of the covering layer carrying the remaining immobile fraction of the substance included in or bound to, connected to or associated with the covering layer, after which it is comprised of its basic elements, which are substantially mobile; and a fourth step (iv) wherein the remaining fraction of the substance included in or bound to, connected to or associated with the covering layer, which is released from the covering layer, is transported to be detected or analyzed. This method is further disclosed below and in the appended claims.

In this context the expression membraneophilic surface is intended to encompass all surfaces that membranes are attracted to, which also might be explained as all surfaces that give rise to attractive forces between the surface and a membrane. The membrane may for example be selected from the group consisting of lipid bilayer membranes, lipid vesicles, proteovesicles, lipid nanotubes, lipid monolayers, cell fragments, organelles and any combinations thereof etc, as further discussed below.

While the present invention only relates to methods for identification of a substance, the configuration of devices suitable for use in the inventive methods are further disclosed below.

Preferably the device comprises at least two supporting solid surfaces.

Each supporting solid surface may comprise one, two or several membraneophilic regions. Each membraneophilic region may be covered by similar or different covering layers.

To the covering layers one or more substances are associated. Preferably, these substances are bound or connected to the covering layers via lipid moieties in the covering layer e.g. a bilayer surfactant membrane with membrane proteins or a membrane selected from the group consisting of lipid bilayer membranes, lipid vesicles, proteovesicles, lipid nanotubes, lipid monolayers, cell fragments, organelles and any combinations thereof etc.

By the expression membraneophilic region is intended a region on the surface or a part of the surface that associates to membranes. It may thus also be called a membrane associating region or part of the surface.

The covering layer, preferably a surfactant membrane, and the substances associated therewith have specific characteristics, which enable chemical or physical modulation or manipulation, with the aim of, for example, chemical manipulation, fractionation, separation or identification of, for example, the substances connected to or associated with lipid moieties in the covering layer. The physical or chemical modulation or manipulation may consist of at least one of the following actions: cleaving of parts of the membrane, cleaving of parts of the substance connected to or associated with lipid moieties, disruption of the membrane.

In addition to the membraneophilic region(s) on the supporting solid surface, the device may also comprise a further membraneophilic region which is constituted by solid particles in an aqueous solution. The solid particles have membraneophilic properties and are covered by (a) a membrane, (b) a lipid mimicking polymer or (c) a surfactant or emulsion system. The solid particles are immobilized.

The device may further comprise an aqueous layer trapped between the supporting solid surface and the covering layer.

The device may also comprise an aqueous solution covering the covering layer.

Furthermore, the device may comprise a layer formed of gel or polymer structure. This layer may be used in order to, e.g., introduce pH gradients or to provide a second stationary matrix for attachment of molecules and functional groups, such as e.g. antibodies. This layer may be placed over and/or under the covering layer.

The device may further comprise a metal film on the supporting solid surface. This metal film is preferably placed directly on the supporting solid surface. The purpose of the metal film is to reduce background fluorescence or auto-fluorescence during fluorescence measurements.

The supporting solid surface of the device may also comprise at least one membraneophobic region. By membraneophobic region is intended a membrane-repelling region or part of the surface.

The membraneophilic region used in the method according to the invention may be constituted or formed of any membraneophilic surface of any shape or geometry or surface made membraneophilic through chemical and/or physical modulation or manipulation.

The chemical modulation or manipulation may for example be performed by treatment with e.g. acids or bases or oxidizers or by other suitable methods.

The physical modulation or manipulation may for example be performed by plasma-treatment or other suitable methods.

The above mentioned membraneophilic region may be formed of a surface consisting of a substance selected from the group consisting of silicon dioxide (SiO₂), glass, Mica or a polymer-modified surface, made membraneophilic through chemical and/or physical modulation or manipulation. Preferably the surface has been treated by a plasma-treatment. This enables a lipid bilayer membrane to adhere to said membraneophilic region.

The above mentioned membraneophilic region may also be formed of a surface consisting of a substance selected from the group consisting of metal oxides and metals, such as, for example, a substance selected from the group consisting of aluminum oxide (Al₂O₃), another metal oxide or gold, made membraneophilic through chemical and/or physical modulation or manipulation. Preferably the surface has been treated with a plasma. This enables intact lipid vesicles to adhere and stay immobilized to said membraneophilic region.

The covering layer of the device may be constituted by a membrane. The membrane may be selected from the group consisting of lipid bilayer membranes, lipid vesicles, proteovesicles - i.e. vesicles containing reconstituted membrane proteins, lipid nanotubes, lipid monolayers - such as self assembled monolayers, SAMs, cell fragments, organelles and any combinations thereof. The membrane can be formed as a network of at least one lipid nanotube connected to at least one container of lipid membrane, in controllable fashion.

One membraneophilic region may be covered with a covering layer having different parts. The membraneophilic region may be covered with different lipids or lipid/lipid mixtures, wherein the lipids or the lipid/lipid mixture on a certain part of the membraneophilic region can be one type of lipid or lipid/lipid mixture, while the lipids or lipid/lipid mixture on a certain different part of the membraneophilic region can be of a different type of lipid or lipid/lipid mixture. The lipid/lipid mixtures can contain different types of lipids or lipids in different phase states coexisting on the same surface. When the membrane is a lipid bilayer membrane it may have been introduced onto the membraneophilic surface by deposition of multilamellar vesicles using micropipettes or optical tweezers, by deposition of lipids using a pipette-writing technique by translation of a pipette filled with lipid material across a surface, by deposition of lipids using fusion of small vesicles, by deposition of lipid bilayer membranes through fluidic channels and flow cells or by direct injection of dissolved lipids. The adsorption of the lipid bilayer membrane to the supporting solid surface can be controlled by various means, e.g., by the adhesion potential of the supporting solid surface. Said adhesion potential may be static and controlled by any of the following interactions: Van der Waals, hydrophobic, electrostatic, pi-pi interactions, hydrogen bonding and covalent interactions. Alternatively, said adhesion potential is dynamic and controlled by any of the following interactions: electrowetting and electrical forces. Magnetic forces might also be used for this purpose.

When the covering layer is constituted by a membrane selected from the group consisting of lipid bilayer membranes, lipid vesicles, proteovesicles, lipid nanotubes, lipid monolayers, cell fragments, organelles and any combinations thereof the substance included in or bound to, connected to or associated with the covering layer is bound to, connected to or associated with lipid moieties in or on the covering layer.

Especially when the covering layer is constituted by a membrane selected from the group consisting of lipid bilayer membranes, lipid vesicles, proteovesicles, lipid nanotubes, lipid monolayers, cell fragments, organelles and any combinations thereof divalent cations may be used to enhance the immobilization of the covering layer to the supporting solid surface. One example of such divalent cations is calcium ions.

The covering layer of the device used in methods according to the invention may also be constituted by intact cells or intact cell organelles.

Furthermore, the covering layer may be constituted by a lipid mimicking polymer. One example of such a polymer is a di-block-polymer.

Moreover the covering layer may be constituted by a surfactant or emulsion system. The surfactant or emulsion system is selected from the group consisting of micelle forming systems, oils, sponge-phases and lyotropic lipids, such as extracts from natural sources or synthetic lipids.

The substance included in or bound to, connected to or associated with the covering layer may be selected from the group consisting of peripheral and integral membrane proteins, glycosylphosphatidylinositol(GPI)-anchored proteins, phospholipids, sphingolipids, drugs, amphiphilic agents, lipophilic agents, sterols, sugars, oligonucleotides and polymers. The substance may be any molecule that can be linked to the covering layer by non-covalent interactions, such as the biotin-avidin interactions.

The substance may further be connected to beads. The beads can have different properties, for example magnetic beads may be used in connection with magnetic fields, charged beads may be used in connection with electric fields and large or bulky beads may be used for hydrodynamic flows. The use of magnetic beads enables separation based on magnetic fields, i.e. magneto-phoresis.

The substance may also be connected to a transport marker. The substance may be introduced into the covering layer or the substance forming the covering layer by a technique selected from the group consisting of electroinjection, electrofusion, optical fusion, fusion induced by heat, fusion induced by electromagnetic radiation, fusion induced by chemical agents and detergent destabilization. This may be done during the formation of the covering layer.

Furthermore, the device used in methods according to the invention may also comprise at least one electrode for application of an electric field over the covering layer. This enables electrophoresis, electroendoosmosis, dielectrophoresis, isotachophoresis or isoelectric focusing.

The device used in methods according to the invention may also comprise at least one magnet for application of a magnetic field over the covering layer.

The device used in methods according to the invention may also comprise at least one inlet and at least one outlet connection for creation of a flow of the aqueous solution covering the covering layer.

Moreover, the device used in methods according to the invention may also comprise at least one inlet and at least one outlet connection for the exchange of additional membrane, protein-lipid mixtures, washing solutions, staining solutions, digestive solutions and other solutions or suspensions around the covering layer.

Furthermore, the device used in methods according to the invention may comprise parts for collection of fractionated samples or specialized domains for performing sample concentration and isolation.

The device used in methods according to the invention may also comprise at least one fluidic channel for transport of material to and from the device.

The construction of a device for use in methods according to the invention comprising at least one electrode, at least one magnet and/or at least one inlet and at least one outlet connection for creation of a flow of fluid as well as at least one inlet and at least one outlet connection for the exchange of additional membrane, protein-lipid mixtures, washing solutions, staining solutions, digestive solutions and other solutions or suspensions around the covering layer and optionally also parts for collection of fractionated samples or specialized domains for performing sample, concentration and isolation and/or at least one fluidic channel for transport of material to and from the device enables the adaptation of the device for separation, isolation and/or concentration of the substance included in or bound to, connected to or associated with the covering layer. The substance included in or bound to, connected to or associated with the covering layer is then selected dependent on its specific characteristics, which should enable chemical or physical modulation or manipulation.

Moreover, the device may comprise molecules, such as tethers, for anchoring the covering layer to the supporting solid surface. These molecules function as a link or a spacer between the covering layer and the supporting solid surface. They may be part-of either the covering layer or the supporting solid surface.

The supporting solid surface of the device may be planar and the membraneophilic surface region then has a specific two-dimensional geometric shape, e.g. consisting of islands, lanes, serpentine shapes.

The supporting solid surface of the device may be of a three-dimensional structure. The membraneophilic regions may then be placed in a recessed structure, e.g. placed in a well or channel. The membraneophilic regions may also be placed on the whole or on one or several parts, such as on the top only, of an elevated structure, e.g. pillars, lanes, pyramid-shapes, step-pyramid-shapes, serpentine shapes, meander-boarder shapes. When the supporting solid surface is three-dimensional, the covering layer may be either two- or three-dimensional. It is often three-dimensional, but sometimes it may for example be corrugated in order to increase the surface, and it is then of course three-dimensional.

The membraneophobic region may be formed of a photoresistant substance, such as SU-8, Teflon, plastics, rubbers, polymers and lipid membranes as non-limiting examples. It may also be formed of a plastic, a polymer or a lipid membrane.

The supporting solid surface may be patterned with two different surface regions, one membraneophilic and one membraneophobic.

The supporting solid surface may also comprise more than one membraneophilic and more than one membraneophobic region, said regions being arranged in a multiplexed or parallel pattern. The pattern may comprise geometrical structures of identical membraneophilic or membraneophobic surface regions. The pattern may also comprise geometrical structures of different membraneophilic or membraneophobic surface regions.

Finally, the aqueous layer of the device used in methods according to the invention may comprise polymers. These may then function as so called polymer cushions.

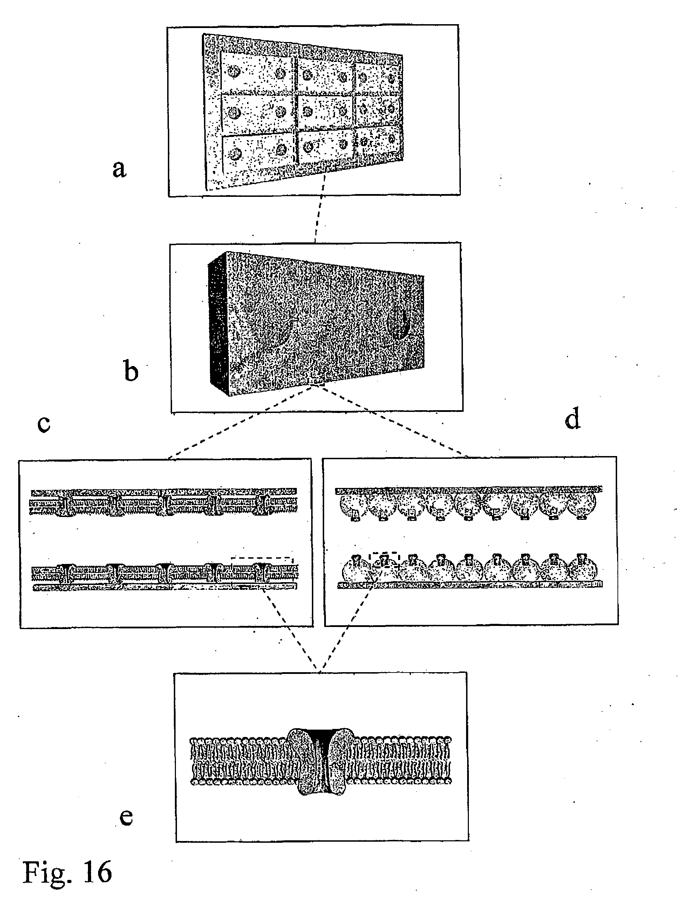

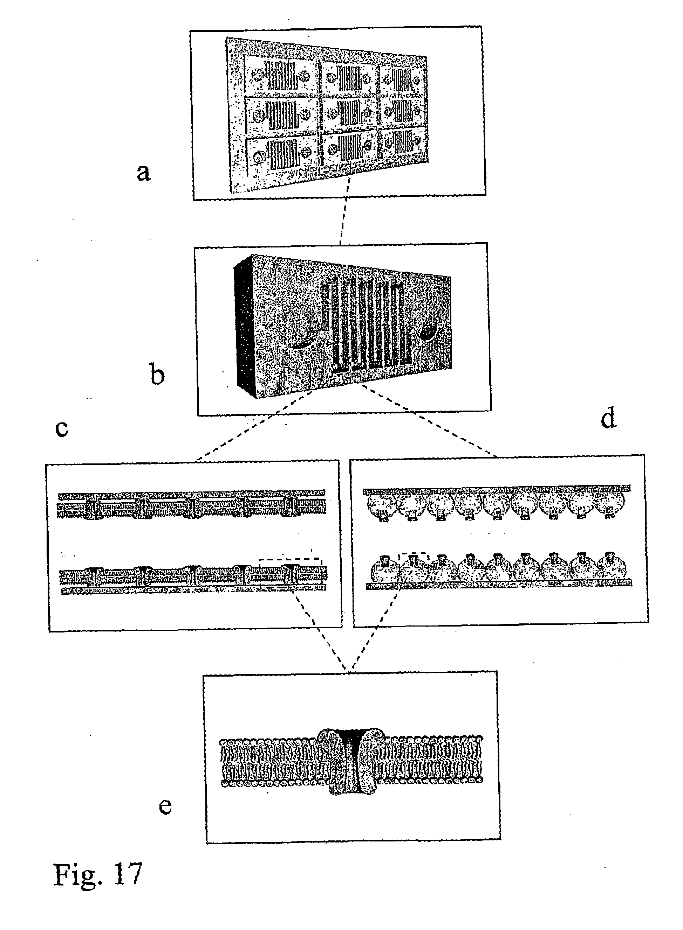

Preferred embodiments of the device used in methods according to the invention are shown in figures 16 and 17, and they are also discussed in more detail below.

The methods according to the invention are i.a. suitable for the following:
for the separation of a substance which is or may be included in or bound to, connected to or associated with a covering layer in a device as disclosed herein,
for the collection of a substance which is or may be included in or bound to, connected to or associated with a covering layer in a device as disclosed herein,
for the identification of a substance which is or may be included in or bound to, connected to or associated with a covering layer in a device as disclosed herein,
for integration into a separation column, which performs separation of e.g. peptides, sugars, lipids or DNA,
for integration into a detection or analysis system,
for coupling to desalting and concentration columns,
for studies of binding interactions,
for studies of diffusion,
for studies of chemical reactions,
for studies of protein-drug interactions,
in the making of chemical computers,
in the making of implantable devices,
in conjunction with so called. Marangoni-flows and other membrane properties, for growing living cells on the membraneophilic region of a device as disclosed herein,
in conjunction with antibodies, for example, being attached to the membraneophilic or membraneophobic region(s) of a device as disclosed herein or being attached to beads having diameters of nanometers to micrometers, and/or for focusing or concentration of macromolecules or for formation of macromolecular aggregates.

In the case methods for analysis and studies of membrane proteins, the following applications, among others, are possible:
- The identification and characterization of a protein, including so called sequence coverage studies, which involves optimized protocols to detect and identify as many peptides as possible in the protein sequence, corresponding to as much as possible of the amino acid sequence. Studies of post-translational modifications are also applicable in this context.
- Mapping of membrane proteins from different cell samples - membrane proteome profiling. Highly complex samples with a wide range of abundance levels are digested and analyzed. Prior to MS analysis the created peptides are separated in one (for example, reversed phase HPLC) or two dimensions (for example, ion exchange HPLC (SCX - strong cation exchange) followed by , for example, reversed phase HPLC). This also enables the studies of low abundance proteins and the discovery of new drug targets.
- Functional studies of membrane proteins - examples are target fishing and receptor deorphanization, e.g. G-protein coupled receptor (GPCR) deorphanization. In the case of target fishing, the purpose is to use ligand binding studies to identify which ligands binds to which proteins. In the case of receptor deorphanization, the purpose is to elucidate the function of receptors and their possible ligands. There are many receptors which function is unknown, hence the name orphan receptors.
- investigation of up-and-down regulation of protein expression levels during states of disease and/or medication, so called expression profiling. Such studies involves comparison of different samples and the evaluation of differences between the samples.

The improved data output compared to when using traditional methods, obtained with the method according to the invention is that:
- Multiple chemical reaction and/or wash steps can be implemented in order to ensure optimized protocols on the chip. Such steps are also implemented without dilution of the sample.
- Extramembrane and transmembrane parts of membrane proteins can be targeted and collected in different steps, using the same sample.
- A single sample can be subjected to different enzyme, sequentially and/or in parallel, to digest various parts of the same sample.
- The high surface-to-volume ratio in the chip in conjunction with sequential digestion etc leads to a high concentration of created peptides with minimal sample loss, which enables higher sensitivity. This promotes sequence coverage studies and the detection of low abundance membrane proteins in the sample.
- Different subcellular fractions from cells can be handled in parallel on one chip.
- Ligand binding, crosslinking and/or digestion can potentially give target peptide binding sites. A protein with and without bound ligand will yield different peptide maps after enzymatic digestion, where the ligand either sterically obstructs the enzyme from cleaving or inflicts a structural change upon binding.

Moreover some general advantages of the method according to the invention using a chip device as disclosed herein are the following:
- Sample is confined to a surface, that is, it is held in a stationary matrix during the analysis, thereby enabling multiple steps of labeling, chemical modifications, digestion etc.
- Ability to use in conjunction with an arsenal of detection techniques on-and offchip, including MS, fluorescence, electrochemistry, SPR, QCM, etc.
- Possibility to integrate the chip to existing chip-based platforms and traditional analysis tools.
- Sample handling is easy to automate.

In parallel, the advantages of using a lipid matrix in a device used in a method according to the invention are that:
- Membrane bound components, such as membrane proteins are kept in their natural lipid bilayer environment.
- Structure and function of the membrane bound components are retained.
- Preparation, transportation, deposition and processing of membrane vesicles containing membrane bound components, e.g. proteins, does not require the use of detergents, since the devices disclosed herein enable keeping the membrane proteins in their natural lipid bilayer membrane environment. This is an advantage since detergents may have disadvantageous effects, e.g. denaturing of proteins. It is also possible to use detergents in few or all steps in conjunction with the invention, for example, to obtain a more pure membrane protein preparation prior analysis.

### Description of the drawings

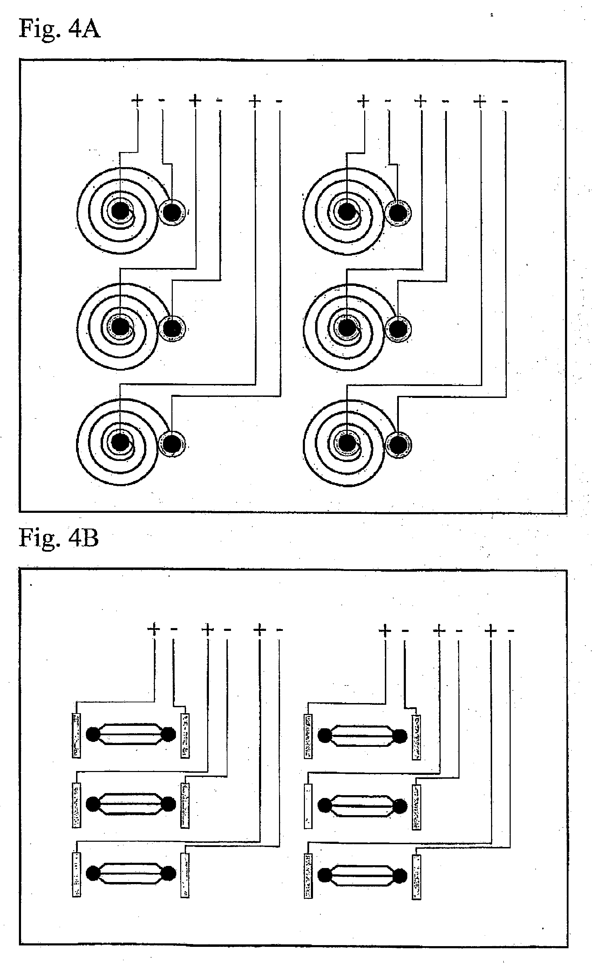

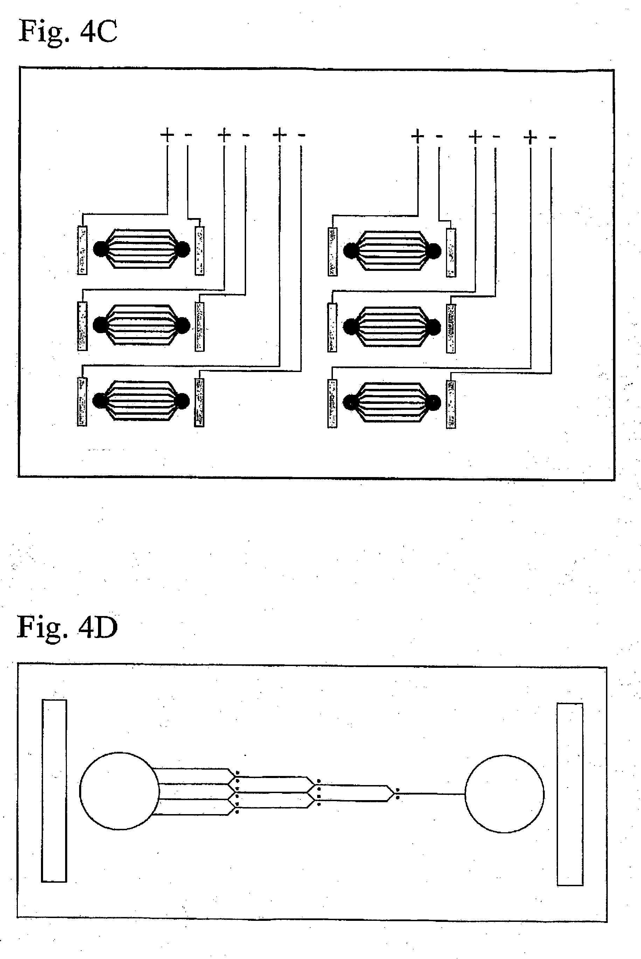

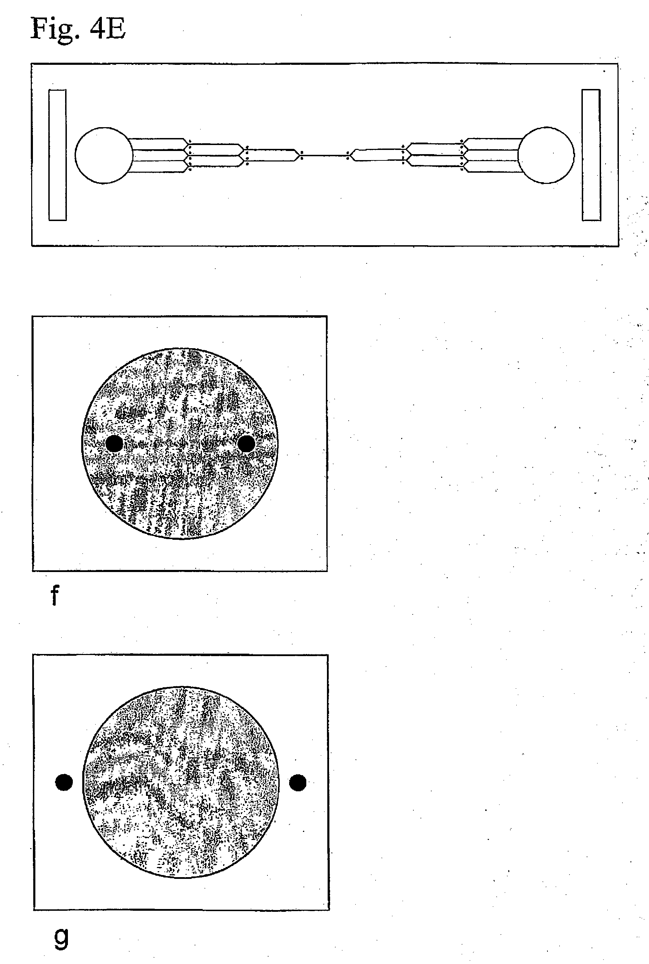

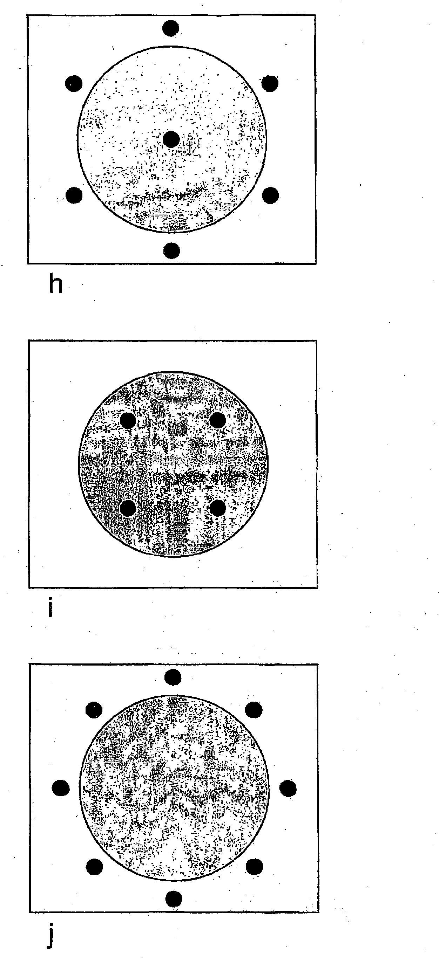

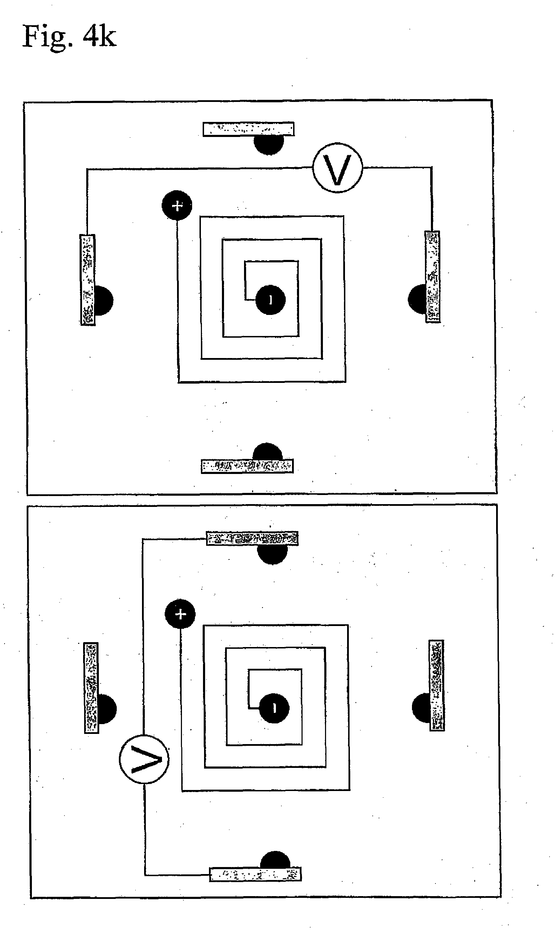

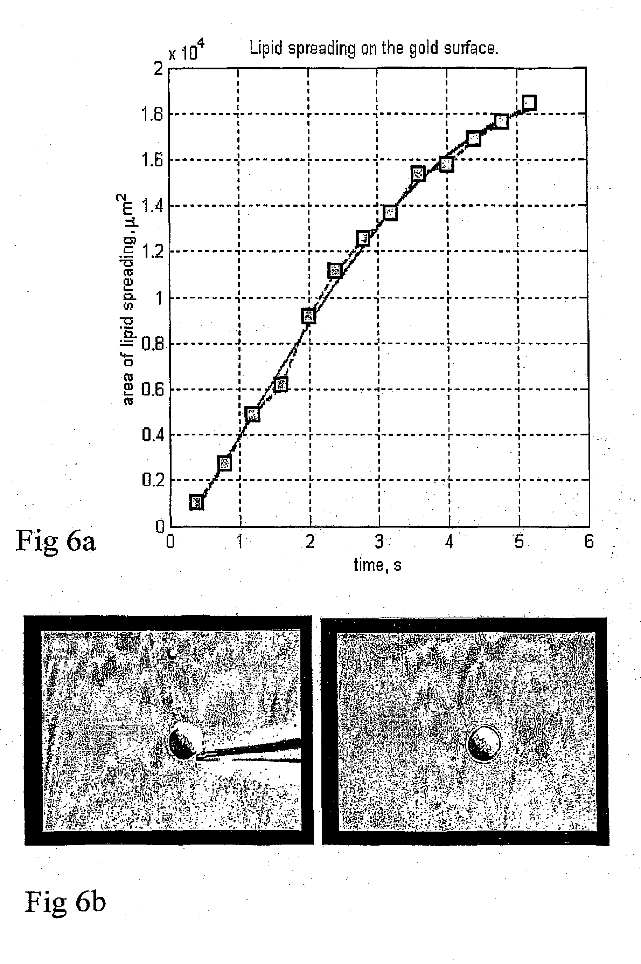

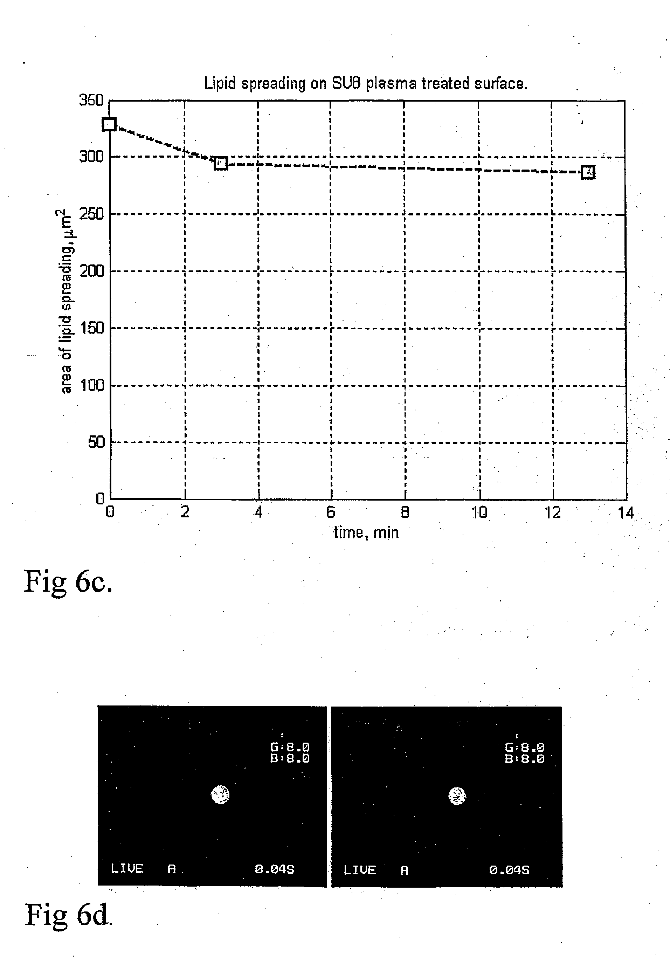

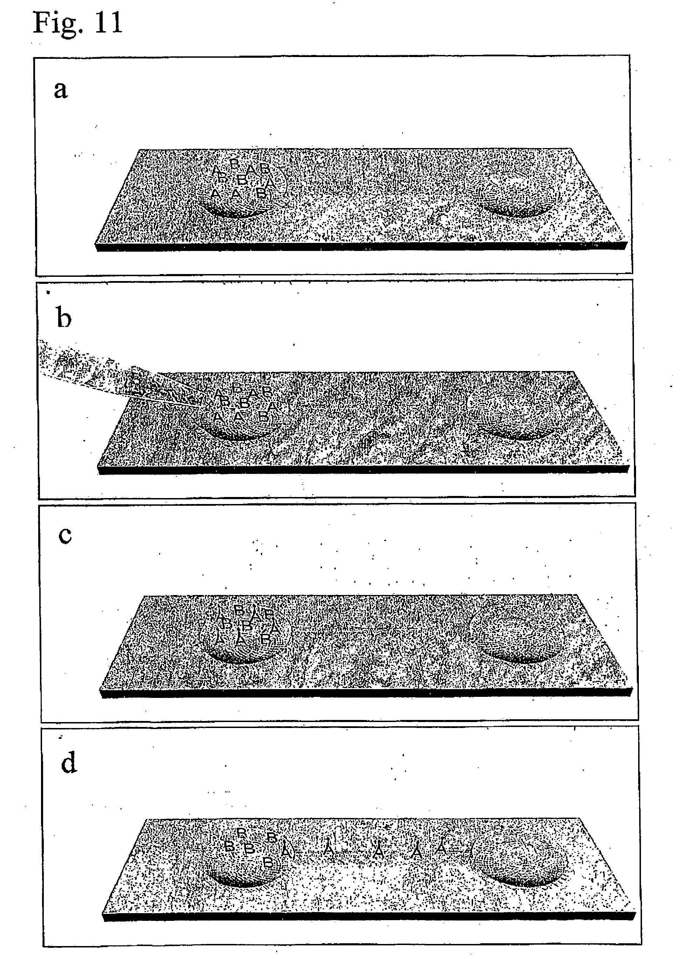

Below reference is made to the drawings, which depict the following.

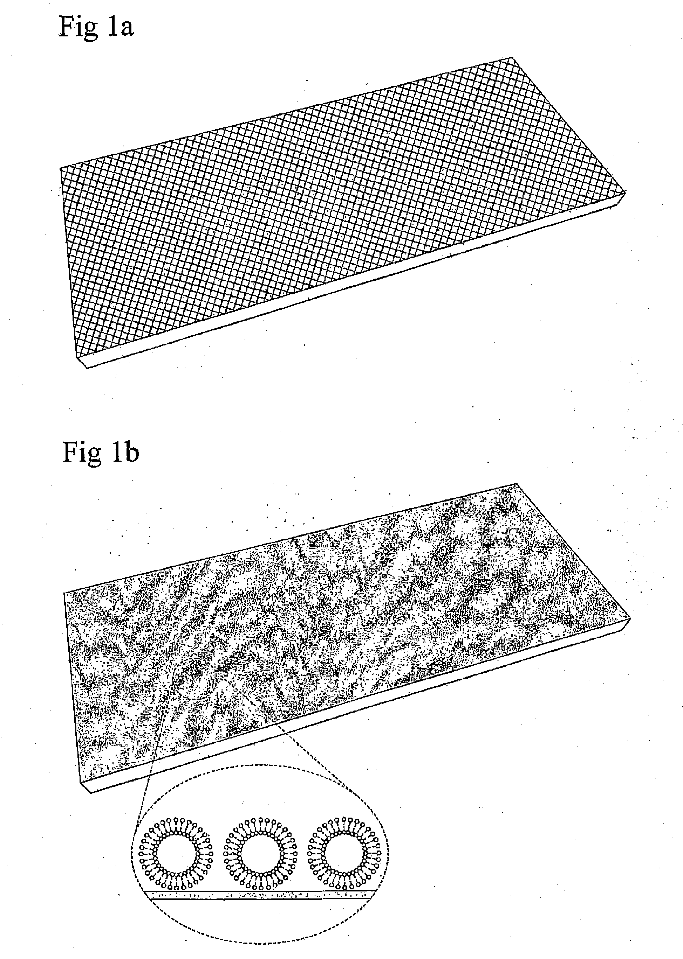

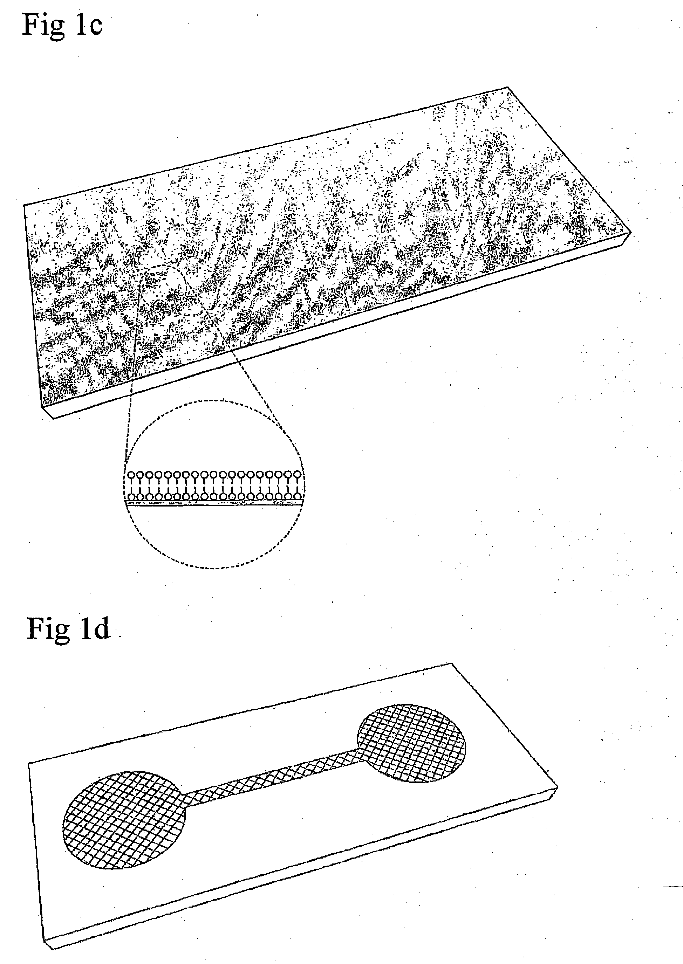

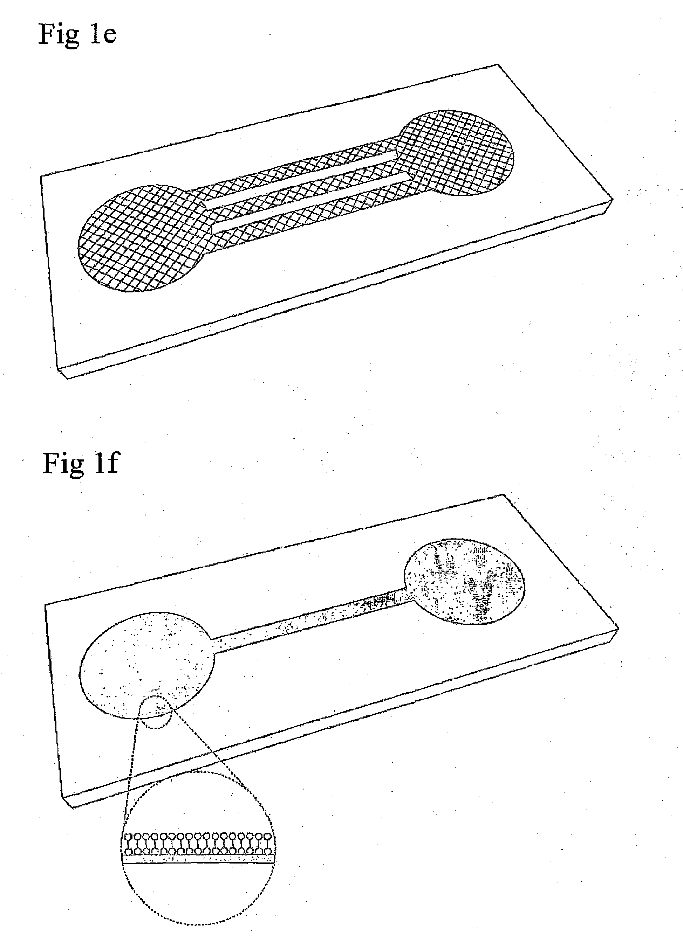

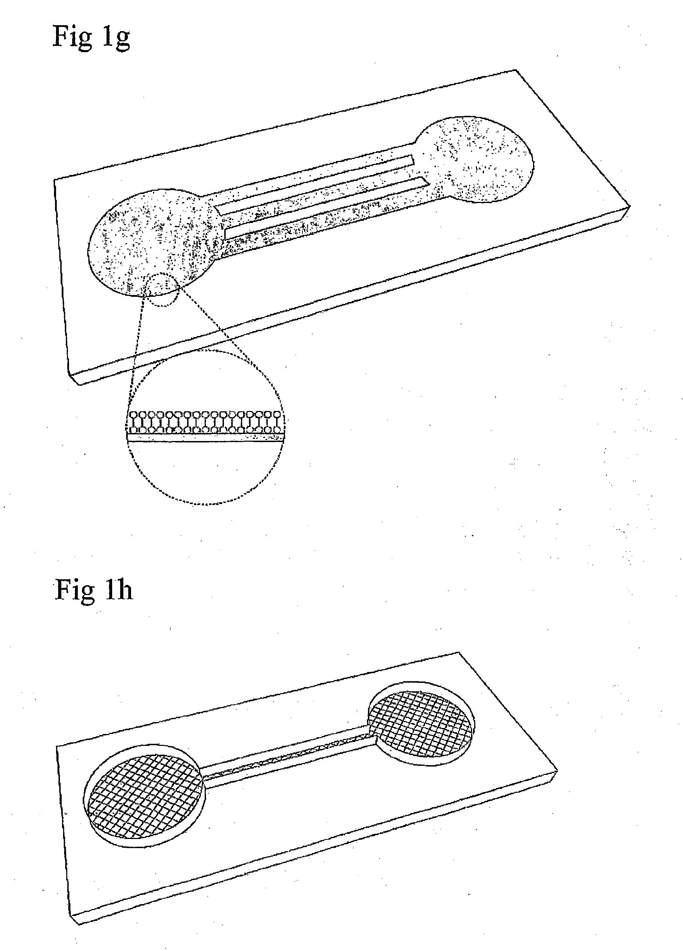

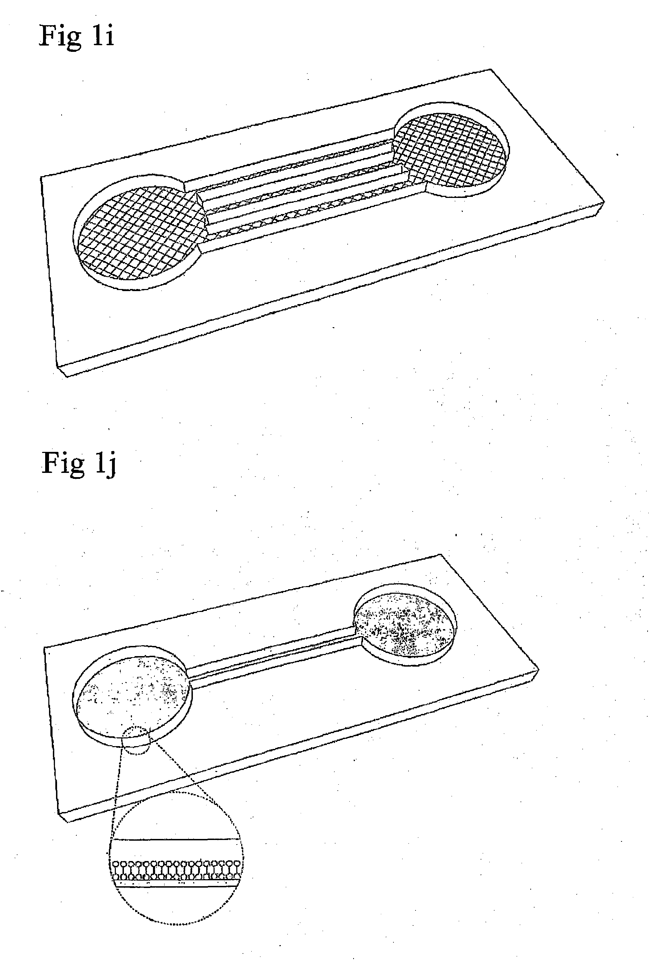

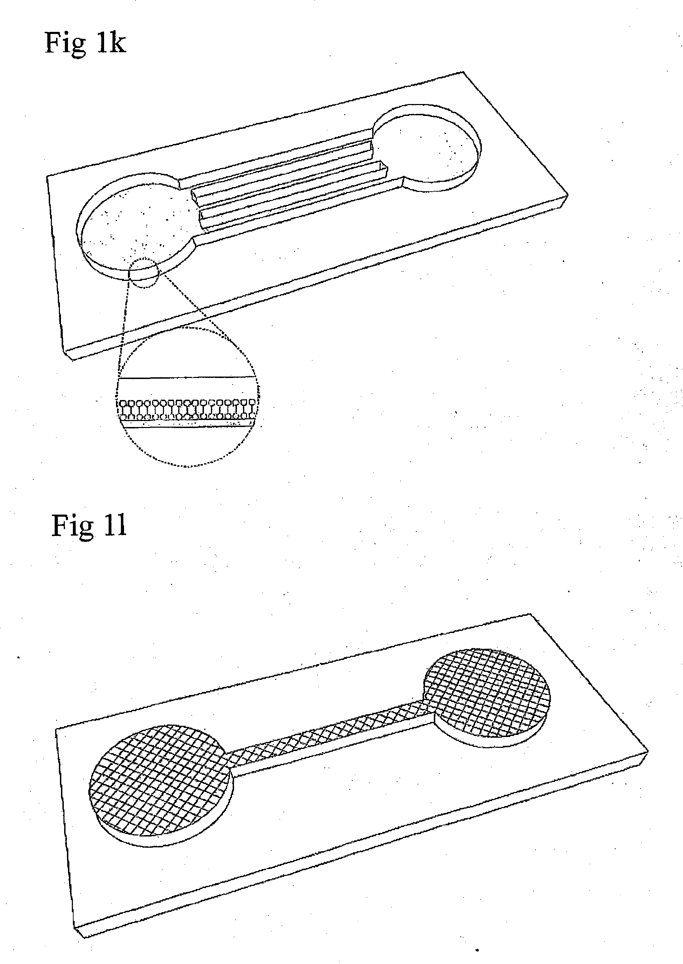

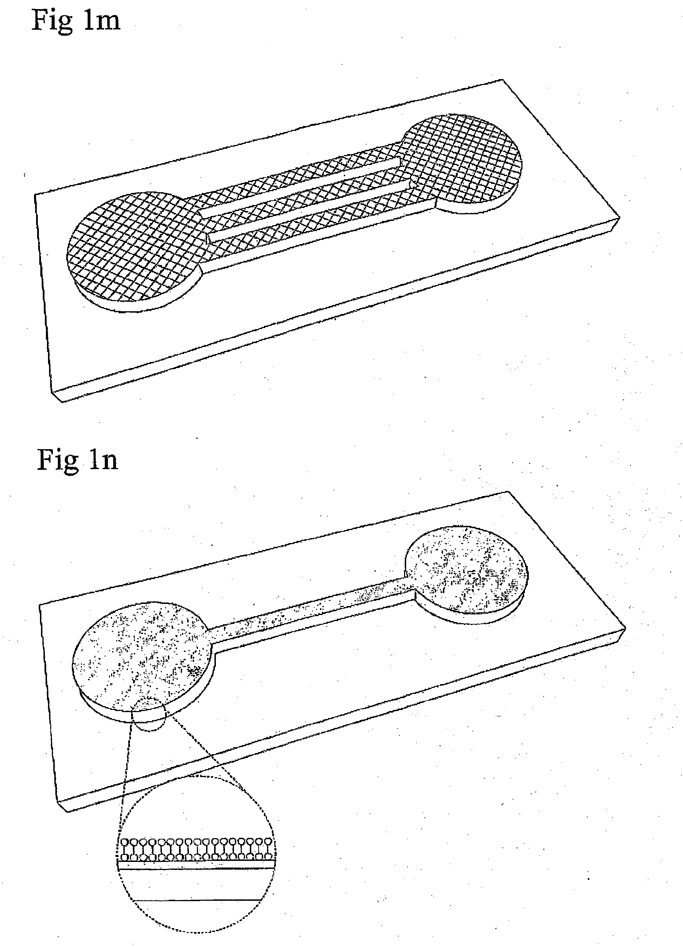

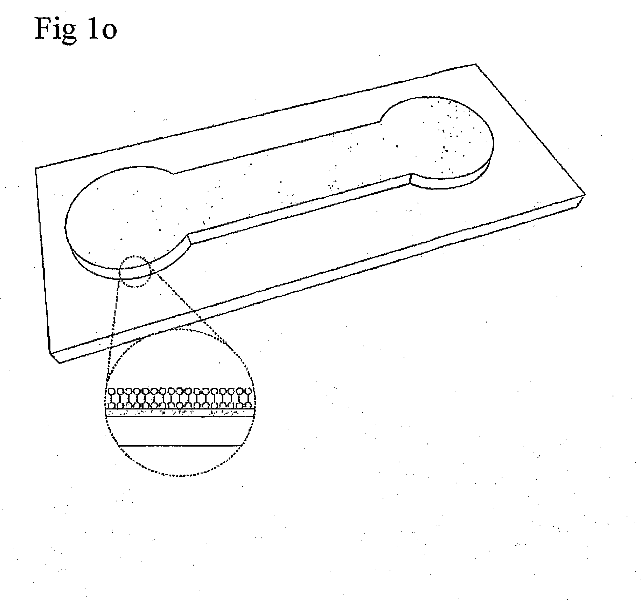

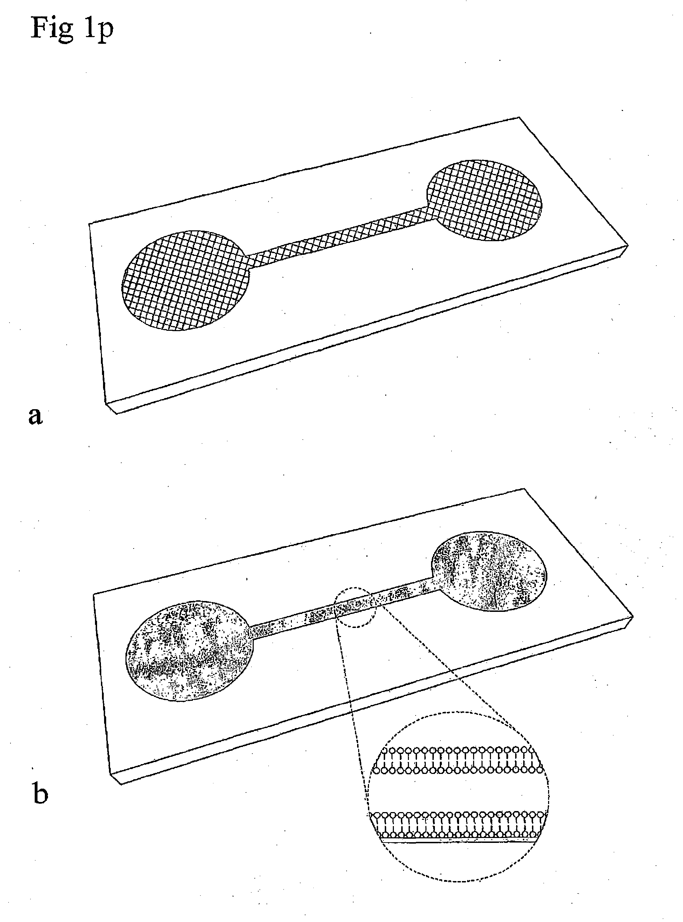

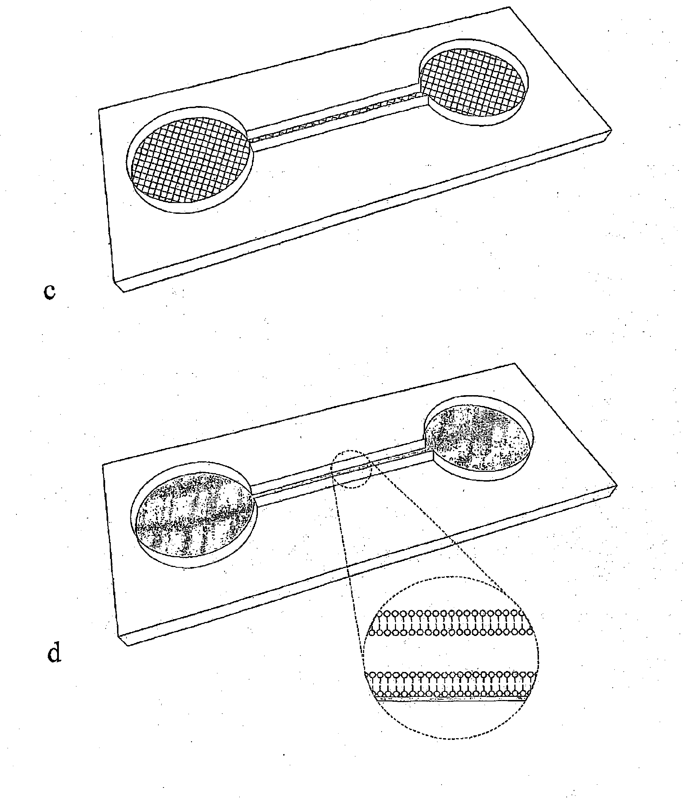

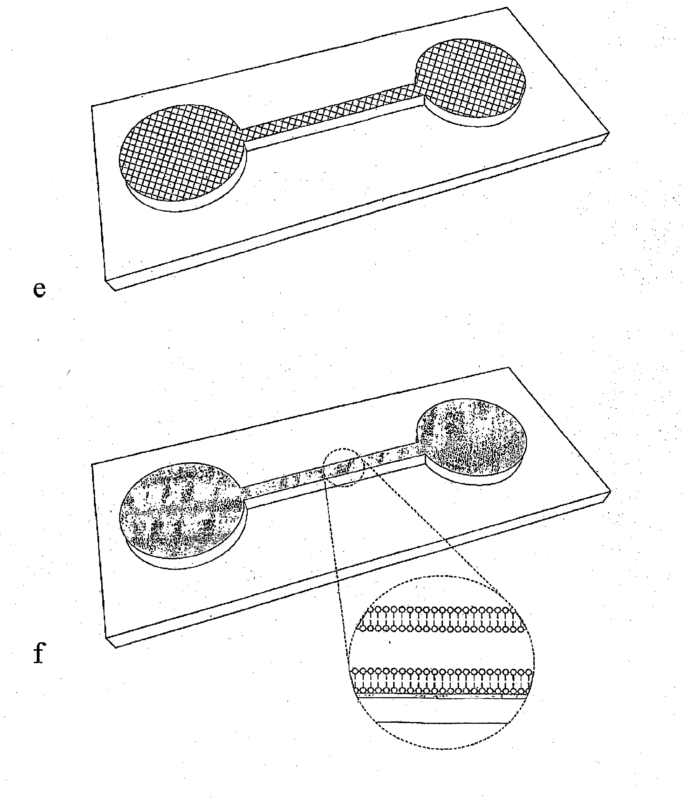

Fig 1. Schematic drawing of chip-structures for formation of supported and suspended bilayer planar and enclosed membranes.
   1. a) One example of a Membraneophilic surface without structures. The membraneophilic area in this, and the subsequent images is depicted with a rastered rendition.
   2. b) Vesicles (spherical lipid membrane structures) attached to the membraneophilic surface shown in figure 1 a.
   3. c) Planar supported membranes covering the membraneophilic surface shown in figure 1 a.
   4. d) One example of a planar surface with membraneophilic/membraneophobic domains where a single lane connects two islands. Lane and islands can consist of the same or different materials and can be of different sizes.
   5. e) One example of a planar surface with membraneophobic/membraneophobic domains where three lanes connects two islands
   6. f) Planar supported membranes covering the membraneophilic but not the membraneophobic surfaces in figure 1d.
   7. g) Planar supported membranes covering the membraneophilic but not the membraneophobic surfaces in figure 1 e.
   8. h) One example of a surface with membraneophilic/membraneophobic domains and surface topography where the membraneophilic domains are placed in wells and consist of two recessed islands connected by a single lane.
   9. i) One example of a surface with membraneophilic/membraneophobic domains and surface topography where the membraneophilic domains are placed in wells and consist of two recessed islands connected by three lanes.
   10.j) Planar supported membranes covering the membraneophilic-but not the membraneophobic surfaces in figure 1h.
   11. k) Planar supported membranes covering the membraneophilic but not the membraneophobic surfaces in figure 1 i.
   12.1) One example of a surface with membraneophilic/membraneophobic domains and surface topography where the membraneophilic domains are placed on the top surface of the elevated structures. In this particular instance two elevated circular pillars are connected by a single lane.
   13. m) One example of a surface with membraneophilic/membraneophobic domains and surface topography where the membraneophilic domains are placed on the top surface of the elevated structures. In this particular instance two elevated circular pillars are connected by three lanes.
   14.n) Planar supported membranes covering the membraneophilic but not the membraneophobic surfaces in figure 11.
   15.o) Planar supported membranes covering the membraneophilic but not the membraneophobic surfaces in figure 1 m. Please note the suspended planar membranes interdigitated between the three-lanes.
   16. p) Suspended tubular membrane structures on surfaces. Nanotube-vesicle networks with surface-immobilized nanotubes.
      1. a. Schematic showing two spots connected by a lane. The membraneophilic surface is in plane with the rest of the surface.
      2. b. A vesicle-nanotube network is attached to the membraneophilic surface. The inset shows the surface-attached nanotube on the connecting lane.
      3. c. Schematic showing two spots connected by a lane. The membraneophilic surface is below the plane of the rest of the surface.
      4. d. A vesicle-nanotube network is attached to the membraneophilic surface. The inset shows the surface-attached nanotube on the connecting lane.
      5. e. Schematic showing two spots connected by a lane. The membraneophilic surface is above the plane of the rest of the surface.
      6. f. A vesicle-nanotube network is attached to the membraneophilic surface. The inset shows the surface-attached nanotube on the connecting lane.

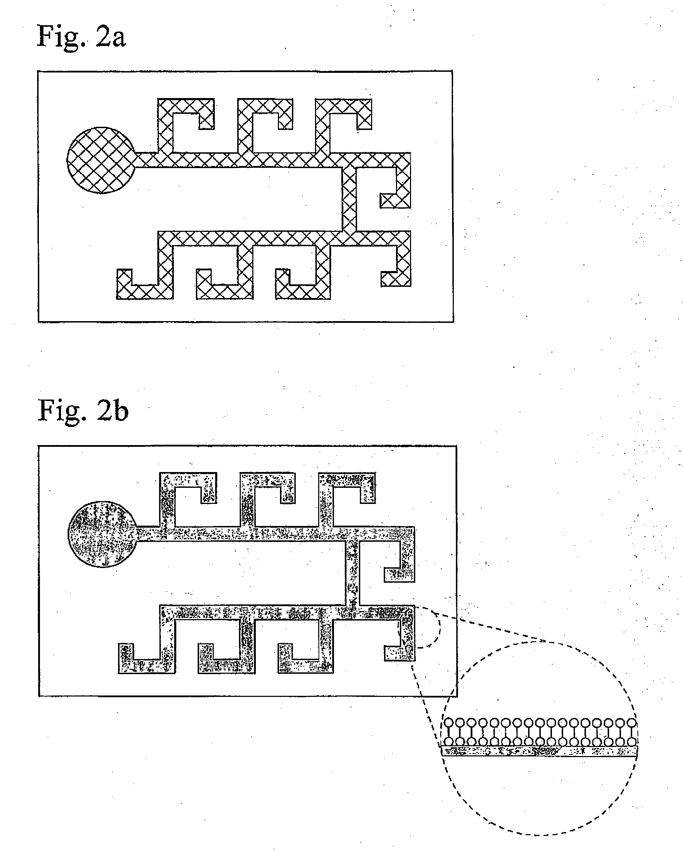

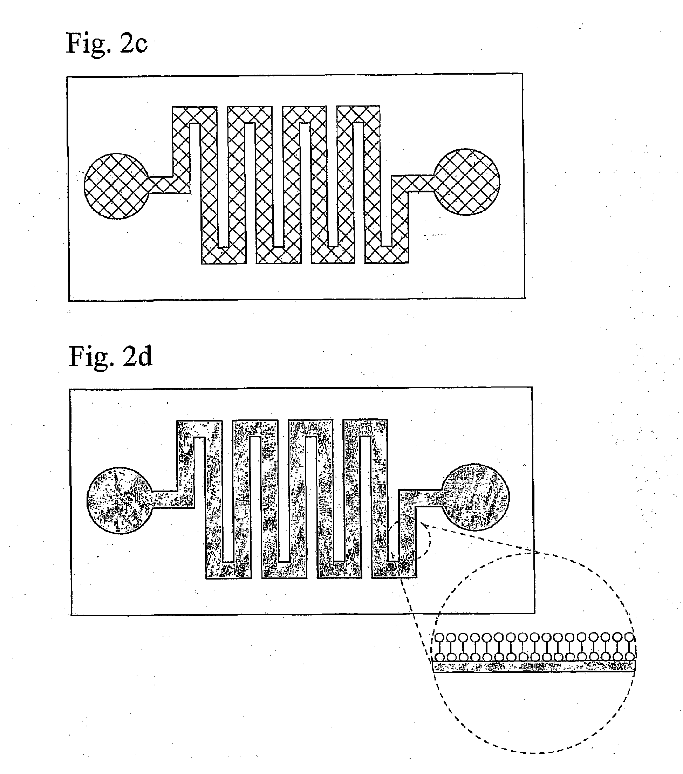

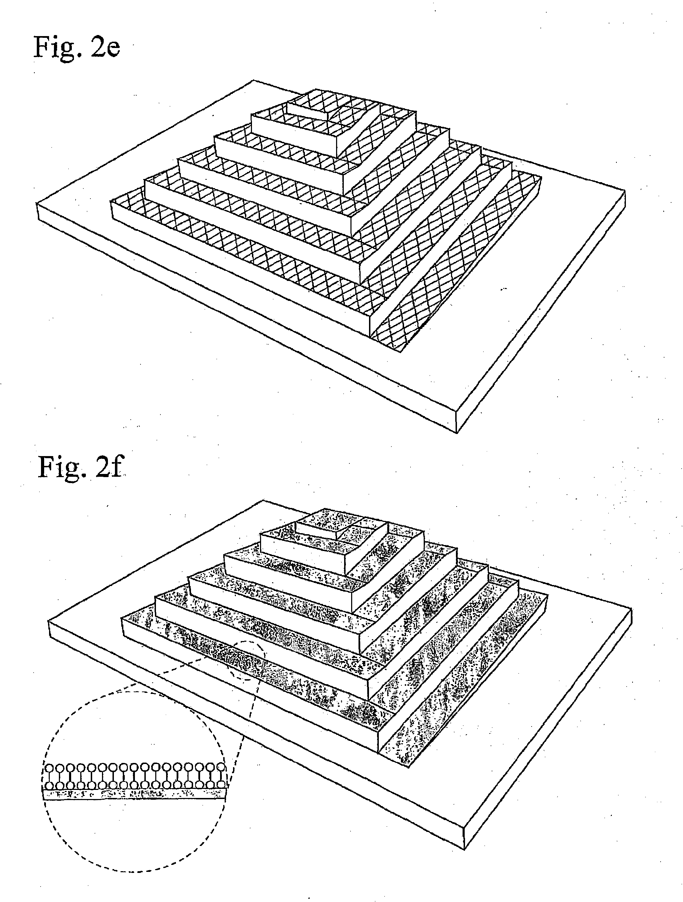

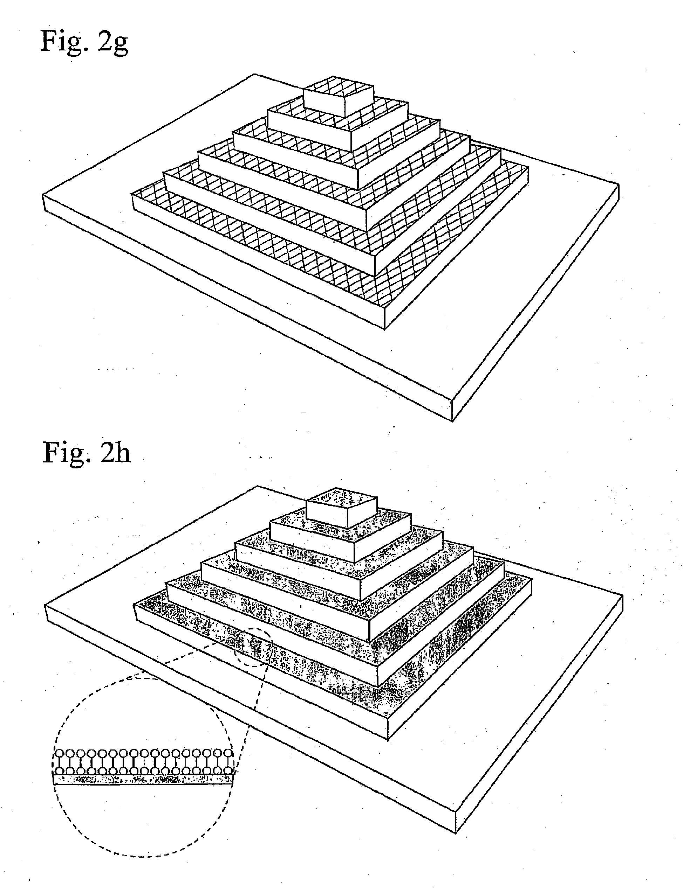

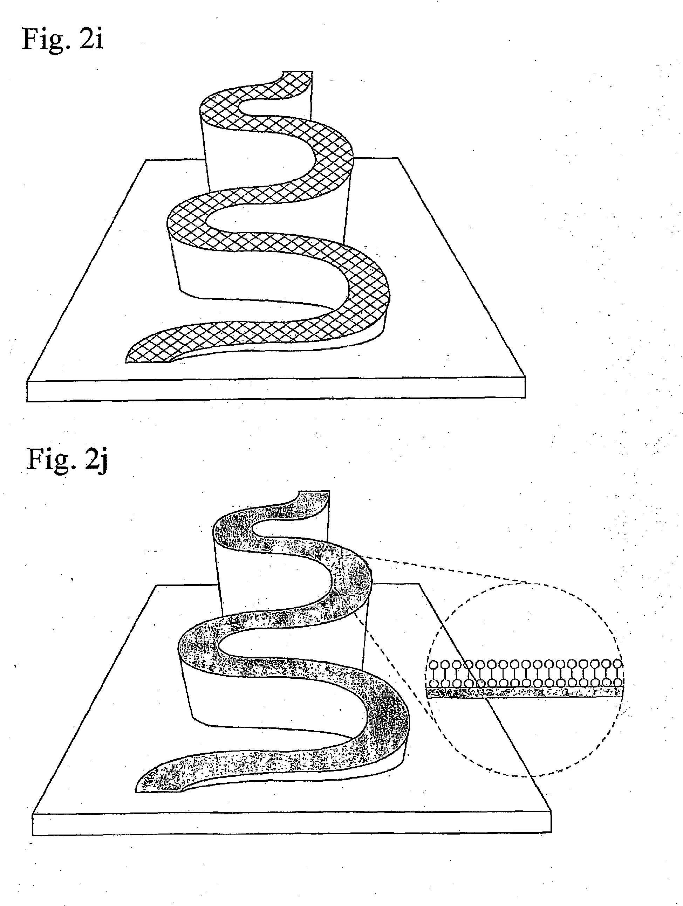

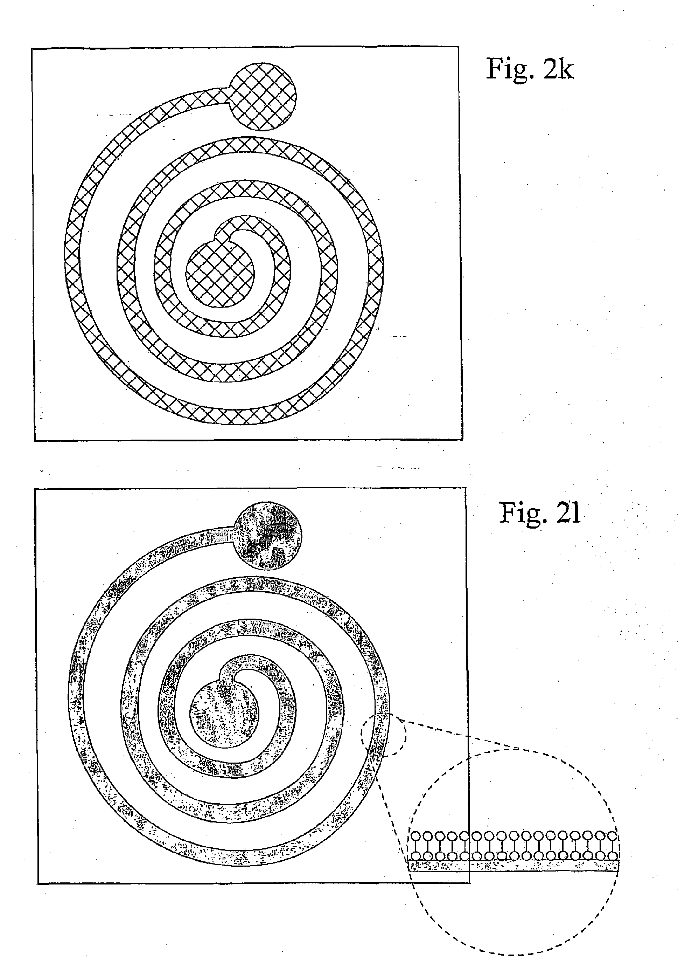

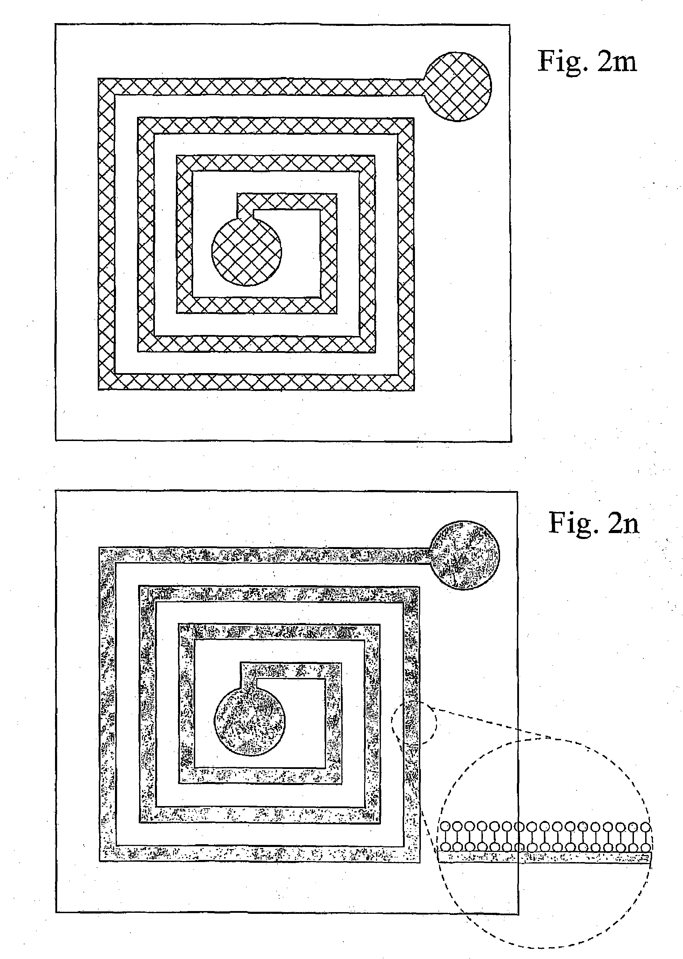

Fig. 2. Ten examples of different, non-limiting substrates including three-dimensional gray-scale substrates having membraneophobic and membraneophobic regions.
   1. a) Membraneophilic meander-boarder type of pattern on a membraneophobic substrate.
   2. b) Surface in 2a covered by a bilayer membrane.
   3. c) Membraneophilic serpentine type of pattern on a membraneophobic substrate.
   4. d) Surface in 2c covered by a bilayer membrane.
   5. e) Membraneophilic pattern on the upper surface of a 3-dimensional membraneophobic pyramid-shaped substrate.
   6. f) Surface in 2e covered by a bilayer membrane.
   7. g) Membraneophilic pattern on the upper surface of a 3-dimensional membraneophobic step-pyramid-shaped substrate.
   8. h) Surface in 2g covered by a bilayer membrane.
   9. i) Membraneophilic pattern on the upper surface of a 3-dimensional membraneophobic serpentine-shaped substrate.
   10.j) Surface in 2i covered by a bilayer membrane.
   11. k) Membraneophilic circular serpentine labyrinth type of pattern on a membraneophobic substrate.
   12.1) Surface in 2k covered by a bilayer membrane.
   13.m) Membraneophilic square-shaped serpentine labyrinth type of pattern on a membraneophobic substrate.
   14. n) Surface in 2m covered by a bilayer membrane.

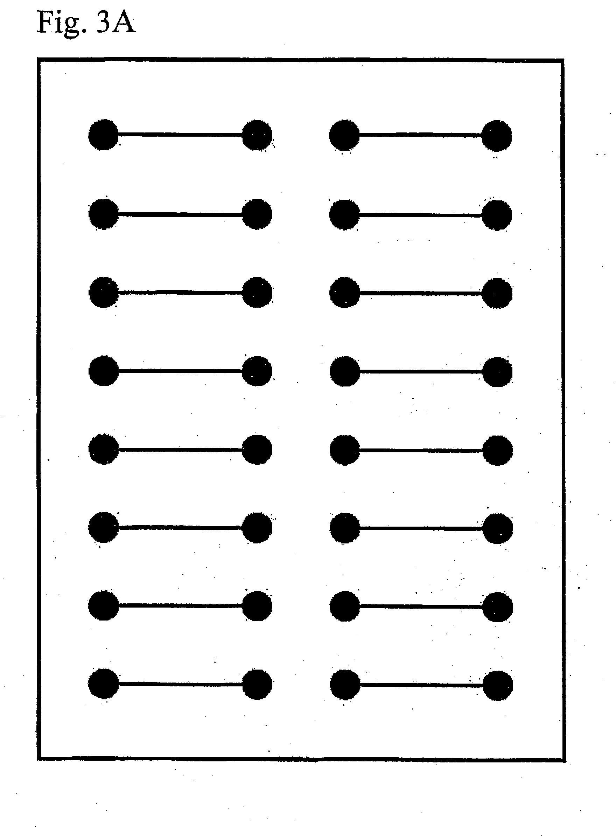

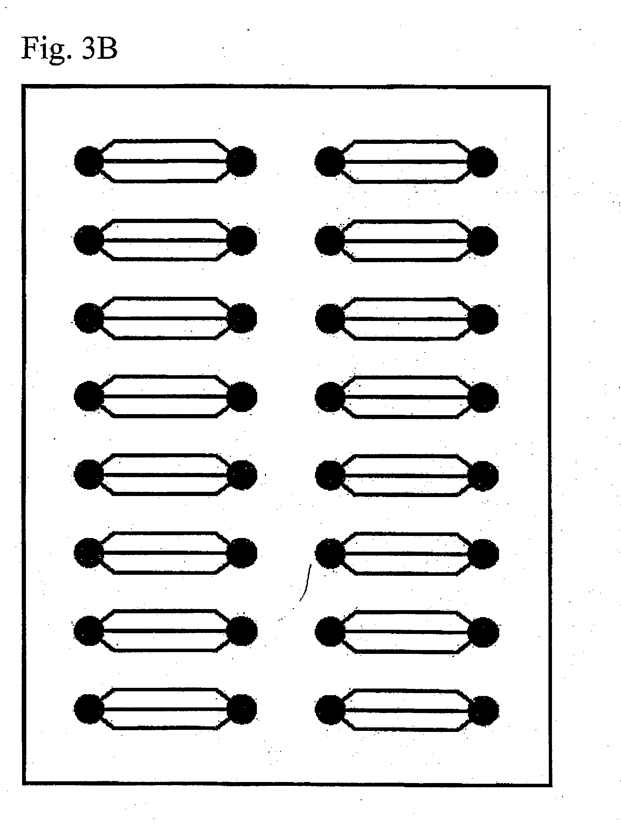

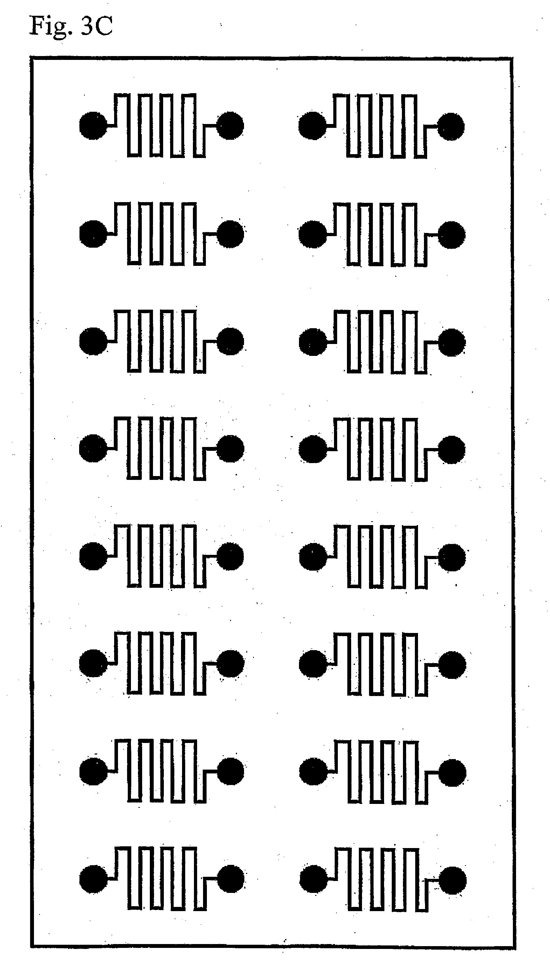

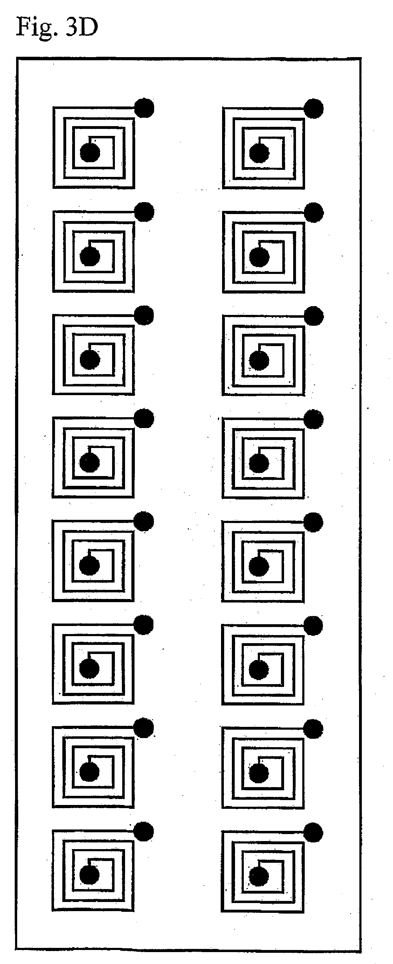

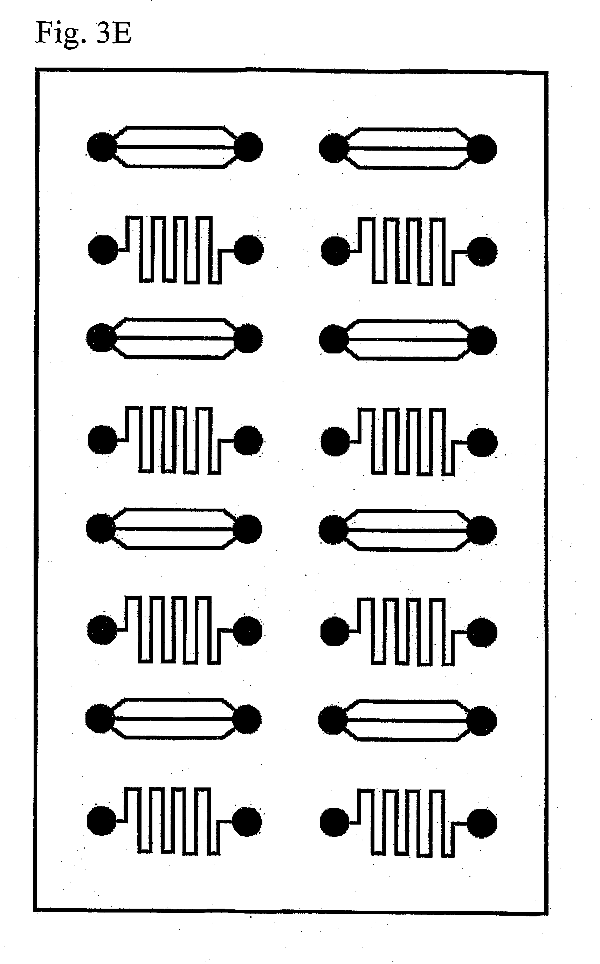

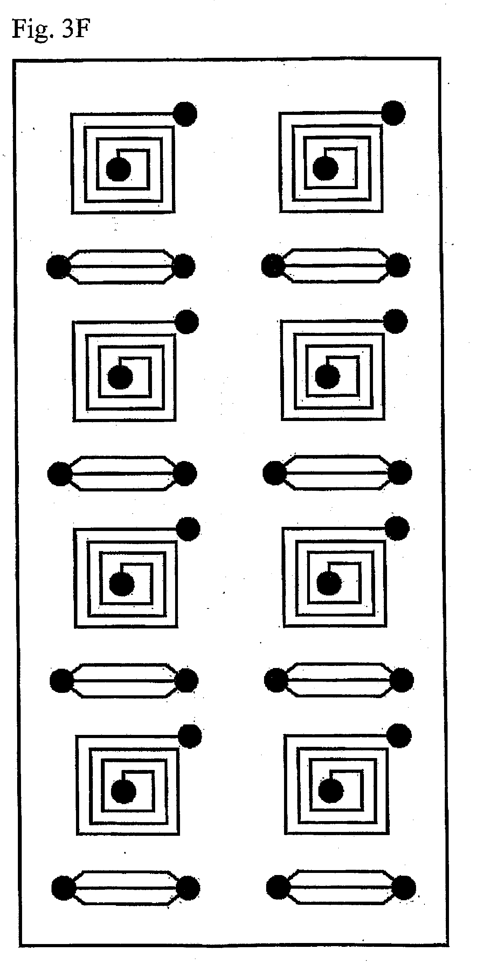

Fig 3. Different multiplexed and parallel devices
   1. a) Shows arrays of sixteen identical membraneophilic patterns consisting of two circular spots connected by a single lane on a membraneophobic substrate. Here the membraneophilic regions are depicted in black color.
   2. b) Shows arrays of sixteen identical membraneophilic patterns consisting of two circular spots connected by three lanes on a membraneophobic substrate
   3. c) Shows arrays of sixteen identical membraneophilic patterns consisting of two circular spots connected by a single lane arranged in a serpentine pattern on a membraneophobic substrate
   4. d) Shows arrays of sixteen identical membraneophilic patterns consisting of two circular spots connected by a single lane arranged in a labyrinth pattern on a membraneophobic substrate
   5. e) Shows arrays of sixteen membraneophilic patterns of two different types consisting of i) two circular spots connected by a single lane arranged in a serpentine pattern and ii) two circular spots connected by three lanes on a membraneophobic substrate
   6. f) Shows arrays of sixteen membraneophilic patterns of two different types consisting of i) two circular spots connected by a single lane arranged in a labyrinth pattern and ii) two circular spots connected by three lanes on a membraneophobic substrate
Fig 4. Examples of different microchips having features for carrying lipid bilayer membranes and additional structures.
   1. a) Chip with six identical membraneophilic regions consisting of two spots connected to a lane arranged in a circular labyrinth pattern. Electrodes are connected to the two spots for application of electric fields.
   2. b) Chip with six identical membraneophilic regions consisting of two spots connected to three lanes. Electrodes are connected to the two spots for application of electric fields.
   3. c) Chip with six identical membraneophilic regions consisting of two spots connected to seven lanes. Electrodes are connected to the two spots for application of electric fields.
   4. d) Chip with one pattern having membraneophilic regions consisting of two spots connected to a bifurcating network of lanes. Electrodes are connected external to the two spots for application of electric fields. Electrodes depicted as small black circular spots are also connected at each bifurcation point of the network for application of electric fields.
   5. e) Chip with one pattern having membraneophilic regions consisting of two spots connected to a bifurcating network of lanes. Electrodes are connected external to the two spots for application of electric fields. Electrodes depicted as small black circular spots are also connected at each bifurcation point of the network for application of electric fields.
   6. f) A circular membraneophilic region connected to two embedded electrodes.
   7. g) A circular membraneophilic region placed proximal to two external electrodes
   8. h) A. circular membraneophilic region with one central and six peripheral electrodes
   9. i) A circular membraneophilic region placed in a quadrapole electrode arrangement
   10.j) A circular membraneophilic region placed in a octapole electrode arrangement
   11. k) A membraneophilic labyrinth pattern having four external electrodes and two additional electrodes connected to the start and endpoints of the labyrinth, respectively.
Fig 5. Examples of different microchips having features for carrying lipid bilayer membranes and additional structures.
   1. a) Chip with recessed membraneophilic regions consisting of two spots connected to three lanes. The two outer lanes, and the two spots are connected to microfluidic channels that in turn are connected to reservoirs. The microfluidic delivery is in this instance controlled by pressure (P).
Fig 6. Spreading of lipids on different pure substrates.
   1. a) Plot showing spreading of soy-bean lipids on a plasma-treated gold surface, made membraneophilic through chemical and/or physical modulation or manipulation versus time. Initial area of the lipid spot is 0.1x10⁴ µm², after 4.8s, the lipid area occupies 1.8x10⁴ µm². The lipid was originally deposited on the surface as a multilamellar liposome.
   2. b) Soy-bean lipid spreading on a plasma-treated gold surface, made membraneophilic through chemical and/or physical modulation or manipulation, as seen through a microscope. The time point for the left image is t=0.4s after the liposome was deposited and the second image represents t=5.2s after the liposome was deposited. These pictures correspond to first and the last points in the graph displayed in figure 6a.
   3. c) Plot showing spreading of soy-bean lipids on a plasma-treated SU-8 surface versus time. The initial area of the spot was 327 µm², and after 13 minutes the lipid material shows no spreading. An apparent decrease of the lipid area is due to photo bleaching effect. The lipid was originally deposited on the surface as a multilamellar liposome.
   4. d) Fluorescence microscopy images showing spreading of a soy-bean liposome on a plasma-treated SU-8 surface with a time interval of 13 min. These pictures correspond to first (left image) and the last (right image) points in figure 6c.

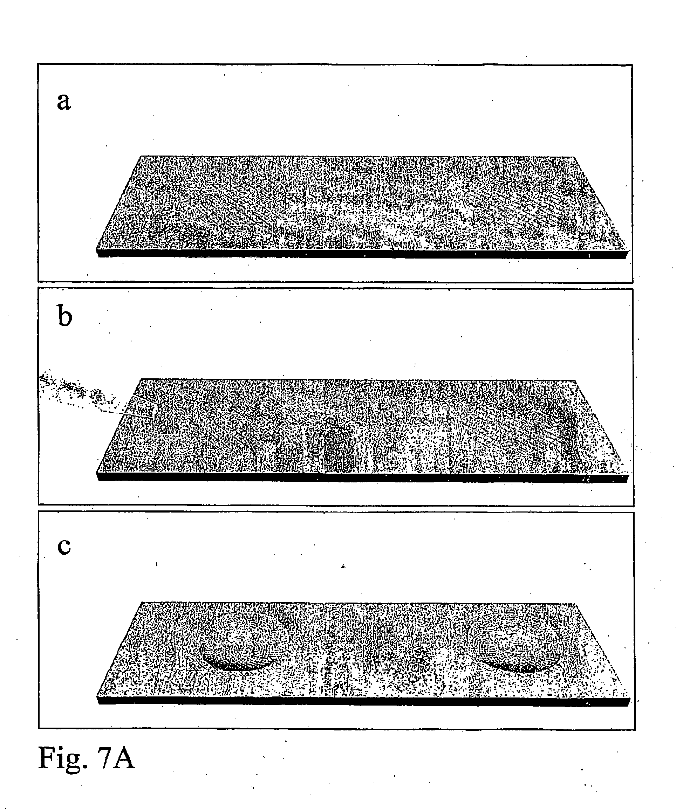

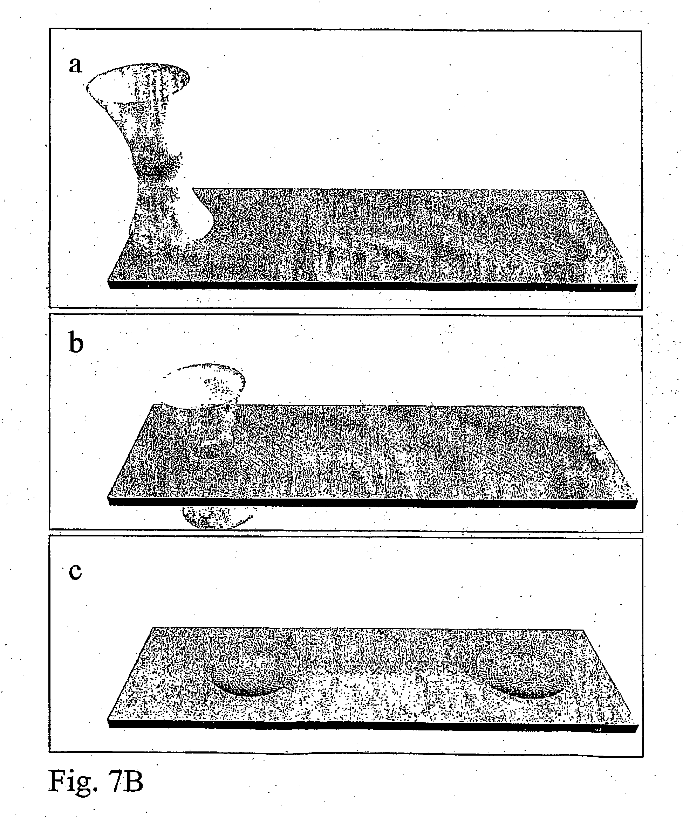

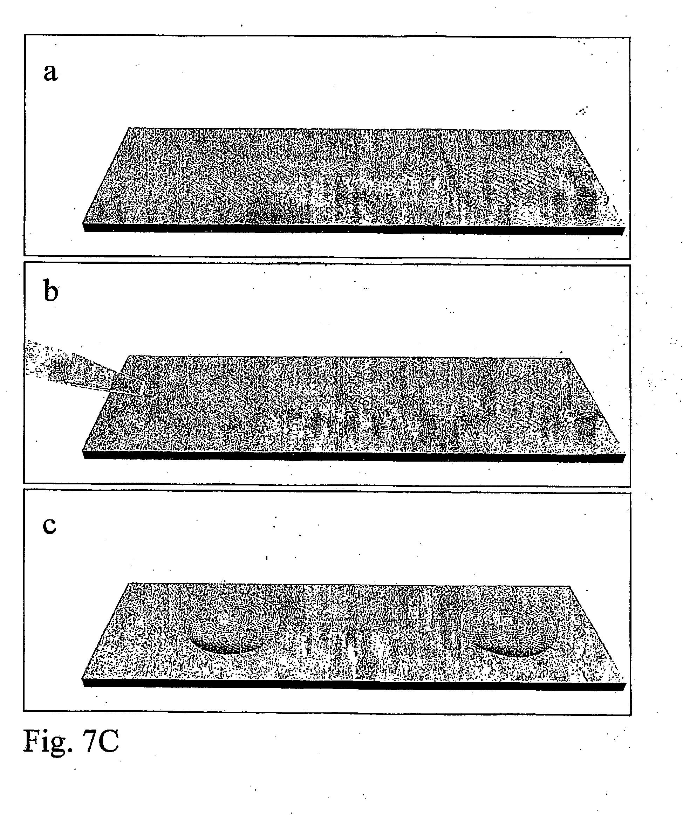

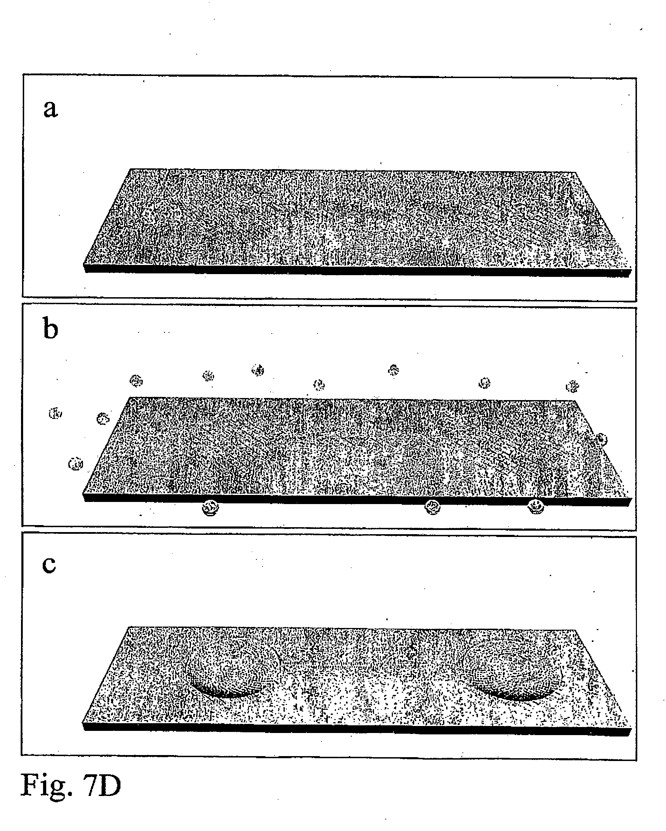

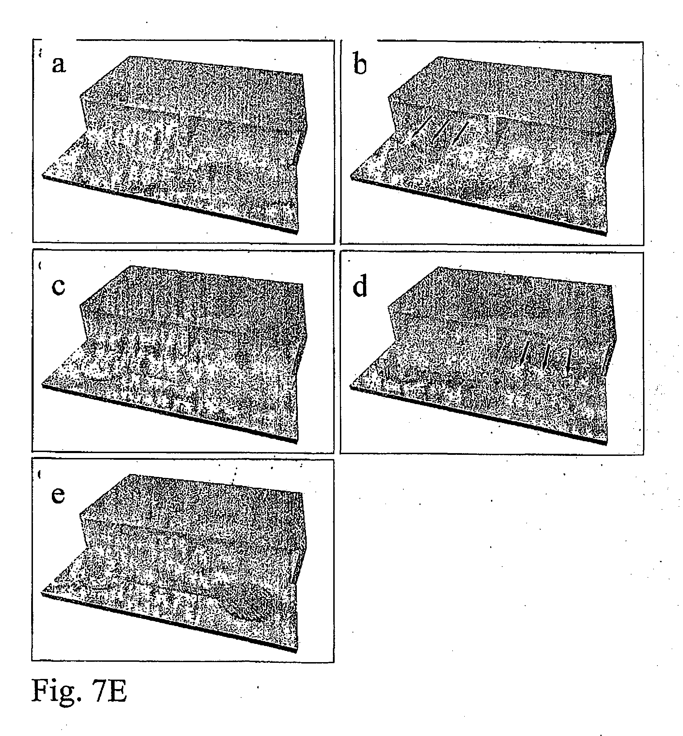

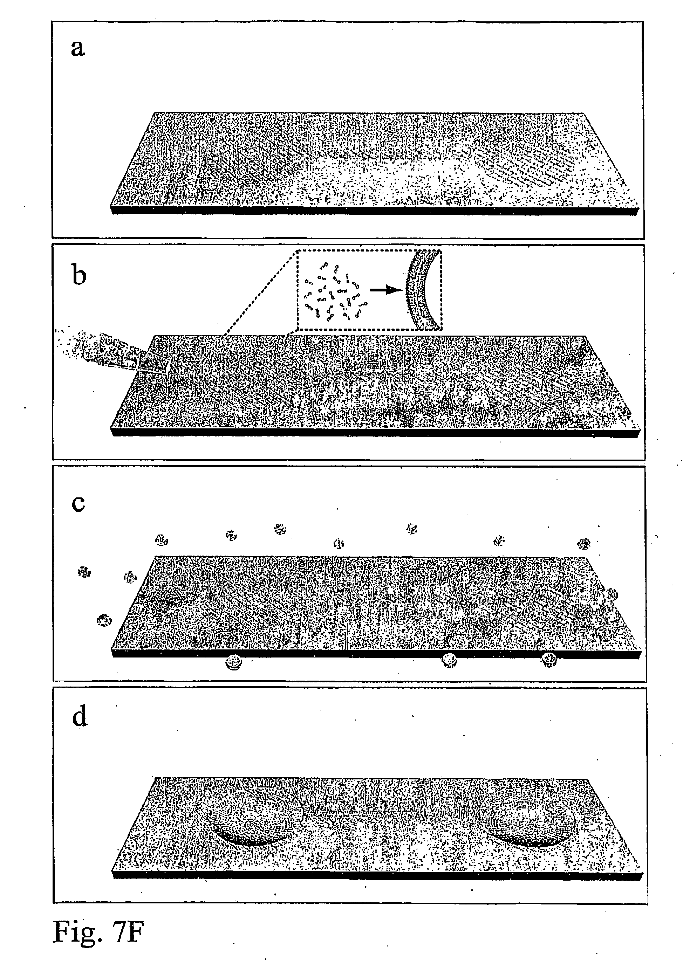

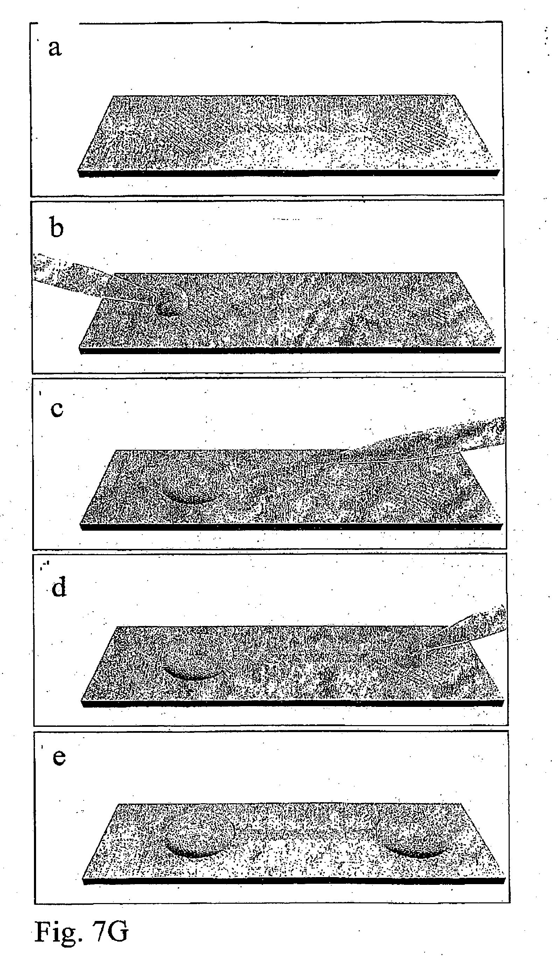

Fig. 7. Schematic showing different techniques to deposit lipids to the devices and how the lipid structures are formed.
   1. A) Deposition of multilamellar vesicles using micropipettes
      1. a) Schematic showing the chip structure containing membraneophilic and surrounding membraneophobic regions.
      2. b)A multilamellar vesicle is translated to the surface using a micromanipulator-controlled micropipette. The vesicle is held by the micropipette tip by applying a low negative (suction) pressure. The vesicle is ejected onto the membraneophilic surface by applying a small positive pressure in the micropipette and the vesicle thereby adheres to the membraneophilic surface.
      3. c) The multilamellar vesicle spreads out onto the membraneophilic surface. The membraneophilic surface of the designed structure is finally covered completely by a lipid bilayer matrix.
   2. B) Deposition of multilamellar vesicles using optical tweezers
      1. a) Schematic showing the chip structure containing membraneophilic and surrounding membraneophobic regions. Also shown is a multilamellar vesicle trapped by a focused beam of high-intensity light. The high-intensity focused light works as an optical pair of tweezers, trapping materials that have different refractive index than the surrounding solution.
      2. b)The focus of the optical pair of tweezers is moved in order to position the multilamellar vesicle onto the membraneophilic surface. The light is turned off, releasing the multilamellar vesicle.
      3. c) The multilamellar vesicle spreads out onto the membraneophilic surface. The membraneophilic surface of the designed structure is finally covered completely by a lipid bilayer matrix.
   3. C) Deposition of lipids using a pipette-writing technique by translation of a pipette filled with lipid material across the surface
      1. a) Schematic showing the chip structure containing membraneophilic and surrounding membraneophobic regions.
      2. b)A micromanipulator-controlled micropipette is filled with lipid material and translated to the surface. A portion of the lipid material is ejected out from the tip of the pipette by applying a low positive pressure. The portion of lipid material (for example uni- or multilamellar vesicle) is then allowed to adhere to the membraneophilic surface, whereby the micropipette is removed, leaving the portion of lipid material on the surface. The tip of the micropipette is then translated to another designed structure of membraneophilic pattern, where a new portion can be ejected.
      3. c) The multilamellar vesicle spreads out onto the membraneophilic surface. The membraneophilic surface of the designed structure is finally covered completely by a lipid bilayer matrix.
   4. D) Deposition of lipids using fusion of small vesicles
      1. a) Schematic showing the chip structure containing membraneophilic and surrounding membraneophobic regions.
      2. b)A solution containing small vesicles (uni- or multilamellar) is placed in the solution above the chip. The small vesicles settle to the surface and adhere to the membraneophilic surfaces. Depending on chemical parameters, such as lipid concentration, lipid composition, pH, ionic strength, buffer composition and physical parameters, such as temperature and the composition of the membraneophilic surface, the vesicles can stay intact or spontaneously form a continuous lipid bilayer structure on the membraneophilic surface. Different wash protocols might also be used for transforming the surface-adhered vesicles into a continuous lipid bilayer membrane.
      3. c) The membraneophilic surface of the designed structure is finally covered completely by a lipid bilayer matrix.
   5. E) Deposition of lipid bilayer membranes through microfluidic channels
      1. a) Schematic showing the chip structure containing membraneophilic and surrounding membraneophobic regions. The chip can also be integrated with microfluidic channels, where the flow of a channel can be highly focused to a part of the chip, or even parts of the individual designed membraneophilic structures.
      2. b)A solution containing small vesicles (uni- or multilamellar) is flushed across a part of the designed membraneophilic structure through the use of the microfluidic channels. The small vesicles settle to the surface and adhere to the membraneophobic surfaces and can be manipulated to stay as vesicles or form a continuous lipid bilayer membrane as described in figure 7Db. The wash protocols can be used in conjunction with the microfluidic channel structures.
      3. c) A part of the membraneophilic surface of the designed structure is finally covered completely by a lipid bilayer matrix.
      4. d)Another solution containing another type of small vesicles (uni- or multilamellar) is flushed across another part of the designed membraneophilic structure through the use of the micro fluidic channels. The small vesicles settle to the surface and adhere to the membraneophobic surfaces and can be manipulated to stay as vesicles or form a continuous lipid bilayer membrane as described in figure 7Db. The wash protocols can be used in conjunction with the microfluidic channel structures.
      5. e) Another part of the membraneophilic surface of the designed structure is finally covered completely by a lipid bilayer matrix. This allows the formation of heterogeneous lipid matrices on the designed membraneophilic surfaces, either on different structures or even within a single structure.
   6. F) Formation of lipid bilayer membrane by direct injection of dissolved lipids
      1. a) Schematic showing the chip structure containing membraneophilic and surrounding membraneophobic regions.
      2. b)Lipids are injected into the solution using appropriate solvents. The lipids can be made to spontaneously form small vesicles (uni- or multilamellar).
      3. c) The small vesicles settle to the surface and adhere to the membraneophilic surfaces and can be manipulated to stay as vesicles or form a continuous lipid bilayer membrane as described in figure 7Db.
      4. d)The membraneophilic surface of the designed structure is finally covered completely by a lipid bilayer matrix.
   7. G) Formation of bilayer devices from nanotube-vesicle networks.
      1. a) Schematic showing the chip structure containing membraneophilic and surrounding membraneophobic regions.
      2. b)A micropipette filled with membrane material is positioned close to the surface and a small portion is ejected onto a membraneophilic region, for example, a spot.
      3. c) The membrane material spreads on the surface but remain connected to the membrane material in the micropipette via a lipid nanotube. The micropipette is then positioned next to another membraneophilic spot.
      4. d)A new portion of membrane material is ejected onto the membraneophilic spot.
      5. e) The membrane material spreads on the membraneophilic surface and the lipid nanotube that connects the two membrane parts and is attached to the underlying membraneophilic lane.

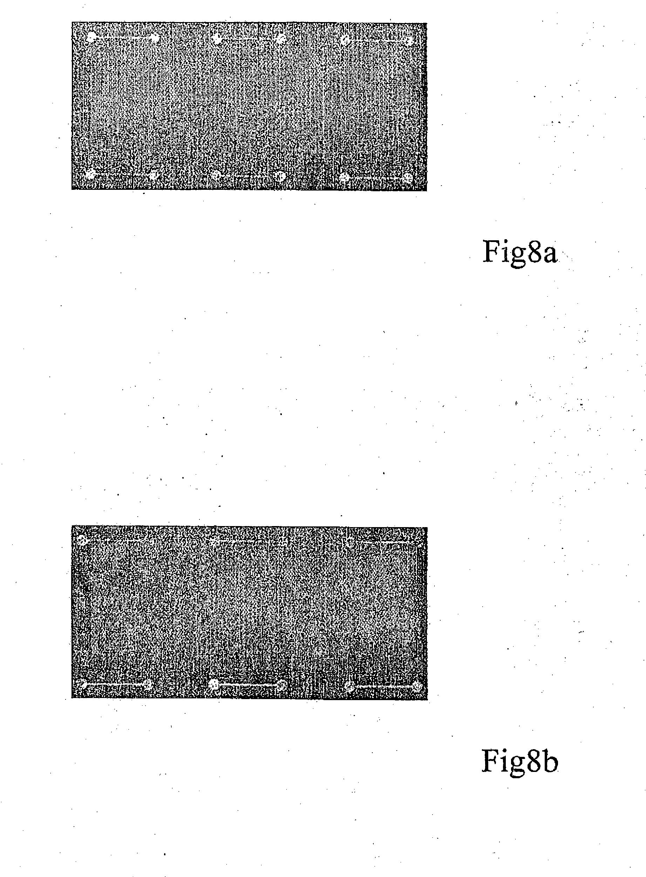

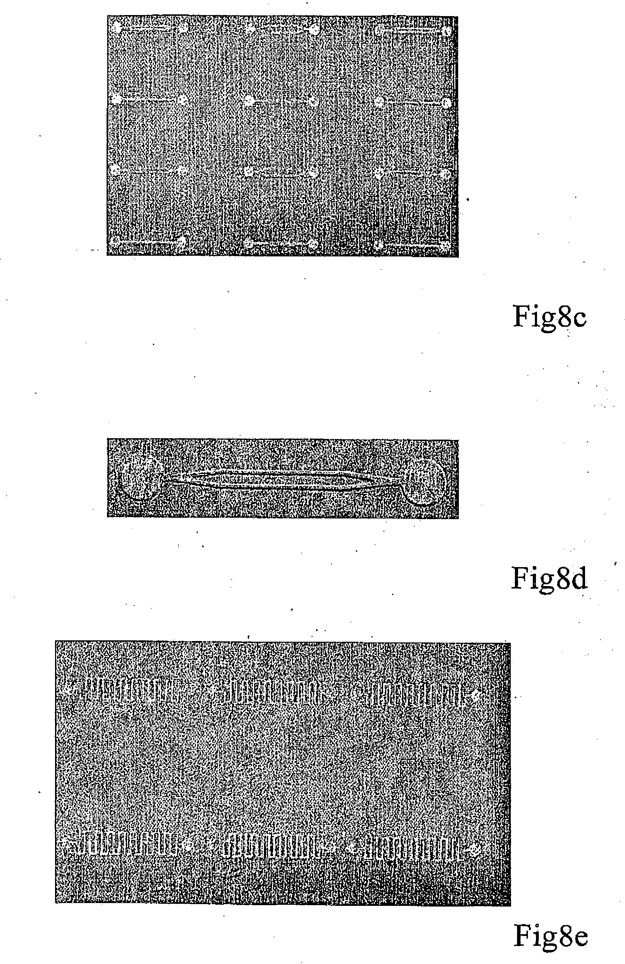

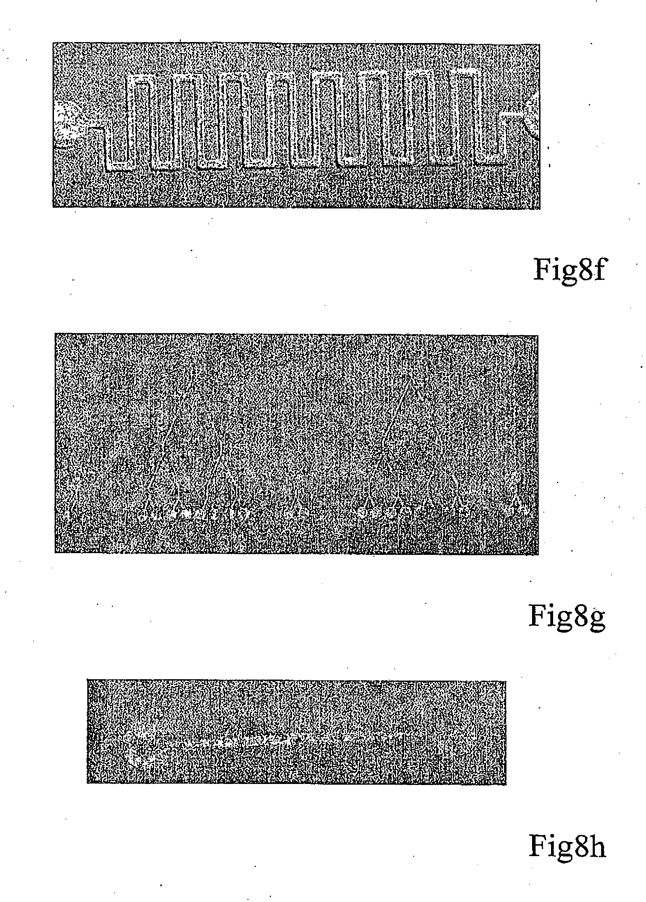

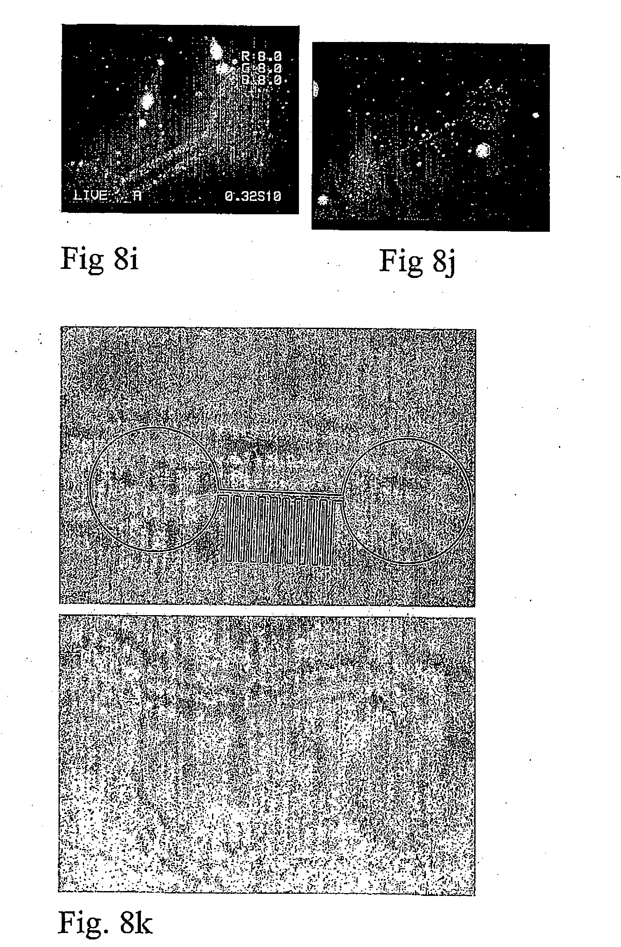

Fig 8. Photomicrographs of microfabricated chip structures of different sizes and features having differential surface properties consisting of membraneophilic (plasma-treated gold or plasma-treated Al₂O₃ surfaces, made membraneophilic through chemical and/or physical modulation or manipulation) and membraneophobic regions (plasma-treated SU-8 surface).
   1. a) Chip with membraneophilic plasma-treated gold patterns, made membraneophilic through chemical and/or physical modulation or manipulation, (appear as light-gray in the images) and plasma-treated SU-8 membraneophobic regions (appear dark grey in the images). The circular spots covered by gold are 15 micrometers in diameter, and the interconnecting lanes of gold are approximately 1 micrometer thick and 75 micrometers long.
   2. b) Same type of structures as in figure 8a, however, with 2 micrometer wide lanes.
   3. c) Chip with membraneophilic plasma-treated gold patterns, made membraneophilic through chemical and/or physical modulation or manipulation, (appear as light-gray in the images) and plasma-treated SU-8 membraneophobic regions (appear dark grey in the images). Different examples of patterns consisting of circular spots, 15 micrometers in diameter, interconnected by two lanes, with varying separation distance between the lanes.
   4. d) Enlargement of one of the structures in c). The lanes are approximately 1 micrometer wide and separated from each other by 2 micrometers.
   5. e) Chip with membraneophilic plasma-treated gold patterns, made membraneophilic through chemical and/or physical modulation or manipulation, (appear as light-gray in the images) and plasma-treated SU-8 membraneophobic regions (appear dark grey in the images). Examples of meandering structures with turns of 90 degrees. The width of the lanes is 2 micrometers in this case.
   6. f) One of the structures in e shown in expanded view.
   7. g) Chip with membraneophilic plasma-treated gold patterns, made membraneophilic through chemical and/or physical modulation or manipulation, (appear as light-gray in the images) and plasma-treated SU-8 membraneophobic regions (appear dark grey in the images). Branching or converging structures with lanes of 2 micrometers width. These structures could be used to, e.g., concentrate or dilute substances moving along the different lanes. In the case of concentrating the samples, the sample input is made on the circular spots and the transported sample from different spots can then be summed up at each branch.
   8. h) Fluorescence image illustrating coverage of lipid confined to the gold pattern, made membraneophilic through chemical and/or physical modulation or manipulation, on one of the microfabricated structures shown. The lipid membrane was stained with a fluorescent membrane dye in order to visualize the spreading.
   9. i) Chip having a membraneophilic pattern of plasma-treated gold, made membraneophilic through chemical and/or physical modulation or manipulation, and a membraneophobic surface defined by plasma-treated SU-8. The fluorescence micrograph show that fluorescently labeled soy-bean liposomes added into the buffer solution predominantly adhere to the membraneophilic pattern of plasma-treated gold, made membraneophilic through chemical and/or physical modulation or manipulation.
   10.j) Chip having a membraneophilic pattern of plasma-treated gold, made membraneophilic through chemicals and/or physical modulation or manipulation, and a membraneophobic surface defined by plasma-treated SU-8. The fluorescence micrograph show that fluorescently labeled soy-bean liposomes added into the buffer solution predominantly adhere to the membraneophilic pattern of plasma-treated gold.
   11. k) Chip with membraneophilic plasma-treated Al₂O₃ patterns, made membraneophilic through chemical and/or physical modulation or manipulation, (appear as light-gray in the images) and plasma-treated SU-8 membraneophobic regions (appear dark grey in the images). The circular spots of Al₂O₃ are 50 micrometers in diameter, and the interconnecting lanes of Al₂Oare approximately 2 micrometer thick and 50 micrometers long.
   12. I) Fluorescence image illustrating coverage of lipid confined to the Al₂O₃ pattern, made membraneophilic through chemical and/or physical modulation or manipulation, on one of the microfabricated structures shown in figure 8k. The lipid membrane was stained with a fluorescent membrane dye in order to visualize the spreading. The fluorescence was inverted to clarify the image, dark purple shows stained lipid.

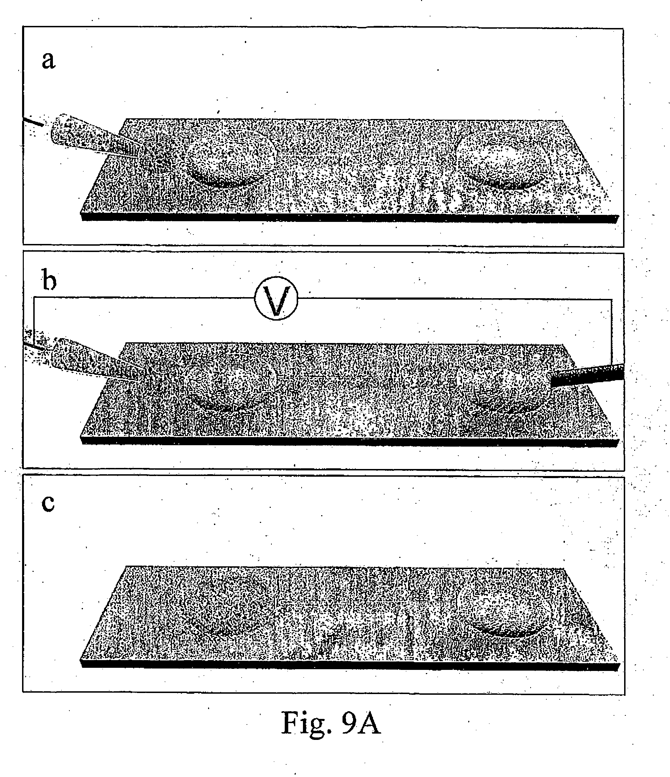

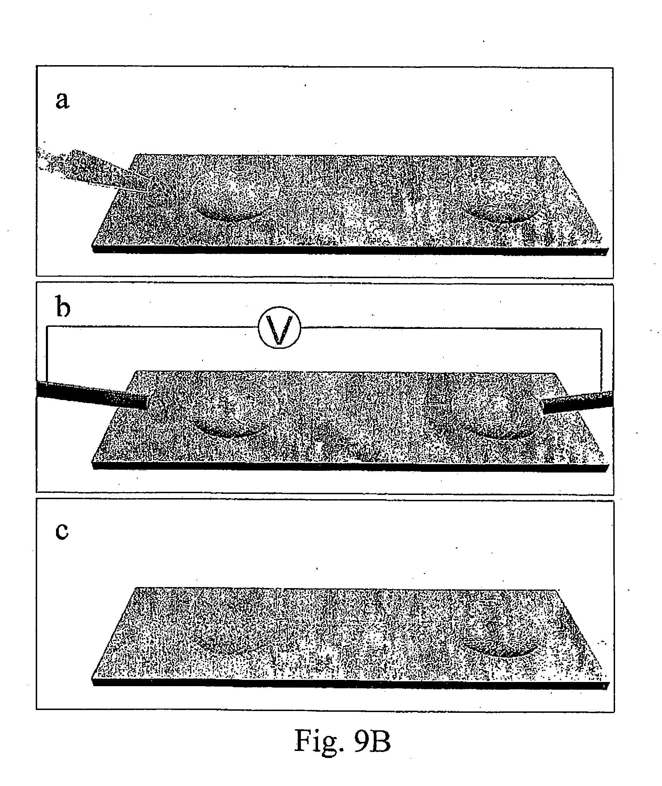

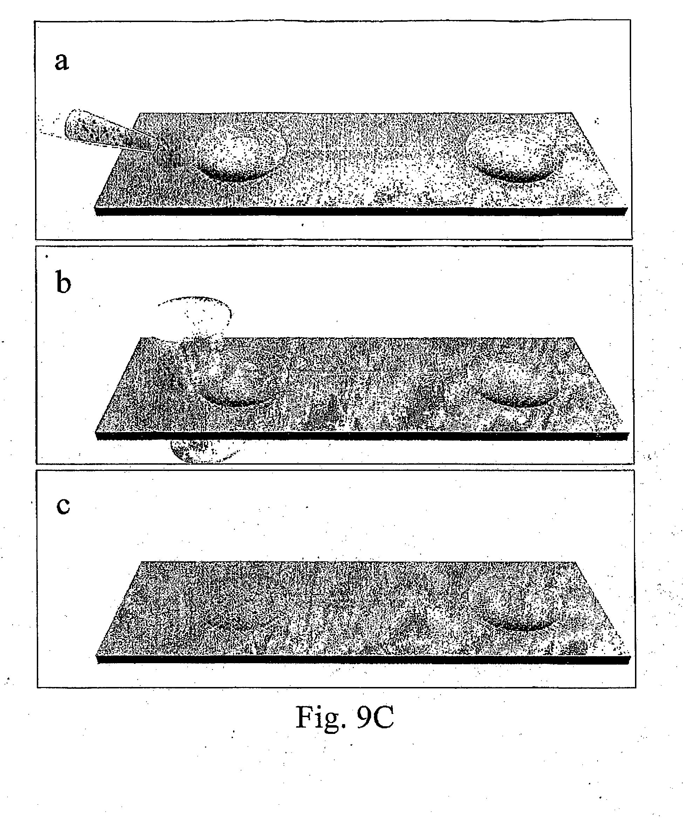

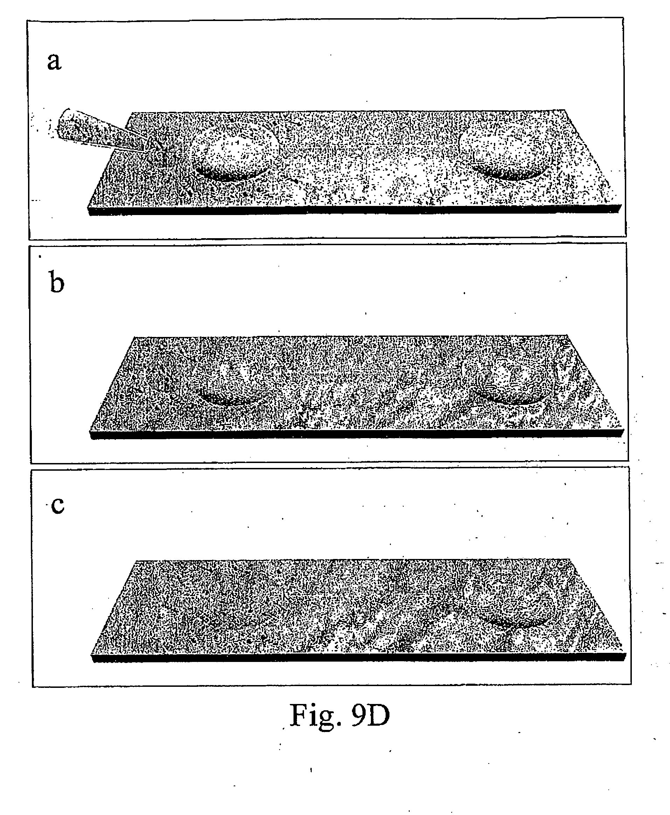

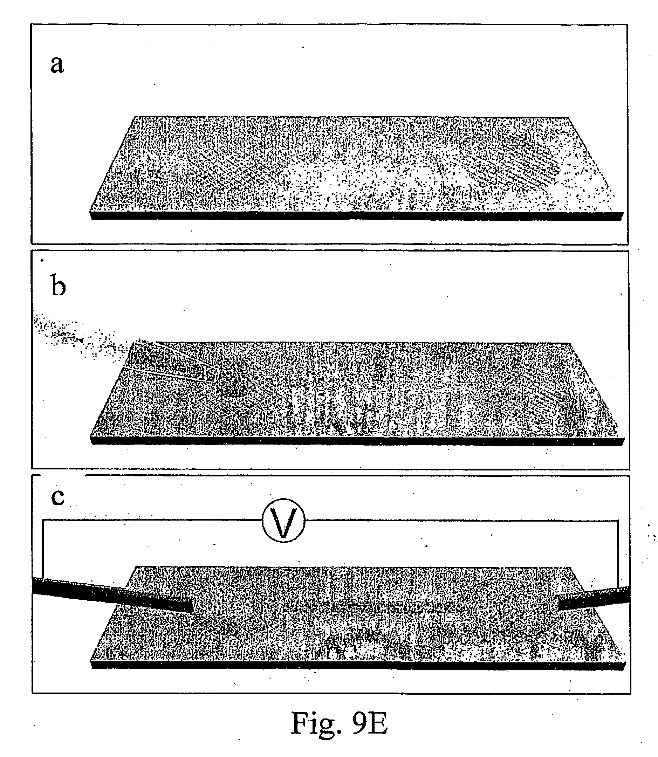

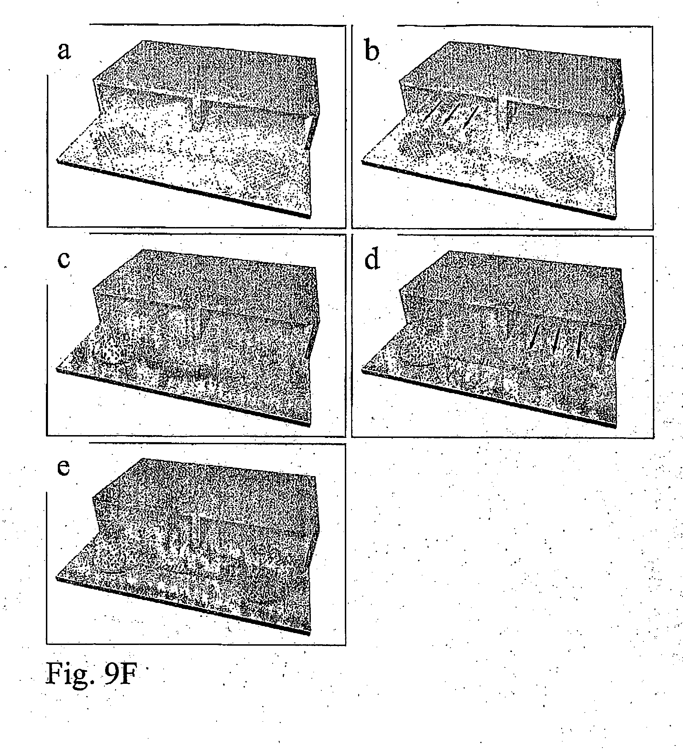

Fig 9. Different methods for introducing biological or synthetic samples to the membrane-covered portion of patterned surfaces. The starting point in all examples given below is a chip structure consisting of a membraneophilic and a membraneophobic area. The membraneophilic area is covered with a lipid bilayer membrane. The bilayer structure needs not to be planar-it can also have a three-dimensional geometry such spherical, tubular, or tubulospherical. Membrane proteins embedded into a lipid bilayer membrane material is then injected or otherwise introduced onto the injection site through various techniques.
   A) Introduction of membranes and membrane-components by electroinjection.
      a) Schematic showing the chip structure containing membraneophilic (covered by a preformed lipid matrix) and surrounding membraneophobic regions. The membrane proteins embedded into a lipid bilayer matrix is ejected from a micropipette tip onto the injection site on the chip.
      b)By applying an electric field between the tip of the pipette and an electrode on the opposite side of the injection site, the two membrane reservoirs merge into one.
      c) This allows the membrane proteins to migrate into the injection region, whereby separation out onto the connecting lanes can be performed.
   B) Introduction of membranes and membrane-components by electrofusion.
      1. a) Schematic showing the chip structure containing membraneophilic (covered by a preformed lipid matrix) and surrounding membraneophobic regions. Membrane proteins in a lipid bilayer membrane matrix are ejected onto the chip, for example by a micropipette, next to the membrane coated surface.
      2. b)Two electrodes are then used to merge the portion of membranes with the membrane-covered injection site.
      3. c) The membrane proteins migrate into the injections site as before and separation can be performed
   C) Introduction of membranes and membrane-components by optical fusion.
      1. a) Schematic showing the chip structure containing membraneophilic (covered by a preformed lipid matrix) and surrounding membraneophobic regions. A lipid matrix containing membrane proteins is first ejected onto the injection site, for example, through the use of a micropipette.
      2. b)A high intensity light is then focused on the border between the two membrane portions (the ejected portion containing the membrane proteins and the membrane-covered injection site on the chip).
      3. c) Optical fusion is then accomplished allowing the two membranes to fuse into one, whereby the membrane proteins migrate into the injection site.
   D) Introduction of membranes and membrane-components by detergent destabilization.
      1. a) Schematic showing the chip structure containing membraneophilic (covered by a preformed lipid matrix) and surrounding membraneophobic regions. As above, a lipid matrix containing membrane proteins is first ejected onto the injection site, for example, through the use of a micropipette.
      2. b)Fusion between the two membrane portions (the ejected portion containing the membrane proteins and the membrane-covered injection site on the chip) is accomplished by for example having a low concentration of detergents in the solution or other chemical agents that promote membrane fusion.
      3. c) After fusion, the membrane proteins can migrate into the injection site.
   E) Introduction of membranes and membrane-components by use of a premixed lipid matrix.
      1. a) Schematic showing the chip structure containing membraneophilic and surrounding membraneophobic regions. The starting point for this method is to have a naked membraneophilic surface, that is, without membrane coverage.
      2. b) The membrane material containing the membrane proteins (the premixed lipid matrix) is then allowed to spread along the membraneophilic surface as in the procedure to form the membrane-covered surface.
      3. c) The membrane proteins are now situated across the entire membrane-coated surface.
   F) Introduction of membranes and membrane-components by use of small vesicles in conjunction with microfluidics.
      1. a) Schematic showing the chip structure containing membraneophilic (which can be covered by a preformed lipid matrix or not) and surrounding membraneophobic regions. The chip can also be integrated with microfluidic channels, where the flow of a channel can be highly focused to a part of the chip, or even parts of the individual designed membraneophilic structures.
      2. b)A solution containing small vesicles (uni- or multilamellar) is flushed cross a part of the designed structure through the use of the microfluidic channels. The vesicles can be made to fuse to pre-form the lipid bilayer matrix by the use of electric fields, so-called electrofusion, or by using so-called fusogenic vesicles that spontaneously fuse to the lipid bilayer matrix.
      3. c) The membranes and membrane-components (such as membrane proteins) have been injected onto the chip. Using this technique, membranes and membrane-components can be injected to specific injection sites on the designed structures. The lipid matrix does not have to be pre-formed for this technique to work. The small vesicles containing membranes and membrane-components can also attach themselves directly to the membraneophilic surfaces. Membranes and membrane-components can also be injected to different structures or even different parts of the same structures by using this technique as similarly described by figure 7E.

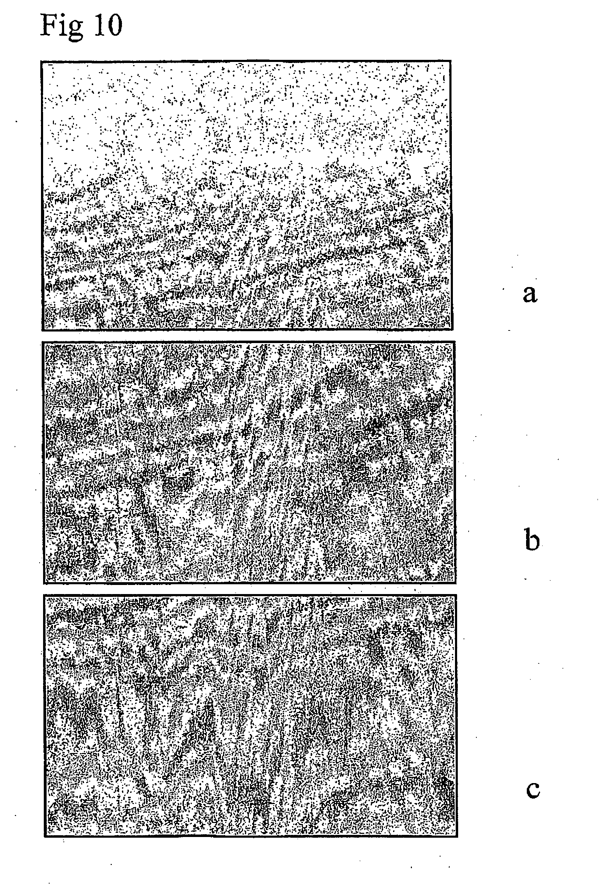

Fig 10. Images illustrating the concept of diffusion as a separation mechanism through the lipid device structures. A structure having a comb-like pattern was used for this experiment and the structure was coated with a continuous lipid bilayer membrane having a fluorescent probe (DiO) integrated into the hydrophobic interior of the lipid bilayer membrane. The lanes had a width of 5 micrometers and the length of the radiating lanes were 25 micrometers. A part of the comb-structure pattern was photobleached and the fluorescence recovery after photobleaching (FRAP) was then followed, The images are color-inverted and the interval between the images is four minutes.
   1. a) In the first image after photobleaching, recovery is noticed at the left and right edges of photobleached area of the image.
   2. b) In the second image after photobleaching, four minutes after the first image, fluorescence show that the DiO-molecules have diffused into the first comb-structure-lanes and continued further towards the center of photobleached area.
   3. c) In the third image after photobleaching, four minutes after the second image, eight minutes after the first image, the DiO-molecules have entered the comb-structure-lanes in the center.
Fig 11. Schematic images showing the use of magnetic beads linked to membrane proteins to achieve separation/extraction of membrane proteins in a lipid bilayer matrix.
   1. a) Schematic image of the starting situation where two different membrane proteins, here denoted by A and B, are located at the injection site of one device structure.
   2. b) By flushing a solution containing magnetic beads which are linked with a specific binding site for a specific membrane protein across the device structure, the magnetic beads attach to the target membrane protein.
   3. c) The magnetic beads have attached to the membrane protein A.
   4. d) By applying a magnetic field across the device structure, the membrane protein A moves along the magnetic field and separates from membrane protein B.

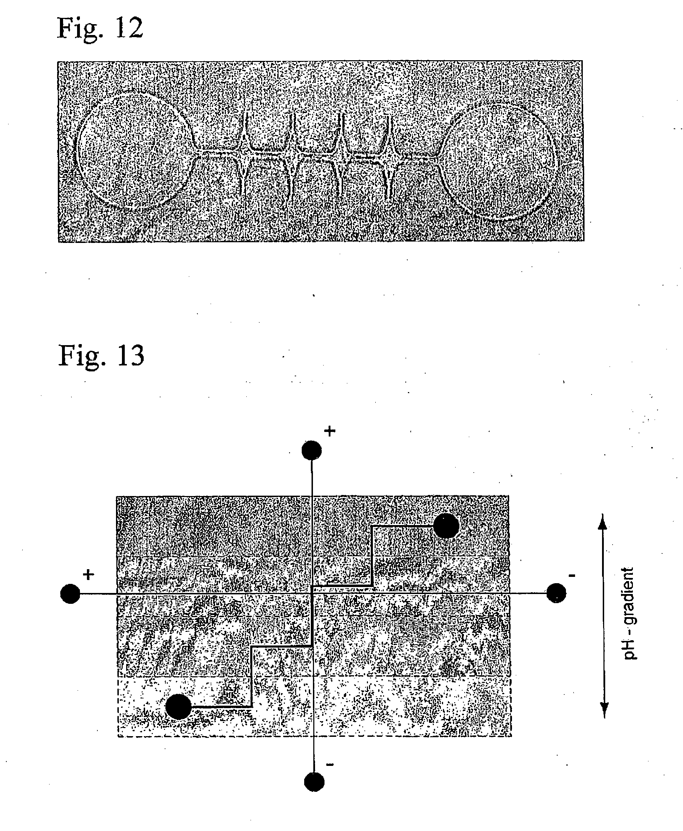

Fig 12. Photomicrograph showing a structure containing two spots having diameters of 15 micrometers, interconnected by a lane having a width of 2 micrometers. The lane is designed to have triangular structures emanating from it. This way fractions along the lane can be collected and focused onto the tips of the triangles, by using a separation mechanism in the second dimension, perpendicular to the first separation taking place along the connecting lane.
Fig 13. Schematic showing separation of membrane-associated species in combination with chemical gradients such as pH-gradients. A pH-gradient can be produced by introducing a structured gel of appropriate material above the chip-structure or by using the chip in combination with microfluidics where several solutions with different pH can be flushed across the device structure.

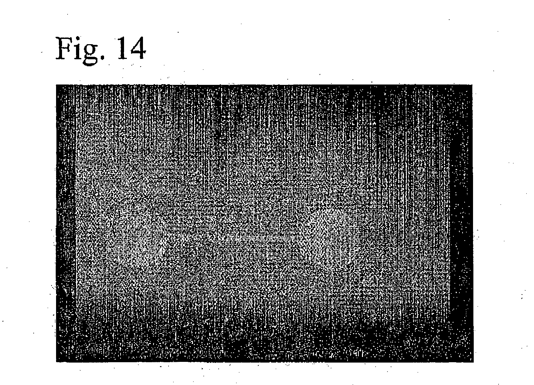

Fig 14. Fluorescence image of streptavidin labeled beta-phycoerythrin linked to the lipid matrix through biotin-labeled lipids. The device structure in this image consisted of two circular spots (diameter 15 micrometer) connected by a single lane 2 micrometers wide. This also illustrates that binding of ligands, antibodies and other reagents can be added after the lipid matrix has been formed. This is especially interesting for applications such as drug screening using microarray protocols for example, where different patches of lipid matrixes containing various membrane protein targets, for example, can be flushed with reagent such as specific antibodies in order to find a specific target protein. Also, this type of approach can be used as a post-labeling procedure after separation of membrane proteins in a lipid matrix, to localize the spots where specific proteins can be found,

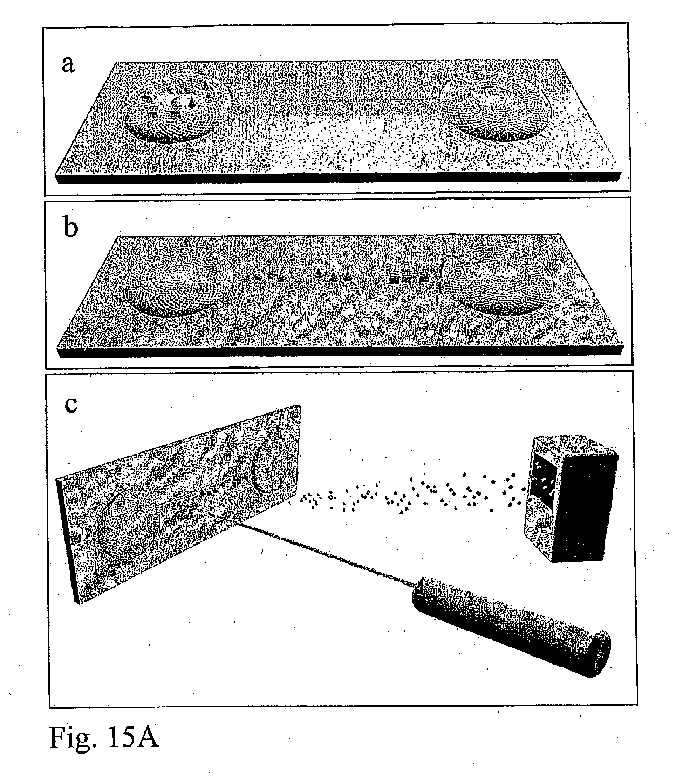

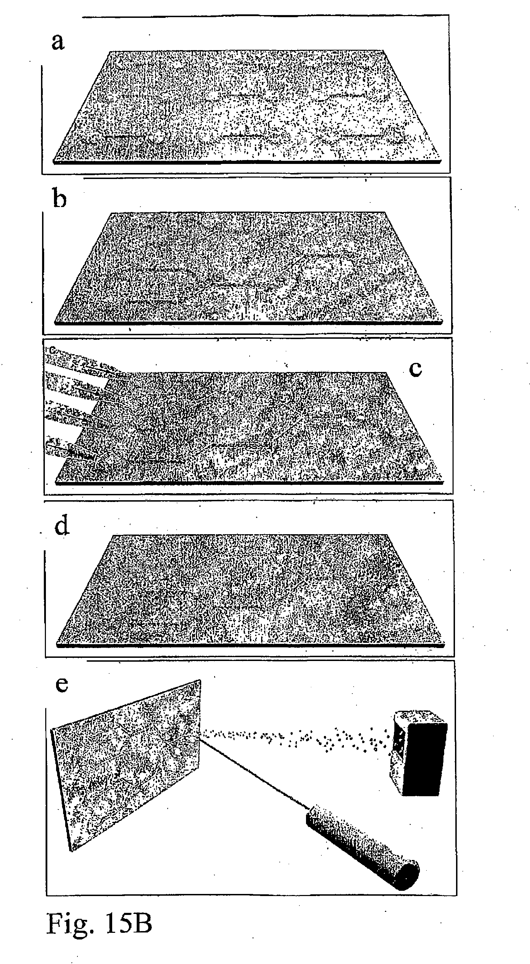

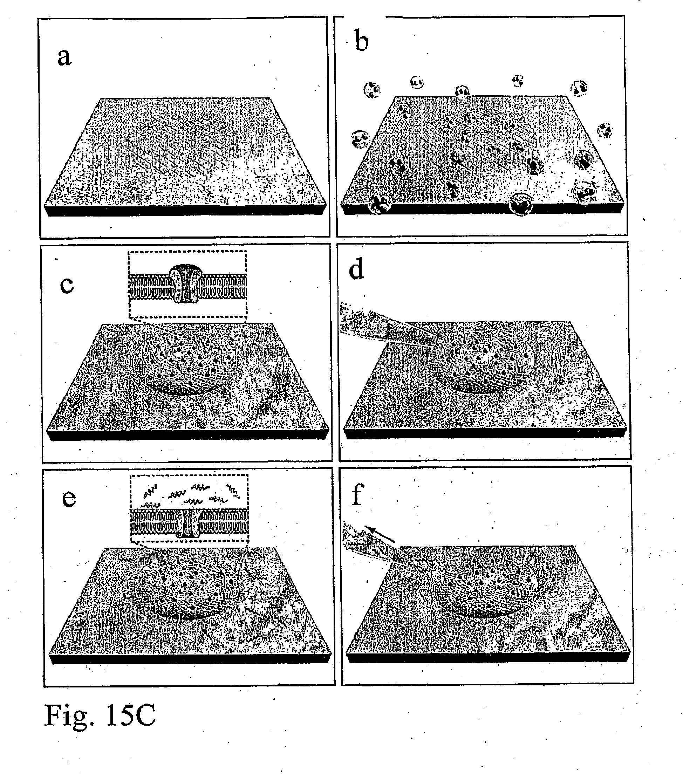

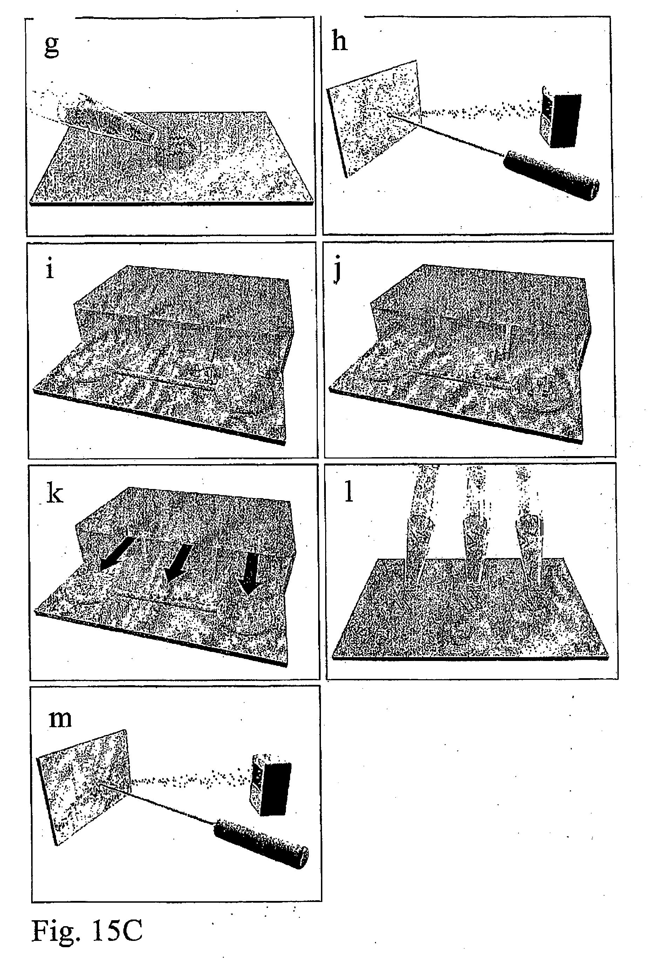

Fig 15. Schematic drawings concerning the integration and use of the chips in conjunction with MALDT-TOF (Matrix-Assisted Laser-Desorption-Ionization - Time-Of-Flight) mass-spectrometry. The structures are scalable in size from micrometers to millimeters and can therefore consist of large single devices or multiple parallel devices, depending on the evaluation protocol, the sample size, sample concentration and detection limit etcetera, when integrating the chips with MALDI-TOF.
   A) Single devices
      a) A single device, where membrane-associated species are confined to the injection site of the lipid bilayer matrix of the device structure.
      b)The membrane-associated species, for example, membrane proteins, are separated using, for example, electric fields, diffusion etcetera.
      c) After separation of these species, the separation can be frozen and the structure analyzed by MALDI-TOF.
   B) Multiple parallel devices
      a) Schematic showing multiple parallel devices, which can consist of one or several devices of the same or different designs, where the separation of membrane-associated species can be performed in parallel.
      b) Schematic showing parallel separation lanes that converge into a single spot. The schematic image illustrates several injection sites.
      c) The membrane proteins are injected into the lipid bilayer matrix of the injection site by techniques described in figure 9.
      d)The membrane proteins are separated using techniques, such as electric fields or diffusion etcetera. The parallel lanes can be used to concentrate a membrane-associated species of low abundance prior to the detection using MALDI-TOF.
      e)After separation, the membrane-associated species have been concentrated into one site, where detection can occur.
   C) Principle of integration with MALDI-TOF-MS (Matrix-Assisted Laser-Desorption-Ionization Mass-Spectrometry) or ESI-MS-MS (Electro-Spray-Ionization Mass-Spectrometry-Mass-Spectrometry) or similar.
      a) Schematic showing the chip containing membraneophilic surfaces surrounded by membraneophobic surfaces.
      b)A solution containing small vesicles (with or without membrane proteins) is placed above the chip. If the vesicles do not contain membrane proteins, the membrane proteins have to be injected into the lipid bilayer matrix using techniques described in figure 10. If the vesicles contain membrane proteins the integration is performed at the same time. The small vesicles settle to the surface and adhere to the membraneophobic surfaces and can be manipulated to stay as vesicles or form a continuous lipid bilayer membrane as described in figure 7Db.
      c) The membrane proteins are attached to the lipid bilayer matrix on the surface (either in intact vesicles or in a continuous lipid bilayer membrane).
      d)The solution above the lipid matrix containing membrane proteins is exchanged to a solution containing digestive agents, for example, proteinase K or trypsin.
      e) The digestive solution will cleave the parts of the membrane proteins that are exposed to the solution and which protrude above the lipid bilayer matrix.
      f) The exposed parts will be cleaved into peptide fragments, which can be collected by pipettes or microfluidic channels. The solution can then be integrated directly into ESI-MS-MS or similar technique either with or without separation steps with techniques such as liquid chromatography (LC) or capillary electrophoresis (CE).
      g)The solution containing the peptide fragments and the appropriate matrix is spotted onto MALDI-TOF plates.
      h)The MALDI-TOF mass-spectrometry technique is then used for identification of the peptide fragments and subsequently of the membrane proteins.
      i) Schematic showing the chip structure containing membraneophilic and surrounding membraneophobic regions. The chip can also be integrated with microfluidic channels, where the flow of a channel can be highly focused to a part of the chip, or even parts of the individual designed membraneophobic structures.
      j) The membrane proteins are separated using different techniques and concentrated on specific parts of the designed structures. Flows containing different digestive enzymes, for example, proteinase K, are then targeted to the different parts of the designed structures. Peptide fragments are released from the lipid bilayer matrix and collected individually.
      k)The collected solutions containing different peptide fragments are then spotted onto MALDI-TOF-plates and analyzed using the MALDI-TOF-MS technique as described in 15H.
Figure 16. Schematic drawings illustrating non-limiting embodiments of the device.
   1. a) Schematic top-view of a device containing a non-limiting number of chambers, in this particular case nine chambers, which can be used in parallel. Each chamber has one inlet and one outlet for adding of material, exchanging of solutions and extraction of material.
   2. b) Enlargement of one of the chamber structures.
   3. c) Enlargement of the walls of the chamber structure illustrating that the surface might have a membraneophilic character and that it might be coated with a continuous lipid bilayer membrane with membrane proteins.
   4. d) Enlargement of the walls of the chamber structure illustrating that de surface might have a membraneophilic character and that it might be coated with a layer of densely packed vesicles with membrane proteins embedded in the lipid bilayer membrane.
   5. e) Enlargement of a membrane protein embedded in a lipid bilayer membrane.
Figure 17. Schematic drawings another non-limiting design of the device above.
   1. a) Schematic top-view of a device containing, in this non-limiting example, nine channel structures, which can be used in parallel. Each channel has one inlet and one outlet for adding of material, exchanging of solutions and extraction of material.
   2. b) Enlargement of one of the channel structures.
   3. c) Enlargement of the walls of the channel structure illustrating that the surface might have a membraneophilic character and that it might be coated with a continuous lipid bilayer membrane with membrane proteins.
   4. d) Enlargement of the walls of the channel structure illustrating that de surface might have a membraneophilic character and that it might be coated with a layer of densely packed vesicles with membrane proteins embedded in the lipid bilayer membrane.
   5. e) Enlargement of a membrane protein embedded in a lipid bilayer membrane.

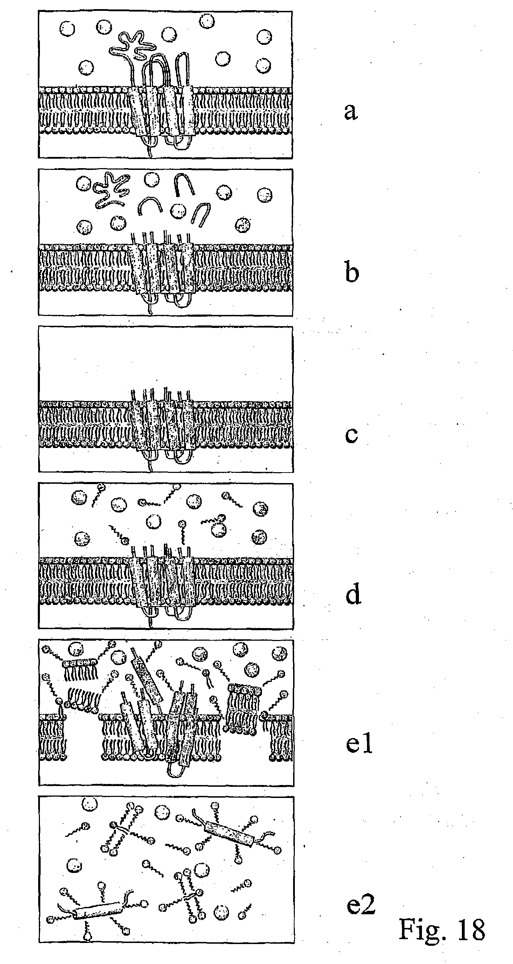

Figure 18 Schematic drawings illustrating the possible two-step on-chip digestion of membrane proteins performed in the examples of devices above.
   1. a) Enlargement of a membrane protein embedded in a lipid bilayer matrix, either in the form of a continuous lipid bilayer membrane or a vesicle, being attached to a membraneophilic surface. Trypsin, or another digestive agent, is added to the solution above the membrane which allows for digestion of the parts of the membrane proteins that protrudes out of the membrane.
   2. b) The formed peptide fragments are collected for further processing and/or detection/identification.
   3. c) This leaves the membrane proteins with the parts that cannot be cleaved by the digestive agents.
   4. d) Detergents, organic solvents or other chemical agents are added to disrupt the membrane and more of the digestive agent is also added.
   5. e) The membrane is disrupted and the lipids form micelles or become stabilized by the detergents. The digestive agent cleaves the remaining parts of the membrane protein into peptide fragments, the hydrophobic parts being stabilized by the detergents, for example. The formed peptide fragments are collected for further processing and/or detection/identification.

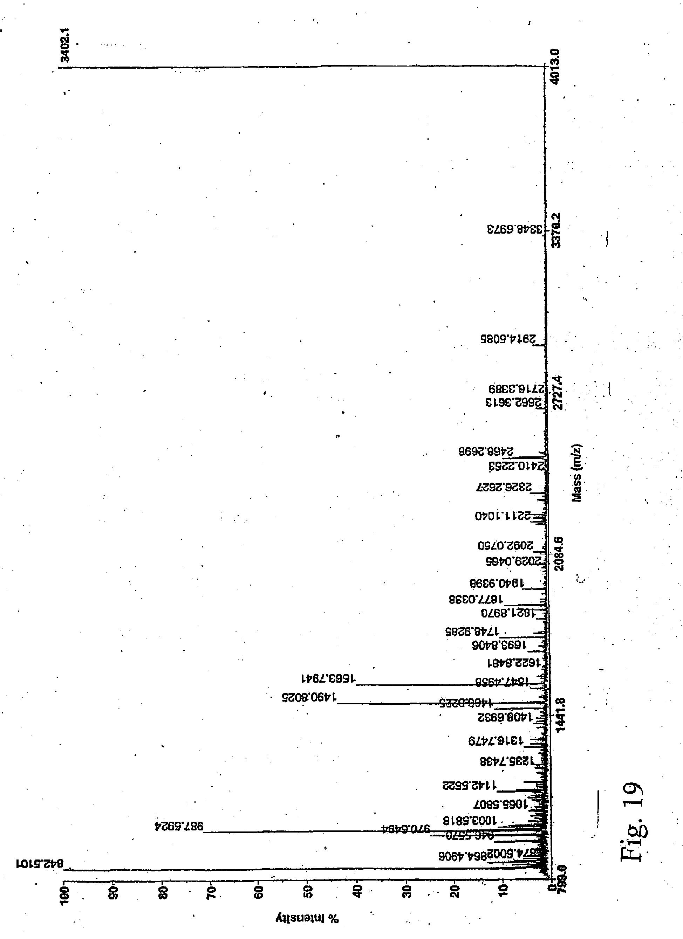

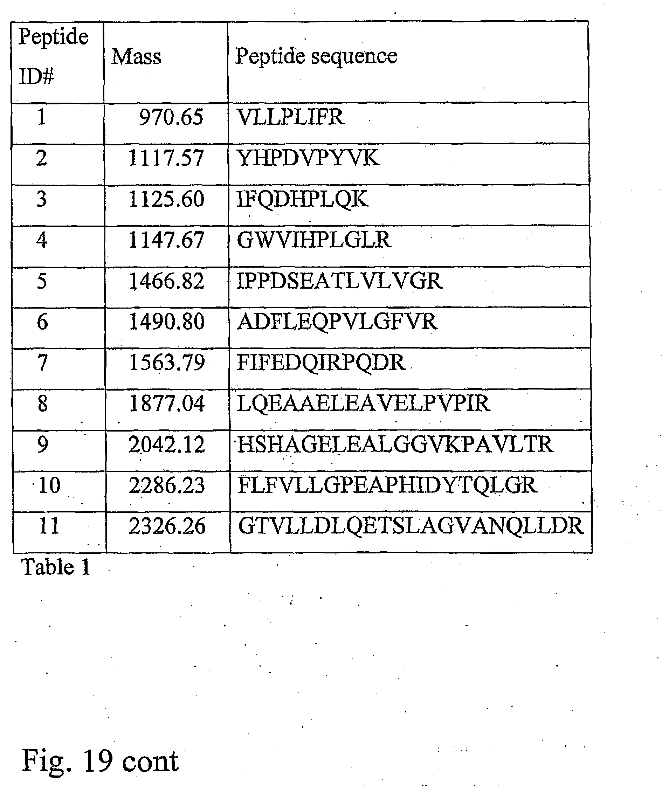

Figure 19. Mass spectrometry trace of peptide fragments obtained from on-chip trypsin digestion of membrane proteins in red blood cells. Peaks with highest intensity were selected and MALDI - TOF in MS/MS mode was performed, the results are shown in table 1. Table 1. Peptide sequences deduced by MALDI - TOF MS/MS mode. The AE1-membrane protein (Anion Exchanger protein) was identified.

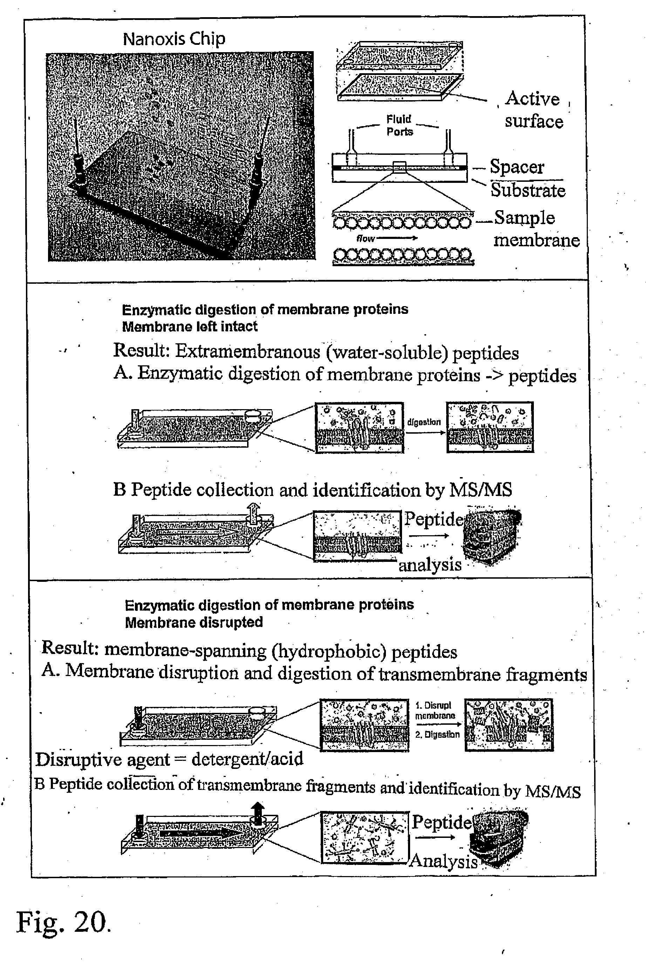

Figure 20A shows a device as described in the present disclosure. An active surface, the membraneophilic surface coats two solid supports. A spacer and fluid ports (with concomitant inlet and outlet holes) forms a flow cell. The sample membrane is allowed to attach to the membraneophilic surface and the surplus material is washed away.
Figure 20B schematically illustrates the enzymatic digestion of membrane proteins, in this case with the membrane left intact. The membrane proteins are situated in the lipid bilayer membrane matrix which is attached to the membraneophilic surface. The device (a flow cell) is filled with a digestive agent, which cleaves the extramembraneous parts of the membrane protein into peptides. The peptides are subsequently collected and analysed, e.g., by mass spectrometry.
Figure 20C schematically illustrates the digestion of membrane proteins, in this case with the simultaneous disruption of the membrane. This step typically follows after the step where the extramembraneous parts have been cleaved off. The device (a flow cell) is filled with a disruptive agent and a digestive agent (may in some cases be left out), releasing the peptides (and a possible further digestive cleavage into smaller fragments). The peptides is subsequently collected and analysed by mass spectrometry.

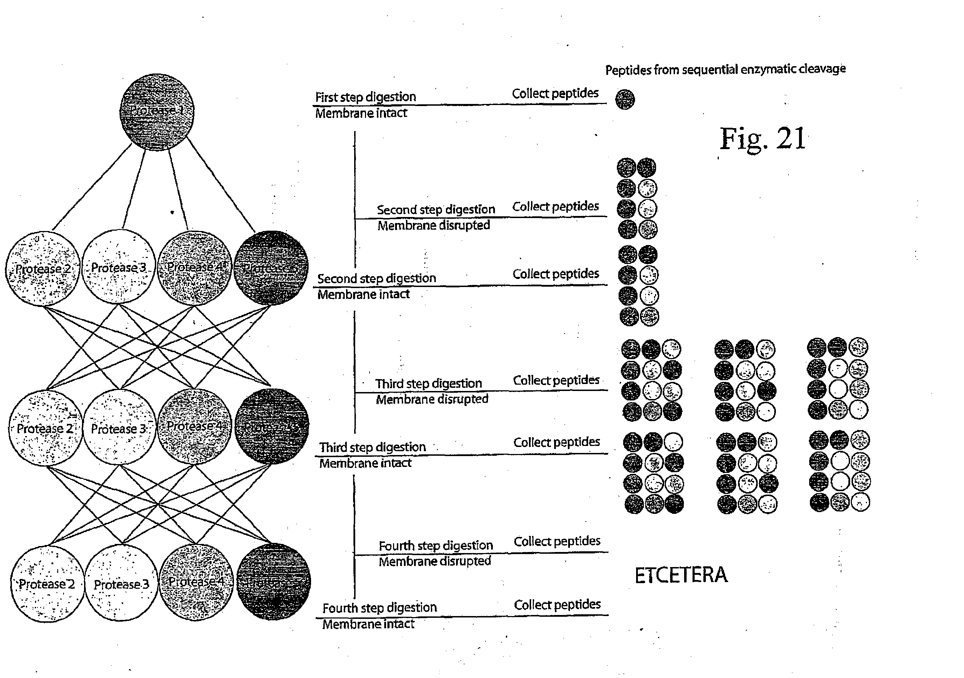

Figure 21 schematically illustrates the concept of sequential digestion, using any number and any combination of digestion cycles. Any number and any combination of different digestive agents or treatments, e.g., enzymes, chemical compounds, any wavelength of electromagnetic radiation, sound at any frequency or exposure to certain temperatures or pressures (illustrated and exemplified here by the different coloured circles). The example shows enzymatic digestion with proteases that can be used sequentially (or in parallel) to obtain different cleavage patterns. In this case the first step digestion is performed with protease 1 and the membrane left intact. The second step digestion is performed by any of the other four proteases, with the membrane either left intact or disrupted. Four different cleavage patterns will arise for the membrane left intact. Since new cleavage sites are exposed during membrane disruption, another set of four cleavage patterns will arise for the digestion performed with disruption of the membrane. Further digestion steps can then be added to the protocol.

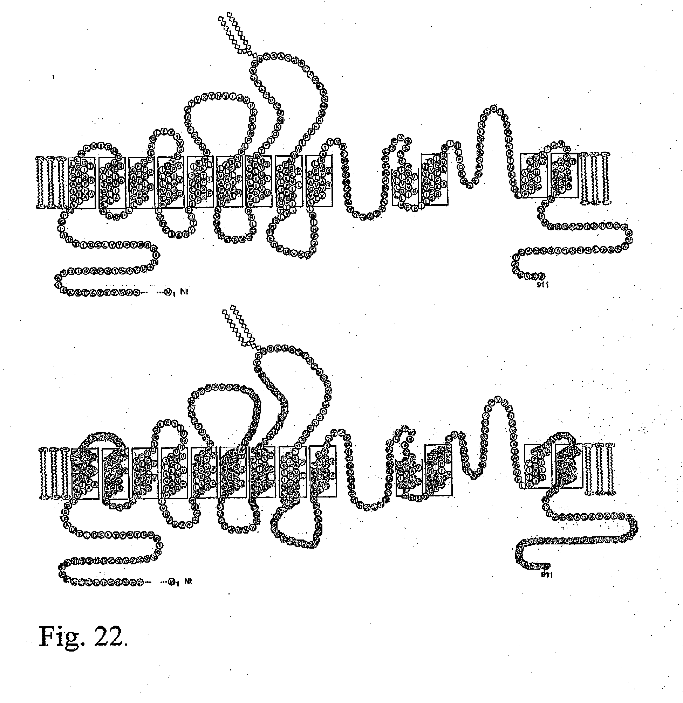

Figure 22 illustrates an example showing the principle of sequential digestion with the aim for sequence coverage studies. Top image: Snakeplot (two-dimensional view of the amino acid sequence with predicted transmembrane, cytosolic and extracellular parts) of the Band 3 Anion Exchanger Protein in the erythrocyte membrane preparation, RBCM. The green dots illustrate the peptides arising from trypsin digestion. Bottom image: Snakeplot showing the result of a sequential digestion of trypsin followed by pepsin digestion. The additional blue dots illustrate the peptides arising from pepsin digestion.

### Detailed description of the invention

Lipid bilayer membranes can adopt certain shapes and geometries. **Spontaneous** shapes found at equilibrium conditions are limited in topology, phase, and geometry. We refer to the Helfrich equation for solutions of shapes as a function of certain key system parameters such as bending modulus, stretching modulus, spontaneous curvature, *etcetera.* Lipid bilayer membranes are thermotropic liquid crystals and behave as 2-dimensional fluids above the transition temperature. Liquid crystalline media is extremely interesting as a solvent for certain molecules such as membrane proteins and other amphiphilic, and lipophilic molecules. However, due to limitations in controlling this fluid, devices are difficult to make using liquid crystalline media for technological applications. Bilayer membranes are interfacial fluids. As such they always exist at boundaries or define boundaries. Membranes can be forced into energetically unfavorable structures using different methods. The resulting structures are thus kinetically arrested, and might have features as extremely high edge energies. Note that by lipid bilayer membrane we do not restrict ourselves to any particular structure of the lipid bilayer membrane. It might thus be a spherical vesicle, a planar bilayer of the Langmuir-Blodgett type, or it may be a tubular structure, or a structure of other type. We also do not restrict the methods according to the invention to any particular type of lipid phase.

One example of energetically strained geometries is lipid membrane vesicles conjugated by nanotubes or thin tethers (Karlsson et al. Nature, 409, 150-152 (2001)). These structures can only exist for prolonged periods of time when the vesicles are immobilized on a surface. Would the vesicles be released from the surface, the system would immediately collapse into one single sphere.

The present disclosure presents ways of controlling the shape of lipid bilayer structures on surfaces. In particular, we have developed methods in which we design surfaces on essentially planar substrates with certain characteristics, e.g. chemical or topographical that upon interaction with lipid bilayer membranes (or constituents that form such membrane assemblies) form conjugate structures with controlled end-point topographies and geometries. The underlying idea is to control e.g. the spreading or formation of a bilayer membrane on a surface in such a way that the resulting bilayer structure has a form that is useful for solving a technological problem. One such non-limiting problem might be to have the ability to separate and analyze membrane proteins, and furthermore, to have the ability to functionally probe proteins, *e.g.* for protein-drug interactions, in a dynamic fashion.

We start by looking at different surfaces and their preparation. The membraneophilic region, designed to capture membranes and membrane material of any type, may be formed of any membraneophilic surface of any shape or geometry or surface made membraneophilic through chemical and/or physical modulation or manipulation. These surfaces are going to be used for creating areas predominantly carrying bilayer membrane structures with some useful geometries, in particular, for separating and manipulating membrane proteins. In the simplest case we wish to fully cover a surface with a bilayer membrane. In the next case we wish to define a line or a plane on a surface covered by lipid bilayer membrane. This membrane-covered area should be in at least one planar direction (i.e. not in the normal extension) and preferably be surrounded by area not covered by bilayer membrane. Thus the two areas should have different surface properties or physical boundaries separating them. In the simplest scenario, we control this differential adsorption to bilayer membranes by controlling the adhesion of membrane to said surfaces. In the case when the lipid is charged and hydrophilic, preferably, the surface area to be covered with membrane may also be charged and hydrophilic. There are a number of chemical and physical parameters that can be used to control the interaction potential ranging from Van der Waals, hydrophobic, electrostatic, π-π interactions, hydrogen bonding and covalent interactions that can be used for this purpose. Some interactions might be static in nature whereas other might be dynamic. Examples of dynamic interactions are electrowetting, magnetic, and electrical forces.

Lipid bilayers can be adsorbed to surfaces using different methods. For, example, there are reports on continuous bilayer formation from collapsing of small unilamellar vesicles on silicon dioxide, and formation of Langmuir-Blodgett films (Reimhult et al. J. Chem. Phys. 117, 7401-7404 (2002), Steltze et al. J. Phys. Chem. 97, 2974-2981 (1993)). According to the present disclosure, several techniques are proposed for deposition of lipid onto the designed structures (see figure 7). One technique involves controlled deposition of multilamellar vesicles using e.g, a pipette-transfer technique. These vesicles contain excess membrane material, and a single 10 µm-diameter multilamellar vesicle can contain 1000 sq. micrometers of lipid membrane. This excess membrane can be donated to a surface under favorable conditions, as will be explained below.

In a simple case, membrane adhesion to a substrate can be controlled using hydrophobic interactions. A planar substrate is provided with regions that favor lipid bilayer membrane association in an aqueous environment. Thus in the typical case we will create a system consisting of a layered structure having *a*) a solid support *b*) an interfacial liquid crystalline material (e.g. lipid bilayer film), and c) a liquid most typically water. The planar substrate has regions where lipid membrane prefers to spread and regions unfavored by membrane association and spreading, Examples of such surfaces are plasma-treated gold or Al₂O₃-surfaces, made membraneophilic through chemical and/or physical modulation or manipulation, and surfaces covered by a plasma-treated photoresist SU-8, respectively. These combinations of surfaces work well for soy bean lipid membranes, and neutral lipid membranes such as phosphatidylcholine, as well as cellular membranes, such as most eukaryotic cell membranes. Also substrates can be made fully covered by Al₂O₃ or gold, made membraneophilic through chemical and/or physical modulation or manipulation, and thus be used to create the corresponding surfaces fully covered with membranes in the form of vesicles or continuous bilayer films. There are many other specific or non-specific surface interactions that can be used for creating patterns of bilayer membranes on surfaces. Surface preparations including self-assembled monolayers, and various tools such as etching, vapor deposition, spin coating etcetera can all be used for this purpose. Surfaces can be created with different features as known in the art of micro- and nano-fabrication. The surfaces can be made membraneophilic through chemicals and/or physical modulation or manipulation Examples of different surfaces produced by such techniques are given below, and furthermore processing techniques to produce such surfaces are given in the experimental section.

For some purposes and applications, the surface can only have membraneophilic properties (figure 1), for example, gold or Al₂O₃-surfaces, made membraneophilic through chemical and/or physical modulation or manipulation. These membraneophilic surfaces can be covered either with intact vesicles (for example small unilamellar vesicles SUVs) or a continuous lipid bilayer membrane. Several applications might be realized from this technology. Membrane proteins can be reconstituted into these lipid systems and techniques, such as microarrays or plate readers, can be used in conjunction with, for example, fluorescence-based detection techniques in order to study ligand binding to target proteins, such as ion channels or G-protein-coupled receptor proteins (GPCRs). This could be performed by having, for example, an ion channel of interest in the lipid bilayer membrane system (either in the form of intact vesicles or a continuous lipid bilayer membrane) and a reporter probe on the inside of the lipid bilayer membrane. The probe should report changes in concentration of the ion which the channel specifically allows through when a ligand is attached to it. Ligands, which are potential drug candidates, can thus be screened using this technique, for example, through an increase in fluorescence. Upon binding, the ion channel opens and allows the ion for which it is specific to enter through the lipid bilayer membrane. The reporter probe thereby fluoresces and a signal can be observed. Another technique which can be used for this purpose is based on surface plasmon resonance (SPR) detection, a technique which detects changes in mass that is attached to the chip surface. Ligands bind to membrane proteins in the lipid matrix and an increase in mass on the chip is thereby observed. The membraneophilic surfaces can also be used for attaching ribosomes in a lipid bilayer membrane matrix, either in the form of intact vesicles or a continuous lipid bilayer membrane. This can, for example, be used for cell-free synthesis of membrane proteins. Amino acids and the coding genes needed for translation by the ribosomes as well as other materials or agents are added to the immobilized ribosomes in the membrane matrix. The coding genes are translated into the membrane protein of interest, which is subsequently inserted into the membrane matrix (a fact which is very important, since membrane proteins cannot be synthesized in a water-based environment). Once a high concentration of the membrane protein of interest has been acquired, the proteins can be harvested for further analysis. Functionality studies and so on of newly synthesized ion channels, GPCRs and other proteins might even be performed directly on the chip where the membrane proteins were synthesized. Another study which can be performed using the ribosomes in a lipid matrix on a surface is the coupling between the genome and the proteome, to investigate, what genes codes for what proteins. The genome is translated into a protein that might have an unknown function and when a sufficiently high concentration has been reached, functionality studies can be performed. Also, the proteins can be harvested for identification and analysis using for example mass spectrometry. This can be done in a number of ways. For example, a digestive solution (containing, for example, enzymes such as trypsin) can be added to the solution, cleaving the parts of the membrane proteins which protrude outside the lipid bilayer membrane, thus forming peptide fragments from the membrane proteins. The digestive solution can also contain detergents or organic modifiers in order to disrupt the membrane matrix and denature the proteins, which allows also transmembrane peptide fragments to be formed from the digestive agents. The peptide fragments are collected and analyzed by for example matrix-assisted laser-desorption-ionization - time of flight (MALDI -TOF) mass spectrometry (see figure 15 and adjoining text paragraphs for further details).

For other purposes and applications, the surface can be structured, using micro- and nanofabrication techniques, in order to create surfaces having both membraneophilic and membraneophobic properties. Thus, a line, a plane, or a plane with particular surface topography or combinations of different surface properties can be made. For example, areas that promote adhesion might be surrounded by other areas not supporting lipid spreading. Examples of different surface patterns are shown in figure 1. Figures 1d and 1e show schematic drawings of planar surfaces with membraneophilic and membraneophobic domains, respectively. In figure 1d, two membraneophilic islands are connected by a single membraneophilic lane and in figure 1e two membraneophilic islands are connected by three membraneophilic lanes. Figures 1f and 1g show the corresponding images of figure 1d and figure 1e, respectively, where the membraneophilic regions are covered by lipid bilayer membranes.

Figure 1h and figure 1i show schematic drawings of a planar surface with membraneophilic and membraneophobic domains, respectively and surface topography. Such structures with recessed topography can be produced by methods of microfabrication. Again, it is intended for use in combination with lipid bilayer membranes that spread on the membraneophilic supports, and figure 1j and 1k show the corresponding images after lipid film growth or adhesion. Figure 11 shows one example of a surface with membraneophilic/membraneophobic domains and surface topography where the membraneophilic domains are placed on the top surface of the elevated structures. Such structures with elevated topography can be produced by methods of microfabrication. In this particular instance two elevated circular pillars are connected by a single lane. Figure 1m shows an example of a surface with membraneophilic and membraneophobic domains, respectively and surface topography where the hydrophilic domains are placed on the top surface of the elevated structures. In this particular instance two elevated circular pillars are connected by three lanes. Planar supported membranes covering the hydrophilic but not the hydrophobic surfaces in figure 11 is shown in figure 1n and planar supported membranes covering the membraneophilic but not the membraneophobic surfaces in figure 1m is shown in figure 1o. Please note the suspended planar membranes interdigitated between the three lanes. As can be seen, this technique can be used to deposit an essentially planar membrane to a surface having different distances from the supporting substrate because of its surface topography. The difference in height of these structures, that is, the thickness of the SU8 layer on top of the Al₂O₃ surface, made membraneophilic through chemical and/or physical modulation or manipulation, can range from nanometers to millimeters.

Figure 1p shows vesico-tubular membrane structures on similar surfaces as in figures 1d, 1h, and 1l. These are nanotube-vesicle networks produced according to procedures described in EP1322546. Thus, the method is not limited to deposition of essentially planar membranes. There are several degrees of freedom in forming a particular pattern which is ultimately limited by the methods of microfabrication and surface deposition/surface chemistry used.

All surfaces can be used to immobilize small vesicles. There are a number of other specific or non-specific interactions that can be used to tailor a surface. This was alluded to above in the text, and reference can be made to, for example, chemically patterned surfaces where the areas to be covered by lipids contain a streptavidin moiety and the lipid contain a biotin moiety. Other examples might include lectin-coated areas and the lipid membrane can carry sugar (or proteins with such sugar residues that bind to the lectin-coated areas. Other strategies might include using SAMs (self-assembled monolayers) to create surfaces of particular interest. Thus, given the chemical and physical diversity of lipid bilayers, we use membraneophilic and the word membraneophobic to emphasize the generalization of the generic concept. The most important issue is that these methods allow you to form differentially lipid-membrane-coated surfaces of particular designs and feature sizes to yield optimized structures for technological applications such as membrane-protein separations. Also it is important to mention that the method is not limited to lipid-bilayer forming surfactants in different forms i.e. planar membranes, vesicles etc., but can include other surfactant or emulsion systems, including micelle-forming systems, oils, sponge-phase and lyotropic lipids etcetera. Also, in some cases, microchips can be made where the local composition of lipids in different phase states are coexisting on the same surface.

The patterned surfaces having differential membraneophilic and membraneophobic properties might also be used as a platform for cell culturing. This way populations or single cells might be grown in different spots. Connecting lanes might, for example, provide sites for where synaptic coupling between cells might occur.

Seven non-limiting examples of different substrates including three-dimensional gray-scale substrates having membraneophilic and membraneophobic regions are shown in figure 2a-2m with the corresponding substrates having the membraneophilic regions covered by lipid bilayer membrane. These are shown to illustrate that there is a great degree of freedom in designing these devices. The structures are scalable and a reference to length scale is not given in the figures. However, the smallest feature size, *i.e*. smallest lane that can be formed, is on the order of 5 nm and the largest that can be made are of macroscopic dimensions. Figure 2a shows a membraneophilic meander-boarder type of pattern on a membraneophobic substrate and 2b shows the surface in 2a covered by a bilayer membrane. Figure 2c shows a membraneophilic serpentine type of pattern on a membraneophobic substrate and figure 2d shows the same substrate covered by a bilayer membrane. Figure 2e shows a membraneophilic pattern on the upper surface of a 3-dimensional membraneophobic pyramid-shaped substrate and figure 2f shows the same substrate covered by a bilayer membrane. Figure 2g shows a membraneophilic pattern on the upper surface of a 3-dimensional membraneophobic step-pyramid-shaped substrate and figure 2h shows the same substrate covered by a bilayer membrane. Figure 2i displays a membraneophilic pattern on the upper surface of a 3-dimensional membraneophobic serpentine-shaped substrate and figure 2j shows the same substrate covered by a bilayer membrane. Figure 2k shows a membraneophilic circular serpentine labyrinth type of pattern on a membraneophobic substrate and 21 shows the same substrate covered by a bilayer membrane. Figure 2m shows a membraneophilic square-shaped serpentine labyrinth type of pattern on a membraneophobic substrate and 2n shows the same substrate covered by a bilayer membrane. These are all non-limiting examples.

Additionally, several structures can be placed on a single chip surface to create parallel or multiplexed devices. Either it can be a plurality of the same structure design or it can be several different substrate designs on the same chip. Figure 3 shows non-limiting examples of different chips having a plurality of substrate features. Figure 3a shows arrays of sixteen identical membraneophilic patterns consisting of two circular spots connected by a single lane on a membraneophobic substrate. Figure 3b shows arrays of sixteen identical membraneophilic patterns consisting of two circular spots connected by three lanes on a membraneophobic substrate, Figure 3c shows arrays of sixteen identical membraneophilic patterns consisting of two circular spots connected by a single lane arranged in a serpentine pattern on a membraneophobic substrate. Figure 3d shows arrays of sixteen identical membraneophilic patterns consisting of two circular spots connected by a single lane arranged in a labyrinth pattern on a membraneophobic substrate. Figure 3e shows arrays of sixteen membraneophilic patterns of two different types consisting of i) two circular spots connected by a single lane arranged in a serpentine pattern and ii) two circular spots connected by three lanes on a membraneophobic substrate. Figure 3f shows arrays of sixteen membraneophilic patterns of two different types consisting of i) two circular spots connected by a single lane arranged in a labyrinth pattern and ii) two circular spots connected by three lanes on a membraneophobic substrate. The chips can furthermore have auxiliary features such as electrodes or microfluidic channels to support a particular functionality as described below. The microchips described above can have specific features such as additional structures and properties allowing for manipulation of materials dissolved in the lipid film, or the lipid film itself. One such feature is electrodes that *e.g,* can be used to electrochemically alter the lipid film or materials contained in the lipid film or to create electric fields that can be used to *e.g.* drive materials transport through electromigration methods such as electrophoresis, electroosmosis, and dielectrophoresis. Chips can carry one or several electrodes and the electrodes might be integrated in the chip surface or the electrodes can be probes that are placed at certain locations on the microchip. Electrodes can be used to drive electromigration of membrane or membrane-associated materials such as membrane proteins.

Different chips having membraneophobic and membraneophilic regions and auxiliary electrodes are shown in figure 4A-K. Figure 4a shows six structures having spiraling lanes with a central end-point and a peripheral start-point. Electrodes are connected to the two spots for application of electric fields. Figure 4b displays a chip with six identical membraneophilic regions consisting of two spots connected by three lanes. Electrodes are connected to the two spots for application of electric fields. Figure 4c shows a chip with six identical membraneophilic regions consisting of two spots connected to seven lanes. Electrodes are connected to the two spots for application of electric fields. Figure 4d is a chip with one pattern having membraneophilic regions consisting of two spots connected to a bifurcating network of lanes. Electrodes are connected external to the two spots for application of electric fields. Electrodes are also connected at each bifurcation point of the network for application of electric fields. Figure 4e displays a chip with one pattern having membraneophilic regions consisting of two spots connected to a bifurcating network of lanes. Electrodes are connected external to the two spots for application of electric fields. Electrodes are also connected at each bifurcation point of the network for application of electric fields. Figure 4f is a circular membraneophilic region connected to two embedded electrodes. Figure 4g shows a circular membraneophilic region placed proximal to two external electrodes. Figure 4h displays a chip having circular membraneophilic region with one central and six peripheral electrodes. Figure 4i shows a circular membraneophilic region placed in a quadrapole electrode arrangement, and figure 4j shows a chip with a circular membraneophilic region placed in an octapole electrode arrangement. Figure 4k shows how external electrodes can be used to switch the electric field so that separation can take place in two or more dimensions.

The electrodes can either be integrated on the chip structure or be external and create focused or homogeneous fields and be used to direct (switch) e.g. migrating proteins to a particular lane in the crossing.

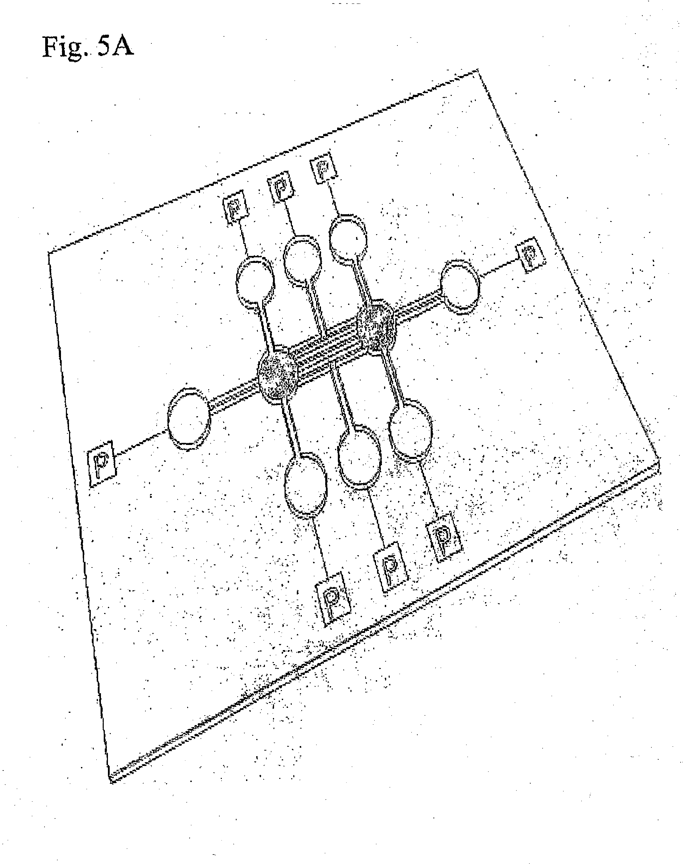

The chips can also be combined with microfluidic channels of different designs. The channels can be directly integrated with the substrate (single-layer devices) or be parts of a separate layer (multilayer devices) bonded to the substrate containing the membraneophilic and membraneophobic patterns, respectively. The channels can be used to direct membrane proteins or different reagents such as dyes or drugs or other agents that might affect the chemical or physical properties of the lipid bilayer membrane or materials including membrane proteins contained in the bilayer membrane either locally (a certain location on the chip or on the lipid film) or globally *i*.*e*. affecting the whole of the chip or the whole of the lipid film in singular or plural devices. Microfluidic channels can also be used to build the membrane films on the membraneophilic areas by transport of membrane-lipids or vesicles. One non-limiting design of a microfluidic chip is shown in figure 5a. This chip has recessed membraneophilic regions consisting of two spots connected to three membrane-covered lanes. The two outer lanes, and the two spots are connected to microfluidic channels that in turn are connected to reservoirs. The microfluidic delivery is in this instance controlled by pressure (P). In general, the channels can have different dimension and geometries and can be either integrated on the chip or supplied externally. In one aspect the channels are produced in PDMS, PMMA, PC, or other rubber or plastic material. Chips with containers can also be made. The containers may be connected to the microfluidic channels or can be used separately as storage devices for reagents, drugs, proteins etcetera.

Patterned substrates having separate membraneophilic and membraneophobic regions provided by the different techniques mentioned above and further detailed in the experimental section, and with the particular properties mentioned above need to be coated with the appropriate lipid bilayer film, including vesicles, or other suitable surfactant film using either chemical or physical methods of deposition. Typically, the substrates are used in water solution or water-based solution such as physiological buffers. Below we will go through some different techniques to introduce and deposit lipids to the surfaces. First we describe the lipid spreading properties of soy-bean multilamellar liposomes on two different patterned model surfaces consisting of plasma-treated gold or Al₂O₃, made membraneophilic through chemical and/or physical modulation or manipulation, surrounded by SU-8 (figure 6). Figure 6a is a plot showing spreading of soy-bean lipids on a gold-plasma treated surface, made membraneophilic through chemical and/or physical modulation or manipulation, versus time. The initial area of the lipid spot is 0.1x10⁴ µm², and after 4.8s, the lipid area occupies 1.8x10⁴ µm². The lipid was originally deposited on the surface as a multilamellar liposome. Figure 6b displays soy-bean lipid spreading on a plasma-treated gold surface, made membraneophilic through chemical and/or physical modulation or manipulation, as seen through a microscope. The time point for the left image is t=0.4s after the liposome was deposited and the second image represents t=5.2s after the liposome was deposited. These pictures correspond to first and the last points in the graph displayed in figure 6a. In figure 6c, a plot showing spreading of soy-bean lipids on a SU8-plasma-treated surface versus time is shown. The initial area of the spot was 327 µm², and after 13 minutes the lipid material shows no spreading. An apparent decrease of the lipid area is due to photo bleaching. The lipid was originally deposited on the surface as a multilamellar liposome. In figure 6d, fluorescence microscopy images show spreading of a soy-bean liposome on a SU8-plasma treated surface with a time interval of 13 minutes. These pictures correspond to first (left image) and the last (right image) points in figure 6c. There are several possible ways of introducing and depositing lipid bilayer membrane material to membraneophilic regions of patterned surfaces. In one aspect lipid membrane material can be provided to the chip substrate by deposition of multilamellar or unilamellar vesicles to the membraneophilic regions using micropipettes or other transfer tools. A scanning stage can be used for translation of the substrate, *e.g.* a motorized and computer-controlled scanning stage. The micropipette action can also be robotically controlled. The procedure is outlined in figure 7a. A multilamellar liposome produced *e.g.* by protocols described in (Karlsson et al. Langmuir, 17, 6754-6758 (2001)) is partly or fully injected into the orifice of a glass micropipette (see *e.g*. Sott et al. Langmuir, 19, 3904-3910 (2003)). The micropipette is preferably controlled by micropositioners such as high-graduation micromanipulators (Narishige MWH-3, Tokyo, Japan is one non-limiting example of such a micromanipulator). The multilamellar vesicle is then brought in contact with the membraneophilic region of the substrate. As the liposome contacts the surface it starts to spread on the membraneophobic region until it is partly or fully covered by lipid membrane. The particular structure of the lipid film depends on the particular type of liposome used and can be uni or multilamellar and with different compositions of lipids. The lipid membrane will in many instances using this lipid deposition technique not be a perfect bilayer membrane such as a Langmuir-Blodgett film. The creeping and spreading behavior of lipid films from multilamellar liposomes is caused by the high binding affinity the membrane material has for the membraneophilic surface which exceeds the energy required to maintain the initial spherical liposome shape. Alternatively, multilamellar liposomes might be deposited to membraneophilic regions using optical tweezers as displayed in figure 7b. Optical tweezers can capture multilamellar liposomes in water solution and move them to target areas. Optical tweezers can also capture unilamellar vesicles provided that the vesicles are filled with a material of higher refractive index than the surrounding solution.

Deposition of lipid membranes to membraneophilic regions' can also be obtained using a pipette-writing technique by translation of a pipette filled with lipid material across a surface. This technique is described in Scott et al. Langmuir, 19,3904-3910 (2003), for formation of vesicle-nanotube networks, and can be used to transfer lipid material from multi or unilamellar liposomes. The procedure is outlined in figure 7c. At least three different resulting structures can be obtained here as in the above examples a) vesicles with suspended tubular structures, b) vesicles with surface-immobilized tubular structures, and c) different types of bilayer membranes. Whereas the methods described above are preferably used to create two-dimensional lipid membrane films on differentially coated substrates, they might also be employed to immobilize and support vesicle-nanotube networks on the surfaces. Methods for formation of such networks are known and might be particularly successful when the lipid creeping or spreading properties of a particular lipid membrane on a particular surface is inefficient or for other reasons is unsatisfactory. It is worth mentioning that diffusion of membrane proteins has been observed across nanotubes (Davidson et al. J. Am. Chem. Soc. 125, 374-378 (2003)). Formation of bilayer devices from nanotube-vesicle networks as stated above where the adsorption is very high is another very attractive avenue to arrive at lipid bilayer film-covered devices.

The membraneophilic regions of the substrates may also be covered by lipid membrane films through deposition of lipids using fusion of small surface-adsorbed vesicles as displayed in figure 7d. These vesicles might be added to the substrate in e.g. water solution. After sedimentation to the surface of the substrate, they preferentially bind to the patterned membraneophilic region were they are immobilized with so high adsorption potentials that they burst open (surface tension is higher than lysis tension). Excess intact vesicles may be washed away.

Deposition of lipid bilayer membranes can also be achieved through administration by microfluidic channels as displayed in figure 7e. For example, unilamellar and multilamellar vesicles can be presented to a differentially coated substrate by means of microfluidic channels. Also multilamellar and unilamellar vesicles can be directed to certain loci on a differentially patterned substrate.

As yet another alternative, formation of lipid bilayer membranes on membraneophilic regions on a patterned substrate might be achieved by direct injection of dissolved lipids as shown schematically in figure 7g. This procedure would then be similar to the direct injection method for formation of liposomes. A suspension of phospholipids in organic solvent would simply be injected into a substrate contained in water solution. With this method small and large liposomes might form in solution and thereafter attach and spread on the membraneophilic areas. Alternatively, the lipids can assemble into a monolayer structure on the patterned structures (SAM), followed by formation of a second layer of lipids to create a lipid bilayer membrane. Intact vesicles can also be attached to the surfaces.

Figure 8 show DIC photomicrographs and fluorescence images of several different microfabricated structures having membraneophilic and membraneophobic regions with different sizes and features. The structural features of these devices can vary in order to find the optimum functional size and shape of the interconnecting lanes and also to explore the limit of resolution of the fabrication protocol. In these images, the patterns consist of SU8 (appears as dark grey in the photomicrographs) on top of a gold surface or Al₂O₃ surface, made membraneophilic through chemical and/or physical modulation or manipulation, (appears light grey in the photomicrographs). The gold surface and the Al₂O₃ surface, made membraneophilic through chemical and/or physical modulation or manipulation, represent the membraneophilic surface and SU8 represents the membraneophobic surface. Figure 8a shows a simple structure consisting of two circular spots of gold interconnected by a thin gold lane. The width of the interconnecting line was varied between 0.5 and 10 micrometers. In this case the lane was 1 micrometer wide. Figure 8b shows a similar pattern as in figure 8a with 2 micrometer wide lanes. These images also display the parallel capability of these fabrication methods, by repetitive spacing of the pattern on the substrate surface. Figure 8c shows many different microfabricated patterns on a single substrate. In this case, two lanes of gold, made membraneophilic through chemical and/or physical modulation or manipulation, separated by varying distances, between 1 and 10 micrometers, interconnecting the two circular gold spots, made membraneophilic through chemical and/or physical modulation or manipulation, were fabricated. Again the width of the lanes could also be varied. These particular structures had 1 micrometer wide lanes. Figure 8d shows a magnified view of one of the structures in figure 8c. The image shows that the fabrication protocol is capable of creating well-defined lanes and structures of high aspect ratio. The depth of these patterns, corresponding to the thickness of the SU-8 layer on top of the Al₂O₃ surface, made membraneophilic through chemical and/or physical modulation or manipulation , can range between nanometers to millimeters, however, the depth is typically 1 micrometer. Figure 8e shows meandering structures, that is, structures where the lanes have turns of 90 degrees, thereby forming two sets of parallel lines. These types of structures might be used in conjunction with multiple sets of electrodes, making it possible to perform electrophoretic separations in two dimensions. Figure 8f shows an enlargement of the structures in e. Figure 8 g shows different branching or converging structures. Figure 8h shows a fluorescence image illustrating the coverage of lipid confined to the gold pattern, made membraneophilic through chemical and/or physical modulation or manipulation. The lipid was deposited on the surface by deposition of multilamellar liposomes made from soy bean lipids. Multilamellar liposomes were prepared using the dehydration/rehydration technique and doped by a fluorescent membrane dye, DiO, in order to be able to visualize the spreading more controllably. Figure 8k shows a chip with membraneophilic Al₂O₃-coated patterns, made membraneophilic through chemical and/or physical modulation or manipulation, (appear as light-gray in the images) and SU-8-coated membraneophobic regions (appear dark grey in the images). The circular spots covered by Al₂O₃ are 50 micrometers in diameter, and the interconnecting lanes of Al₂O₃ are approximately 2 micrometer wide and 50 micrometers long. Figure 8I shows a fluorescence image illustrating coverage of lipid confined to the Al₂O₃-coated pattern, made membraneophilic through chemical and/or physical modulation or manipulation, on one of the microfabricated structures shown in figure 8k. The lipid membrane was stained with a fluorescent membrane dye in order to visualize the spreading. In this case, the lipid coverage was performed by using a suspension of small unilamellar vesicles (SUVs).

After formation of the bilayer membrane onto the membraneophilic area, deposition or introduction of various components into the membrane matrix, including lipids, membrane proteins (peripheral and integral), glycosyl-phosphatidylinositol (GPI)-anchored proteins or other proteins linked to the bilayer membrane by for example biotin-avidin interactions, can be performed by several different techniques.

Electroinjection involves the deposition of surplus or extra membrane material from an external source, for example, a lipid suspension, liposome preparation etcetera, close to the membrane-coated surface, using a micropipette, as shown in figure 9a. This surplus membrane material contains the transport marker and can be incorporated into the membrane coated area by subsequent application of one or several electric pulses across the two membranes, as shown in figure 9b. The electrical stimulation triggers and induces membrane fusion and is created by the use of a carbon fiber microelectrode on one hand and a platinum wire situated inside the micropipette, both connected to a pulse generator, After fusion the transport markers are situated at one of the circular spots (figure 9c).

Another technique involves deposition of surplus membrane material containing the transport marker next to the membrane-coated area by various techniques including for example optical tweezers and micropipette techniques (figure 9d). The two membranes are then fused by one or several electric pulses across the two membranes, using two carbon fiber electrodes, connected to a pulse generator (figure 9e). This technique is referred to as electrofusion. As in figure 9a, after fusion the transport markers are situated at one of the circular spots (figure 9Bc).

Membrane fusion can also be triggered or promoted by electromagnetic radiation, denoted by optical fusion. In this case the contact zone between the two membrane compartments are subjected to of light (usually UV-light) in order to destabilize the membrane and induce fusion, as shown in figure 9Ca-c.

Similarly, the fusion of membranes can also be promoted by adding various chemical agents such as detergents, calcium ions, and other divalent metal ions, as well as polymers, nystatin/ergosterol, specialized fusogenic lipid analogs, including, for example, cationic lipids or myristate, and also certain peptides have been proven to induce membrane fusion, as shown in figure 9Da-c.

Alternatively, the deposition of lipids using the spreading of lipids or fusion of small vesicles to the membraneophilic surface can include transport markers in the lipid preparation and therefore create a lipid surface with transport markers across a patterned surface, as shown in figure 9Ea-c.

Membranes and membrane-components such as membrane proteins can also be introduced into the lipid matrix by use of microfluidics, as shown in figure 9Fa-c, in a similar manner to figure 7Ea-e. Flows of solutions containing, for example, small fusogenic vesicles can be flushed across different structures or even parts of the same structure. The fusogenic vesicles containing membrane-components, such as membrane proteins, will merge with the pre-formed lipid bilayer matrix. Thereby, injection of, for example, membrane proteins can be highly localized and targeted to specific sites on the chip surface.

As discussed above, the lipid membrane devices used in methods according to the current invention can be used for separation of membrane proteins embedded in the membrane matrix. One obvious method for achieving separation of membrane proteins included in membranes is by utilizing electric fields. The electric field can directly affect the membrane proteins or it can affect other components in the lipid matrix *e.g.* specialized lipids that associate to proteins. We call membrane-associated materials for MAM's, which can be any molecule or particulate material including membrane proteins. We call membrane materials for MM's and can be any lipid or liquid crystalline phase, and we call a membrane patch containing an associated molecule or material for MP. In theory, electrophoretic migration of a species contained in the membrane material is a function of the charge-to-frictional drag ratio of that species. Therefore, both MAM's, MP's and MM's can be moved individually or collectively depending on the net charge and size carried by each entity. This is described in more detail below. Also depending if the lipid structure is a tubular structure or a 2-dimensional sheet and the surface charge of these systems, electroendoosmotic delivery or transport can be efficient to different degrees. Electrical migration methods can thus be used to impose differential migrations velocities of e.g. membrane proteins dissolved in the MM and can be used as a method to fractionate and identify these proteins from analysis of migration times. It should be made clear that the migrational velocity of a certain protein may be a complex function of several parameters, for example, the size of the MAM's, the charge and lipid interaction of the MAM, to mention a few.

However, in a second case, separation of membrane proteins can be based on only thermal fluctuations or diffusion. This might be especially efficient when the length scale of the device is small enough so that displacement by thermal movement is relatively large. Different membrane proteins have different diffusional velocity, depending on size of the protein and the degree of interaction with the surrounding lipid bilayer matrix. For example, lipid-covered devices with radiating arms of nanometer-to-micrometer dimension can be used for separation of MAM's based on diffusion. Figure 10 shows an example of a fluorescence-recovery-after-photobleaching (FRAP) experiment of a DiO-labeled lipid membrane in a comb structure pattern consisting of a single lane, which is connected to four radiating lanes. The widths of the lanes are 5 µm and the length of the radiating lanes is 25 µm. After lipid deposition in the form of small unilamellar soybean liposomes doped with 1 mol% DiO, the lanes of the comb-structure were bleached by intense excitation light (488 nm) produced from a mercury lamp and selected through a filter. After photobleaching, snapshots of the recovery were recorded in a time-lapse series of pictures. The time between each picture in figure 10 is four minutes. The pictures illustrate an even and symmetrical recovery of the fluorescence as DiO molecules move diffusively in the lipid bilayer. The pattern is completely refilled with fluorescence within minutes. If several different lipid membrane-associated species with different lateral diffusion coefficients exist in the lipid bilayer matrix within such a comb-like structure, fractionation of these species could be realized. If the different species to be fractionated is located on one side of a comb-like structure, added there by one of the previously described techniques, they would diffuse with different velocities into the lanes, thereby filling the different lanes further into the comb structure one at a time. This would result in a fractionation of the membrane-associated species, where the fastest diffusive species is located in one radiating arm, furthest away from the original point of release, the next radiating arm would consist of a mixture of the two fastest species and so on. Such a fractionation mechanism purely based on thermal motion or lateral diffusion in the patterned lipid membrane provides a crude yet simple way to purify membrane proteins that are otherwise difficult to separate from each other. Noticeably, such a fractionation could be optimized and tuned with the design of the comb-structure, by variation of parameters such as number, length and width of radiating arms, length and width of the lane, separation distance between the radiating arms etcetera.

Another separation mechanism that can be implemented into these chip structures relies on creating a flow of fluid across the lipid matrix by the use of microfluidics, for example. Membrane-associated species experience different drag forces upon flow across the lipid matrix, depending on their structure and interaction with the bilayer. If a membrane protein has large protruding parts exposed to the solution above the lipid bilayer membrane, it is affected by the fluid motion and will thus experience a hydrodynamic drag force, or Stokes drag force. Depending on the size and shape of the protruding parts, the lipid interaction and the fluid flow velocity, different membrane-associated species may experience different drag forces and thus be separated in the lipid matrix.

The velocity of a membrane-associated species may be modified if it attaches to a ligand, antibody, or modified bead etcetera. Thus, this species may be identified/detected by a change in the transport velocity, upon binding of a specific target molecule, for example. Depending on the type of separation method used (electric/magnetic field, diffusion, flow etcetera) the velocity may increase or decrease. These types of investigations might be applied to drug discovery applications where drug candidates are flushed across a lipid membrane device containing one or several different target molecules and at the same time registering changes in their velocity. A change in velocity is thus evidence of binding between the target molecule and the drug candidate.

It is also possible to induce magnetic fields across the structures of these devices using the same principles of separation as with electric fields, for example. Magnetic beads can be linked to membrane proteins of interest using, for example, antibodies for the target membrane protein. Such magnetic beads are commercially available and can easily be linked to specific antibodies for a number of membrane proteins. This way, membrane proteins that are located inside the lipid matrix can be attached to magnetic beads simply by flushing the solution above the lipid device structure with a solution containing the magnetic beads modified with a specific antibody for example (figure 11). After the magnetic beads have attached to the target membrane proteins in the lipid matrix a magnetic field can be applied across the system to induce motion of the target protein, without affecting the rest of the membrane proteins in the bilayer (figure 11 d). This can be used for separation of several membrane proteins by repetitive extraction of different membrane proteins in the lipid matrix and collection of the fractions. It can also be used to extract membrane proteins which are abundant and thus easy to extract in order to enhance the purification of other membrane proteins that are present in a low copy number.

Different separation techniques might be used together, at the same time or in steps, in the same or in different dimensions. This will also affect the designs of the device structure, together with factors such as what type of separations that are to be used (diffusion-based, electric field-based, magnetic field-based etcetera), the sample size and what type of sample (crude extracts, membrane fractions, purified proteins etcetera). In some instances, a separation can occur in one dimension and the different fractions may be collected into lanes or triangles branching in the opposite direction from the first separation lane. This can be done by utilizing the same separation mechanism as the first separation or another separation mechanism in the opposite direction. The use of branching triangles may also be used to concentrate fractions into smaller spots or lanes before analysis (figure 12). An example of combination of separation techniques is to use electric fields and fluid flows at the same time. From electrophoresis theory electroosmotic flow (or fluid flow, created form the electric field) is directed in the opposite direction of the electric migration of some charged species. In this instance different species in the membrane will be affected differently by the different techniques and this might lead to a more optimized separation of differently charged species.

The chip might also be integrated to have chemical gradients, such as pH-gradients on different parts of the chip or even different parts of the same structure. Normal gel-based separation methods often rely on separation of proteins in two dimensions, such as electric fields in one dimension followed by a separation in a second dimension in combination with pH-gradients for example. When the protein attains a neutral charge, which occurs at its isoelectric point (pl), the protein stops migrating in the electric field. A problem with membrane proteins is precipitation at the isoelectric point. However, when situated in the protected hydrophobic interior of a lipid bilayer, this will not occur to an equal extent. The chip structures can be combined with pH-gradients in order to facilitate separation of membrane proteins using the same principles as those normally applied to 2-D-gel separations. These pH-gradients can be produced by combining the chip structures with a gel that can create the pH-gradient above the lipid matrix. Also, microfluidics can be combined with the chip-structure in order to flush parts of the chip-structure with different solutions of various pH (figure 13). This way, proteins migrate in an electric field until the protein is neutral in charge, which can occur at its isoelectric point, pl, as described above.

Figure 14 illustrates the adding of reagents, antibodies and ligands etcetera after the lipid matrix has been created onto patterned structures. In this example, the lipid matrix consisted of polar lipid extract from soybean, doped with 2 mol% biotin X DHPE lipids. The lipid matrix was formed by adding an extruded lipid preparation of the above composition to the chip-structure for 30-60 minutes. The lipid preparation thus consisted of 100 nm diameter small unilamellar liposomes (SUVs), which attach to the surface and spread onto the surface. After formation of the lipid matrix, the surplus lipid suspension was washed away, by flushing the flow cell 3-5 times with the same buffer as in the liposome preparation. After washing, a solution of beta-phycoerythrin (0.2 mg/ml in the same buffer as before) was added to the chip-structure and after 30 minutes the surplus beta-phycoerythrin was washed away, using the same protocol as before. Also, the flow cell was emptied completely of solution for short time periods 2-3 times to remove unspecific bound material to the SU8 surface. Figure 14 show the resulting fluorescence image of one of the device structures, thus illustrating that binding of ligands, antibodies and other reagents can be added after the lipid matrix has been formed. This is especially interesting for applications such as drug screening using *e.g,* microarray protocols, where different patches of lipid matrixes containing various membrane proteins can be flushed with reagents such as specific antibodies in order to find a specific target protein. Also, this type of approach can be used as a post-labeling procedure after separation of membrane proteins in a lipid matrix, to localize the spots where specific proteins can be found. Finally, as mentioned above, specific digestive reagents can be added to the solution above the lipid matrix in order to cleave fragments from membrane proteins for further processing and identification using *e.g.* MALDr-TOF.

The use of lipid bilayer network structures as separation beds for membrane-associated species can also result in several different separation mechanisms, which can work together or separately to induce separation. These types of networks can be produced in a number of ways depending on the liposome type (multilamellar, unilamellar), and nature of surface interaction etcetera. Some membrane-associated species, such as membrane proteins, can be susceptive to curvature changes in such a way that they preferentially reside in planar lipid bilayers, Also, some membrane proteins have a preference for certain lipids or lipid complexes, such as lipid rafts. Such rafts may also display different affinity for regions of various membrane curvatures. Through this, separation may occur through extraction of membrane-associated species into tubular regions in a network structure, thus separating it from other species, which do not tend to reside in regions of high membrane curvature, found in tubular regions. This type of separation strategy may also be applicable through the coupling of lipid network structures directly to living cells. Extraction of membrane proteins through this approach, may lead to distinction of various membrane proteins from the cellular membrane.

Transport of species through lipid nanotubes have previously been achieved by the creation of lipid flows across systems of different membrane tension. Lipids flow from regions of low tension to regions of high tension. Membrane-associated species, such as membrane proteins can possibly be separated in the lipid matrix of lipid nanotubes, through differential interaction with the lipid matrix (integral or peripheral membrane protein) and size (different hydrodynamic drag from surrounding solution) etcetera. Upon lipid flow, membrane-associated species in the lipid nanotube can experience differential drag from the lipid matrix and the surrounding solution, which might lead to separation of these species.

Since it is possible to inject proteins embedded within their native lipid matrix onto the chip, it is possible to separate membrane protein complexes which are associated with each other. Not only might this be important for studies where it is vital to keep the functionality of the membrane protein complex, but it also makes it possible to identify which proteins are associated with each other. This might be achieved by designing a structure making it possible to perform a two-step separation, for example, through the branching structures or the structures of meandering character, First, the membrane protein complexes are separated as a unit in one step or in one dimension. Then, the complex is exposed to an environment allowing the breaking up of the complex into the different protein subunits. The breaking up might be achieved by treatment with urea. The chip can be integrated with a microfluidic system or a flow-cell type of system, allowing the exchange of the external fluid around the lipid bilayer membrane to, for example, add urea in order to break the protein complexes. The solution is then changed back to the original solution, or the chemical environment in respect to pH or ionic strength might be changed, whereupon a second dimension separation can be performed to separate the different subunits of the protein complex. Also, different lanes having different chemical environments or even a gradient along the separation lane might be used in these two or multiple separation steps.

Figure 15 shows schematic drawings concerning the integration and use of the chip structures in conjunction with MALDI-TOF MS (Matrix-Assisted Laser-Desorption-Ionization - Time-Of-Flight Mass-Spectrometry). Since these structures are scalable in size from the micrometer to millimeter range, the structures can consist of large single devices or multiple parallel devices, depending on the evaluation protocol, the sample size, the sample concentration and detection limit etcetera, when integrating the chip structures with MALDI-TOF mass-spectrometry detection. Figure 15A shows a single device, where membrane-associated species are confined to the injection site of the device structure. After separation of these species, using, for example, electric fields, diffusion etcetera, the separation can be stopped and "frozen" by different means and the structure can be analyzed by MALDI-TOF. Figure 15B shows multiple parallel devices, which can consist of one or several devices of the same or different designs, where the separation of membrane-associated species can be performed in parallel. The parallel devices can also be coupled in order to concentrate a membrane-associated species of low abundance prior to the detection using MALDI-TOF. The schematic image illustrates several injection sites and after separation, the membrane-associated species have been concentrated into one site, where detection can occur. An even simpler approach, which is illustrated in figure 15C, involves the adsorption of intact vesicles or a lipid bilayer membrane to the -chip structure, which works as a matrix for membrane-associated species, such as membrane proteins. The membrane proteins can be immobilized onto the chip structures using the techniques described by figure 9. By exchanging the solution above the lipid matrix to a solution containing some digestive enzyme or enzymes, for example, trypsin, the part of the membrane protein which protrudes outside the lipid bilayer matrix and the hydrophilic parts of the membrane protein can be cleaved into peptide fragments. The peptide fragments can then be collected and investigated by MALDI-TOF. This type of protocol can also be implemented using microfluidic systems. After performing a separation in the lipid matrix, the different parts of the designed structures can be exposed to the digestive solutions by use of focused flow from microfluidic channels and the peptide fragments that are released can be collected individually for MALDI-TOF mass-spectrometry identification.

Depending on the function of the surface, being membraneophilic or membraneophobic, and the type of application that the device performs, the type and appearance of the device can also vary. Figure 16 shows some non-limiting examples of devices consisting of a chamber, channel or container, respectively. For some applications, such as MALDI-TOF applications, the device can consist of a chamber where all of the exposed surface area inside the chamber is modified to be membraneophilic, adsorbing lipid in the form of either intact vesicles or a supported lipid bilayer (Figure 16). In other applications, the surfaces inside the chamber can also be structured with membraneophilic structures embedded in membraneophobic areas. For some applications only one of either the top-lid or the bottom substrate can be structured with both membraneophilic and membraneophobic areas, while the opposing surface may be a plain surface having either membraneophobic or membraneophilic character.

In applications where a large surface area is needed, the surface-to-volume ratio can be increased through several modifications. The device can consist of a channel structure in order to optimize the surface area (Figure 17). Such channel structures can be made in various materials and modified with membraneophilic surfaces. The channel widths can vary between micrometers up to meters and the height can vary between micrometers up to meters de-pending on the volume and surface area requirements. The exposed surface area in the channel structure, chamber or container can also be structured by microfabrication protocols to produce structures (channels, pillars, groves etcetera) on the surface prior to modification of the surface with membraneophilic and/or membraneophobic character. In order to increase the surface area, particles modified with membraneophilic surfaces can also be added to the chamber, container or channel structure. The size of such particles can range between nanometers and meters depending on the application. In other applications the particles added to the device can also be modified with membraneophobic surfaces.

When running parallel or multiple tests in conjunction with control samples it is convenient to run parallel experiments on the same platform. Therefore, several parallel devices consisting of chambers, channel structures or other device types filled with particles can be integrated into a single device structure (Figure 16 and Figure 17).

Applications for the devices may rely on adding and extracting solutions through inlet and outlet channels. Such solutions include lipid preparations to coat membraneophilic surfaces with lipids, lipid/protein preparations for protein applications such as in MALDI-TOF integration, wash solutions in order to remove excess lipid/protein preparations, staining solutions for proteins or lipids in, the preparations, or digestive solutions (containing digestive enzymes such as trypsin, peptidase, proteinase K, for example) for applications where proteins are digested in the chamber, channel structure or container. Such solutions are preferably added through an inlet channel (Figure 16 and Figure 17). Extraction of these solutions and peptide solutions where proteins have been digested preferably takes place through an outlet channel. This type of additions or extractions of solutions may be assisted by including single or multiple microfluidic channels into and out of the chamber, channel structure or container. The digestion of membrane proteins may take place in a two-step process as illustrated in Figure 18. In the first step, the digestive agent can only cleave the parts of the membrane protein which protrudes out into the solution, leaving the hydrophobic parts (the transmembrane regions of the membrane protein) intact. The peptide fragments formed from the protruding parts of the membrane proteins are collected for further processing and/or detection/identification. This first step may be repeated several times with different concentrations of the digestive agents, with different digestion times, with different modifiers of the digestive agent, or sequentially with several different enzymes. In the second step, the digestive agent is added in conjunction with detergents, organic solvents or other chemical agents that disrupts the membrane. The digestive agent cleaves the rest of the membrane protein into peptide fragments. The lipids and the hydrophobic peptide fragments are stabilized by the added detergents. The peptide fragments formed from the membrane proteins are collected for further processing and/or detection/identification.

Figure 19 in Example 4 shows a mass spectrometry trace (MALDI - TOF MS) of peptide fragments obtained from on-chip trypsin digestion of membrane proteins from red blood cells. The peptide fragments are from the first step process, meaning that they originate only from parts protruding outside the membrane into which it is embedded. The peaks of highest intensity were selected and MALDI - TOF MS/MS was performed, resulting in identification of the peptides shown in Table 1.

Since the devices can have several chambers or channel structures working in parallel, this allows for several different performances. The sample can be the same in all chambers or channels and the treatment and digestion performed on the sample can be identical in all parallel chambers or channels. The sample can also differ between the chambers and channels, while retaining the same treatment and digestion protocol. Also, the sample can be the same in all chambers and channels, however, the treatment and digestion protocol can differ between the chambers and channels. It is also possible to have both different samples and different treatment and digestion protocols in the different chambers and channels, but still on the same device.

For applications when some type of active transport is to be performed in the lipid bilayer, integration of electrodes for creation of electric fields is needed. Such electrodes can be inserted through the inlet/outlet channels or be integrated into one of the membraneophilic surfaces, for example, through deposition of thin metal films. The electrodes can consist of some conducting metal, for example platinum. Active transport may also be performed by magnetic fields, where magnetic beads attached to target proteins in the lipid bilayer for example through antibody coupling, are dragged through the solution by application of magnetic fields.

In the case of a device used in conjunction with the above mentioned methods, techniques and protocols for analysis and studies of membrane proteins, the following applications are possible. First of all identification and characterisation of a protein (preferably a membrane protein), including so called sequence coverage studies, which involves optimized protocols to detect and identify as many peptides as possible in the protein sequence, corresponding to as much as possible of the amino acid sequence. Second, studies of post-translational modifications are also applicable in the same context. Third, mapping of membrane proteins from different cell.samples - membrane proteome profiling. Highly complex samples with a wide range of abundance levels are digested and analyzed. Prior to MS analysis the created peptides are separated in one (for example, reversed phase HPLC) or two dimensions (for example, ion exchange HPLC (SCX - strong cation exchange) followed by, for example, reversed phase HPLC). This also enables the studies of low abundance proteins and the discovery of new drug targets. Fourth, functional studies of membrane proteins - examples are target fishing and receptor deorphanization, e.g. G-protein coupled receptor (CrPCR) deorphanization. In the case of target fishing, the purpose is to use ligand binding studies to identify which ligands binds to which proteins. In the case of receptor deorphanization, the purpose is to elucidate the function of receptors and their possible ligands. There are many receptors which function is unknown, hence the name orphan receptors. Finally, investigation of up-and-down regulation of protein expression levels during states of disease and/or medication, so called expression profiling. Such studies involves comparison of different samples and the evaluation of differences between the samples.

The improved data output obtained with the described device used in conjunction with the above mentioned methods, techniques and protocols compared to other techniques is based on the fact that multiple chemical reaction and/or wash steps can be implemented in order to ensure optimized protocols on the chip. Such steps are also implemented without dilution of the sample. Also, extramembrane and transmembrane parts of the membrane proteins can be targeted and collected in different steps, using the same sample. It also means that a single sample can be subjected to different enzymes, sequentially and/or in parallel to digest various parts of the same sample. The format of the chip with a high surface-to-volume ratio in conjunction with sequential digestion etc leads to a high concentration of created peptides with minimal sample loss, which enables higher sensitivity. This promotes sequence coverage studies and the detection of low abundance membrane proteins in the sample. Also, different subcellular fractions from cells can be handled in parallel on one chip. Finally, ligand binding/crosslinking/digestion can potentially give target peptide binding sites. A protein with and without bound ligand will yield different peptide maps after enzymatic digestion, where the ligand either sterically obstructs the enzyme from cleaving or inflicts a structural change upon binding.

Moreover the general advantages of methods using a chip device are first of all that the sample is confined to a surface, that is, it is held in a stationary matrix during the analysis, thereby enabling multiple steps of labelling, chemical modifications, digestion etc. The chip device have the ability to be used in conjunction with an arsenal of detection techniques on-and off- chip, including MS, fluorescence, electrochemistry, SPR, QCM, etc. There is also the possibility to integrate the chip to existing chip-based platforms and traditional analysis tools. Finally, the sample handling is easy to automate.

In a parallel issue, the advantages of methods of using a lipid matrix in a device are that the membrane bound components, such as membrane proteins are kept in their natural lipid bilayer environment. This means that the structure and function of the membrane bound components are retained. Preparation, transportation, deposition and processing of membrane vesicles containing membrane bound components, e.g. proteins, does not require the use of detergents. This is an advantage since detergents may have disadvantageous effects, e.g. denaturing of proteins.

Figure 20 shows a general description of the features of the chip device used in the methods according to the invention and some of its functions. The chip provides a way to capture membrane material from a number of sources (cell membranes, subcellular membrane fractions, proteoliposomes etc.). The chip format offers ways to add and extract fluids and materials from the chip during the process and handling of the sample to be analyzed and studied. This can be done through fluid ports located anywhere on the chip structure.

The figure also illustrates how the chip device works in the sense that peptides can be cleaved off and eluted from the chip in several steps and thereby also target different structures of the protein by using different protocols and proteases sequentially. The first step can, for example, target the extramembraneous (water soluble) parts and create peptides for analysis of these parts specifically. A following step can then target the transmembrane parts by using a different protocol and a different protease. The peptides produced by digestion can be collected in a second fraction and analyzed.

This type of approach is advantageous to use in, e.g. sequence coverage studies and membrane protein analysis in general.

As mentioned above a number of different protocols can be implemented for sequence coverage studies. Figure 21 shows the concept of sequential digestion using different proteases.

As pictorially described in figure 21 digestion of the protein may be done sequentially by one or several different proteases. After the first digestion, peptides can be collected for analysis. The following digestion steps can be done in several, different ways using different protocols and different proteases. The second step can, for example, be done according to figure 21, where the membrane is either disrupted and digested using anyone of protease 2, 3, 4, 5 or left intact and the protein is digested using anyone of protease 2, 3, 4, 5. Similar protocols can be implemented in following steps. Any combination of the proteases (1-5), in any order can be done in order to perform the sequential digest of a target protein. Also, the same protease in different concentrations can be used sequentially. It is also possible to perform sequential digest by amending or altering other parameters, such as time or temperature. Before or after digestion the protein or peptides can be exposed to chemical or enzymatic modification steps. Examples of this is reduction and alkylation of disulfide bonds, enzymatic digestion/removal of oligosacharides at glycosylation sites, investigation of phosphorylation sites etc, where so called post-translational modifications are studied.

### EXAMPLES

### 1. Fabrication of microchips having membraneophobic and membraneophilic areas.

### Materials

28 mm diameter glass coverslips with a thickness of 130-170 µm were purchased from Menzel-Glaser (Braunschweig, Germany). Electron beam resists, photoresists and developers were obtained from AZ Electronic Materials (Somerville, NJ, USA) and primers were from Shipley (Marlborough, MA, USA). All other chemicals used in the fabrication were of VLSI grade and from Merck unless otherwise stated. 100 mm, low reflective blank quartz masks coated with 100 nm of chrome used in the mask fabrication were obtained from Nanofilm (Westlake Village, CA). Materials used in the evaporation were purchased from Nordic High Vacuum (Kullavik, Sweden).

### Substrate Preparation

The glass coverslips were sonicated in acetone for 20 minutes, cleaned in a 80°C RCA1 solution (a 1:1:5 solution of H₂O₂ (25%), NH₃ (30%) and Milli-Q) for 10 minutes, dipped in a buffered oxide etching solution (a 6:1 solution of NH₄F (30%) and HF (49%)) for 2 minutes, rinsed in Milli-Q water in a QDR and blow dried under N₂.

### Fabrication of SiO₂ patterns on Au

The substrates were inserted into a AVAC HVC-600 thin film deposition system and a film consisting of 1.5 nm of titanium, 13.5 nm of gold, 1.5 nm of titanium, and finally 60 nm of silicon dioxide was evaporated onto the substrates by means of electron beam evaporation. The substrates were spin coated first with an adhesion promoter (HMDS), and then with AZ 5214-E image reversal at 6000 rpm for 45 seconds, pre-exposure baked for 2 minutes at 90 °C on a hotplate and-exposed through a patterned dark field chrome mask on a Carl Suss MJB-3 mask aligner for a dose of 120 mJ/cm² at 365 nm wavelength. The substrates were post-exposure baked for 30 seconds at 125 °C on a hotplate and subsequently flood-exposed for a dose of 720 mJ/cm². The pattern was developed in a 1:5 mixture of AZ351. developer and DI water for 60 seconds, rinsed in a QDR and blow dried under N₂. The result was a clear field pattern. The exposed silicon dioxide was etched in a Plasmatherm RIE m-95 reactive ion etcher (180 seconds, 100 W, 25 mTorr, 32 cm³ CF₄/s, 8 cm³ H₂/s, 1 cm³ O₂/s). To confirm that the silicon dioxide was etched away completely, profilometer measurements, two-point resistance measurements, and gold wet etch tests were performed. The substrates were stripped of the remaining resist using Shipley 1165 remover and rinsed in acetone, 2-propanol and DI water in a QDR and blow dried under N₂. The substrates were finally desummed (60 seconds, 250 W, 250 cm³ O₂/s) using a TePla 300PC Microwave Plasma system to remove remaining siloxanes on the silicon dioxide originating from the Microposit primer.

The process is optimized for AZ-5214E image reversal resist, but it can be adapted to basically any Novolac-based image reversal- or negative tone resist.

The etching can with some resists also be performed using a wet etching system, e.g. buffered oxide etching solution (a 1:6 mixture of 49% hydrofluoric acid and 30% ammonium fluoride). The surfaces can be made membraneophilic through chemical and/or physical modulation or manipulation

### Fabrication of patterns in SU-8 2002 on Au or Al₂O₃

The substrates were inserted into an AVAC HVC-600 thin film deposition system and a film (either 50 nm of Al₂O₃ or 1.5 nm of Ti + 13.5 nm of Au) was evaporated onto the substrates by means of electron beam evaporation. The substrates were spin coated with MicroChem SU-8 2002 negative tone photoresist at 4000 rpm for 30 seconds, pre-exposure baked for 2 minutes at 95 °C on a hotplate and exposed through a patterned clear field chrome mask on a Carl Suss MJB-3 mask aligner for a dose of 120 mJ/cm² at 365 nm wavelength. The substrate was post-exposure baked for 60 seconds at 95 °C. The pattern was developed in MicroChem's SU-8 developer for 60 seconds, briefly rinsed in 2-propanol and blow dried under N₂. This resulted in a dark field pattern of SU-8 2002 on Au or Al₂O₃. The substrates were finally descummed (60 seconds, 250 W, 250 cm³ O₂/s) using a TePla 300PC Microwave Plasma system. The surfaces can be made membraneophilic through chemical and/or physical modulation or manipulation

### 2. Preferential coating with liposomes of the membraneophilic area of microchips having membraneophobic and membraneophilic areas.

### Materials and Methods

### Vesicle Preparation

Vesicles were prepared from soybean lecithin (SBL) dissolved in chloroform (100mg/mL) as a stock solution and stained with DiO (typically 1 mol% with respect to lipid concentration). The rehydration/rehydration method described by Criado and Keller (Criado, M., Keller, B. U. FEBS Lett. 224, 172-176 (1987)) with modifications (Karlsson, M. et al. Anal. Chem. 72, 5857-5862 (2000)) was used to prepare unilamellar liposomes. In short, a droplet of 5µL lipid dispersion (1 mg/mL) was placed on the cover slip and dehydrated in a vacuum desiccator for 25 min. When the lipid film was dry, it was rehydrated with buffer solution (Trizma base 5 mM, K₃PO₄ 30 mM, KH₂PO₄ 30 mM, MgSO₄ 1 mM, EDTA 0.5 mM, pH 7.8). After a few minutes, the liposome were formed. By using a glass pipette a small sample of lipid suspension was transferred into a droplet of buffer solution that was positioned on the testing surface.

### Microscopy and Fluorescence

The cover slips were places directly on the stage of an inverted microscope (Leica DM IRB. Wetzlar, Germany) using a Leica PL Fluotar 40x objective. For epiflourescence illumination the 488 -nm line of an Ar⁺ laser (2025-05, Spectra-Physics) was used. To break the coherence and scatter the laser light, a transparent spinning disk was placed in the beam path. The light was sent through a polychroic mirror (Leica) and an objective to excite the fluorophores. The fluorescence was collected by a three-chip color CCD camera (Hamamatsu, Kista, Sweden) and recorded by using a digital video (DVCAM,DSR-11, Sony, Japan). Digital images were edited by using the Adobe Premiere graphic software and Matlab.

### Chemicals and Materials

Trizma base, glycerol, and potassium phosphate were obtained from Sigma-Aldrich Sweden AB. Soybean lecithin (SBL, a polar lipid extract) was obtained from Avanti Polar Lipids, Inc. (700 Industrial Park Drive, Alabaster, AL 35007). Chloroform, EDTA (titriplex III), magnesium sulfate, potassium dihydrogen phosphate, potassium chloride, sodium chloride, and magnesium chloride were from Merck (Darmstadt, Germany). Deionized water (Millipore Corp., Bedford, MA) was used to prepare the buffer. DiO (3,39-dioctadecyloxacarbocyanine perchlorate) was obtained from Molecular Probes (Leiden, The Netherlands). The polar lipid extract consisted of a mixture of phosphatidylcholine (45.7%), phosphatidylethanolamine (22.1%), phosphatidylinositol (18.4%), phosphatidic acid (6.9%), and others (6.9%).

A number of different surfaces were tested: plasma treated silicon dioxide, SU-8, plasma treated SU-8, borosilicate glass, plasma treated gold, platinum, plasma treated platinum, Al₂O₃, plasma-treated Al₂O₃, all surfaces made membraneophilic through chemical and/or physical modulation or manipulation. The experiments were done in the following way. On the surface that has to be tested we first transfer a droplet of buffer, and than inject the liposomes solution into the droplet. After a few minutes liposomes reach the surface, adhere on to it and spread at a rate depending on the surface properties. From the experiments with different surfaces we found a combination of membraneophilic and membraneophobic properties. The gold plasma treated surface, made membraneophilic through chemical and/or physical modulation or manipulation, showed the highest lipid spreading (fig 8i). The non-spreading surface was a plasma treated SU-8 surface (fig 8j). We combine membraneophilic and membraneophobic regions in order to create a structure with differential lipid coverage. The following figure illustrates that parts of the surface covered with gold, made membraneophilic through chemical and/or physical modulation or manipulation, provide essentially higher adhesion of the lipid material comparing with SU8 covered regions. Patterned areas on the pictures correspond to the area covered with gold. Fluorescent images are collected about 20 minutes after adding liposomes into buffer solution.

### 3. Diffusion of membrane-associated species as a separation mechanism (not part of the invention, for information purposes only)

An example of diffusive motion in a patterned lipid bilayer is shown in figure 10. The lipid was first deposited by fusion of small unilamellar vesicles (SUVs) to the membraneophilic surfaces. The SUVs consisted of soybean lipids doped with 1 mol% DiO, for fluorescence visualization (total lipid concentration: 240 µl soybean lipids, stock solution 100 mg/ml chloroform, was dried by evaporation for approximately 5 hours and rehydrated in 2 ml buffer solution, which consisted of 20 mM NaCl, 10 mM Trizma, pH 9,5). After adhesion and fusion of the SUVs to the membraneophilic surface (waiting time 30-60 minutes usually), the surplus lipid material in the surrounding fluid was removed by exchanging the buffer solution above the structured surface several times. Fusion of vesicles was also promoted by exchanging the buffer solution with a buffer containing calcium ions and higher ionic strength to create osmotic imbalance (100 mM NaCl, 5 mM Trizma, 2 mM CaCl2, pH 8,6), and by draining the device flow cell completely of buffer at short time intervals. FRAP (fluorescence recovery after photobleaching) experiments was performed, where a portion of the patterned structures containing DiO-labeled lipid bilayer matrix was bleached using high intensity excitation light for a few minutes, in order to confirm the formation of a continuous lipid bilayer. After bleaching, the intensity of the excitation light was decreased by using filters and the fluorescence recovery was recorded by taking snapshots of the patterned lipid structure at several time-points. The recovery was fast and varied between different designs of patterns, depending on the length of the lanes and the complexity of the patterns. The example shows recovery of the fluorescence in a comb structure, consisting of four radiating arms from a single lane. The width of the lanes in this pattern is 5 µm, the length of the separating lane is 100 µm and the length of the radiating arms is 25 µm. Snapshots of the structure was taken at 30 second interval. The snapshots show diffusive lateral motion of DiO-molecules in the lipid bilayer matrix.

### 4. Integration of the lipid device with mass spectrometry

Integration of the lipid device technology with mass spectrometry allows for identification of protein associated with the lipid matrix in the device. To illustrate this, the following experiment was performed using red blood cell membrane (RBCM) protein. The cells were first washed with phosphate-buffered saline (PBS), pH 7.4. Cholesterol was extracted and removed with β-cyclodextrin (6 mM) in PBS. The cells were lysed and washed extensively with 2 mM EDTA, pH 8.3, 1 mM DTT, and protease inhibitors. The RBCM was then treated with high salt solution (1 M KCI, 1 mM DTT, pH 7.8) and high pH solution (0.1 M Na₂CO₃, 1 mM DTT, pH 11.3) to remove as much non-integral membrane protein as possible. RBCM was diluted to 0.8 mg/mL in 20 mM NaCl, Trizma 10 mM, pH 8.0 and ultrasonicated for a total duration of 15 minutes (5 second pulses followed by 5 second intervals) to reduce the membranes to vesicles. The vesicle solution was passed through 200 nm pore-size PVDF membrane filter to remove titanium particles formed from tip of the ultrasonicator. The lipid device was assembled with an Al₂O₃ covered chip (50 nm thick layer, deposited through evaporation in a thin film deposition system (AVAC HVC-600)) at the bottom, made membraneophilic through chemical and/or physical modulation or manipulation. Teflon spacers to provide the desired height of the cell (100-500 µm) and a top-lid covered with Al₂O₃, made membraneophilic through chemical and/or physical modulation or manipulation. The device was filled with RBCM vesicle suspension, and vesicles were allowed to attach to the surface for 1.5 hours. The surplus vesicles were then washed away using at least 2 mL washing buffer (20 mM NaCl, Tris 10 mM, pH 8.0). The chamber was then washed with at least 2 mL of calcium buffer (2 mM CaCl₂, 20 mM NaCl, 10 mM Tris, pH 8.0) to improve attachment of the vesicles to the surface. The calcium buffer was then washed away using the washing buffer. This procedure resulted in a layer of densely packed vesicles that were attached to all Al₂O₃ surfaces, made membraneophilic through chemical and/or physical modulation or manipulation, in the device. The chamber was filled with a solution containing trypsin (0.02 mg/ml in 20 mM NaCl, Tris 10 mM, pH 8.0) and incubated at 37° C for a specific time (3 to 5 hours). The obtained peptide solution was collected from the device in a total volume of 200-400 µL. Peptides in the digest solution were concentrated, desalted and eluted onto a MALDI-plate as follows: aliquots of digest were passed through membranes containing C₁₈-modified silicon beads. 3 membranes were used, and each received 75 µL of digest. The bound peptides were washed with 0.1 %TFA (50 µL per membrane). The peptides were eluted with 70% ACN (2 µL per membrane), and pooled onto a single spot of a MALDI-plate. The peptides in the spot were analyzed with Matrix-Assisted Laser Desorption/Ionisation-Time Of Flight (MALDI-TOF) mass spectrometry. A mass spectrum from this experiment is shown in Figure 19. Eleven unique peptides were identified using MALDI TOF in MS/MS mode. These are listed in Table 1. By comparing the sequences of these peptides with those contained in a protein database such as SWISSPROT, the origin of the peptides was found to be the integral membrane protein AE1 (anion exchanger 1, accession number P02730).

### 5. Profiling of red blood cells (erythrocyte membrane preparation).

An erythrocyte membrane preparation was used to evaluate and receive results regarding profiling studies. In brief, a similar preparation as described above, denoted as RBCM was used in the experiment. The final step of filtering the vesicle suspension was removed since the LC-MS/MS analysis proved to be sensitve for polymer contamination from the filters (in this case a 7-Tesla (Linear Trap Quadrupole- Fourier Transform) LTQ-FT mass spectrometer (Thermo Electron) equipped with a nanospray source was used). Also, a different buffer was used, in this case 10 mM Trizma, 300 mM NaCl, pH 8, was used since a high ionic strength seemed to increase the adsorption of membrane material to the surface.

In this case the chip device consisted of two glass substrates (area 68 x 109 mm, thickness 0.6-0.8 mm, Menzel Gläser), both modified with 3,5 nm chromium and 7,5 nm gold, made membraneophilic through chemical and/or physical modulation or manipulation. The two substrates were bonded together by double-sided tape (1524 medical transfer adhesive, 3M) approximately 70 µm thick, which also sets the distance between the two substrates. In one of the glass substrates, two holes - one inlet and one outlet had been drilled through the substrates. Nanoports (Upchurch Scientific) had been added on top of the holes to give capabilities to liquid inlet/outlet contacts. In this case a syringe with an adapter could be connected to the Nanoport and liquid could be forced through the chip device.

The following describes a typical digestion experiment with one enzyme in several steps, that is a sequential digest protocol.

Briefly, the chip (volume approximately 500 µl) was filled with vesicle suspension (RBC, tipsonicated) and was left to stand for 1 hour approx. This vesicle preparation was not filtered. In order to remove titanium and glass particles possibly released into the sample during the tipsonication process, the vesicle suspension was divided into 2 ml aliquots in eppendorf tubes and centrifuged for 10 minutes at 10 000 rpm.

Excess vesicles were washed away with buffer (10 mM Trizma, 20 mM NaCl, pH 8), approximately 2 ml. The adhered vesicles were subjected to calcium containing buffer, approximately 2 ml (same as above with 2 mM CaCl₂) to bind them properly to the surface. Finally, after 15 minutes, normal buffer (no calcium) was added to the chip, approx. 1,5 ml. No solutions were filtered prior use. All buffer tubes and pipette tips were rinsed with MQ prior use.

Trypsin was added to the chip (0,005 mg/ml in buffer same as above, approx. 500 µl) and the chip was incubated for 30 minutes at 37 °C. The solution was re-circulated (approx. 200 µl) before incubation.

The peptides created in this step were then eluted from the chip by adding buffer to one port and extracting the solution through the other port continuously (approx. 500 µl was extracted in concurrence to the chip volume). The extracted peptide solution was then again incubated at 37 °C overnight (18 hours) to assure complete digestion by the trypsin. The sample was finally frozen directly.

Trypsin was again added to the chip (0,005 mg/ml in the same buffer) and the chip was incubated for 2 hours at 37°C.

The peptides created in this step were then eluted from the chip by adding buffer to one port and extracting the solution through the other port continuously (approx. 500 µl was extracted in concurrence to the chip volume). The extracted peptide solution was then again incubated at 37°C overnight (16 hours) to assure complete digestion by the trypsin. The sample was finally frozen directly.

Trypsin was again added to the chip (0,005 mg/ml in the same buffer) and the chip was incubated overnight (16 hours) at 37 °C.

The created peptides were then analyzed using a nanoflow LC-MS/MS system. Specifically, for the liquid chromatography an Agilent 1100 binary pump was used, together with a reversed phase column, 200 x 0.055 mm, packed in-house with 3 µm particles Reprosil-Pur C₁₈-AQ (Dr. Maisch, Ammerbuch, Germany). The flow through the column was reduced by a split to approximately 100 nl/min. A 40 min gradient 10-50% CH₃CN in 0.2% HCOOH was used for separation of the peptides.

The nanoflow LC-MS/MS were done on a 7-Tesla (Linear Trap Quadrupole- Fourier Transform) LTQ-FT mass spectrometer (Thermo Electron) equipped with a nanospray source modified in-house. The spectrometer was operated in data-dependent mode, automatically switching to MS/MS mode. MS-spectra were acquired in the FTICR, while MS/MS-spectra were acquired in the LTQ-trap. For each scan of FTICR, the six most intense, doubly or triply charged, ions were sequentially fragmented in the linear trap by collision induced dissociation. All the tandem mass spectra were searched by MASCOT (Matrix Science, London) against the HUMAN database.

A number of various protocols were applied in order to optimize and complement the data regarding the identification of the membrane proteins present in the membrane preparation.

So far, when compiling the data, 310 proteins have been detected and identified in all the chip and analysis runs for the erythrocyte membrane preparation. This identification is based on 1496 unique peptide sequences found during the MS/MS data search. If more than 2 peptides are required for identification, 104 proteins can be assigned in the preparation. If proteins detected and identified based on a single peptide (with expect value < 0,05) is added to the list, an extra 147 proteins can be assigned. Finally, if proteins identified based on 1 peptide with expect value higher than 0,05 is included an additional 59 proteins can be added to the list. In a more detailed view and comparing the results to literature data approximately 70% of the membrane proteins described in the literature have been identified during this study.

### 6. Sequence coverage studies of anion exchange protein, band 3 in the RBCM preparation (erythrocyte).

Sequence coverage studies aims at detecting as many as possible of the amino acids in a particular protein. A high sequence coverage means that peptides are created from most parts of the protein. A high sequence coverage can be obtained by using various proteases either in parallel or sequentially to cleave the target protein at many sites and produce a large number of peptides suitable for the LC-MS/MS analysis.

There are several alternate ways in constructing protocols for sequence coverage studies. For example, so called limited proteolysis can be applied where non-specific enzymes are used to digest a sample without going to completion. Non-specific enzymes have many potential sites for cleavage and if digestion approach completion, comparatively short fragments will be produced. A limited digest can on the other hand lead to larger peptide fragments by digesting the sample using low enzyme concentrations and/or short digestion times. Such larger peptide fragments may overlap in sequence and thereby facilitate higher sequence coverage. Another way is to use more specific enzymes and use several of them either in parallel or sequentially. Through this, in the same manner as with the non-specific proteases, the protein is cut in different ways that in some cases may produce overlapping sequences. A combination of specific and non-specific proteases are of course also possible in this aspect.

The following paragraph describes a protocol with the aim for sequence coverage studies. In short it is a sequential digest using different protocols and proteases.

Again, in this case the chip device consisted of two glass substrates modified with 3,5 nm chromium and 7,5 nm gold, made membraneophilic through chemical and/or physical modulation or manipulation. The two substrates were bonded together by double-sided tape (1524 medical transfer adhesive, 3M, approximately 70 µm thick), which also sets the distance between the two substrates, In one of the glass substrates, two holes - one inlet and one outlet had been drilled. Nanoports (Upchurch Scientific) had been added on top of the holes to give capabilities to liquid inlet outlet contacts. In this case a syringe with an adapter could be connected to the Nanoport and liquid could be forced through the chip device.

Briefly, the chip (volume approximately 500 µl) was filled with vesicle suspension (RBC, tipsonicated) and was left to stand for 1 hour approx. This vesicle preparation was not filtered. In order to remove titanium and glass particles created during the tipsonication process, the vesicle suspension was divided into 2 ml aliquots in eppendorf tubes and centrifuged for 10 minutes at 10 000 rpm.

Excess vesicles were washed away with buffer (10 mM Trizma, 20 mM NaCl, pH 8), approximately 2 ml. The adhered vesicles were subjected to calcium containing buffer, approximately 2 ml (same as above with 2 mM CaCl₂) to bind them properly to the surface. Finally, after 15 minutes, normal buffer (no calcium) was added to the chip, approx. 1,5 ml. NOTE: No solutions were filtered prior use. All buffer tubes and pipette tips were rinsed with MQ prior use.

Trypsin was added to the chip (0,005 mg/ml in the Trizma buffer same as above, approx. 500 µl) and the chip was incubated for 2 hours at 37°C. The solution was recirculated (approx. 200 µl) before incubation.

The peptides created in this step were then eluted from the chip by adding buffer to one port and extracting the solution through the other port continuously (approx. 500 µl was extracted). The extracted peptide solution was then again incubated at 37°C overnight (20 hours) to assure complete digestion by the trypsin. The sample was finally frozen directly without acidifying.

Pepsin protocol was followed to perform a second digestion step. Briefly, pepsin (0,001 mg/ml) dissolved in MQ water with 14% formic acid, and 5% acetonitrile (just prior experiments) was added to the chip (approx. 500 µl added) and recirculated briefly by adding and extracting the solution through the chip prior incubation at 37 °C overnight. The peptides were extracted from the chip by suction. Before freezing, the pepsin was inactivated by the addition of 300 µl acetonitrile to approx. 300 µl sample.

In summary, the results from this analysis showed that the first trypsin step resulted in 30% sequence coverage of the band 3 anion exchange protein. The following pepsin digest step resulted in 67% sequence coverage. Together, the sequence coverage was calculated to be 69% approximately. This shows a clear overlap of the peptide sequences found in the pepsin step when compared to the trypsin fragments. However, it seems that the pepsin digest step is promoted by having a first digestion step with trypsin prior the pepsin step.

The figure below shows the added results from sequence coverage studies of band 3 anion exchange protein. The first panel shows the fragments obtained by digestion with trypsin. The second panel shows the total results when adding the fragments found by pepsin digest.

## Claims

1. A method for identification of a substance, which substance is included in or bound to, connected to or associated with a covering layer in a device, said device comprising:
at least one supporting solid surface comprising at least one membraneophilic region;
a covering layer that is at least partially adhered to the membraneophilic region, said covering layer consisting of (i) a surfactant membrane, (ii) a lipid mimicking polymer, (iii) a surfactant or emulsion system or (iv) a liquid crystal; and
a substance included in or bound to, connected to or associated with the covering layer,
said method comprising a first step (i) wherein the device is contacted with at least one digestive agent or cleavage agent, which carries out controlled cleaving of parts of the substance, after which the substance consists of a substantially mobile fraction and an immobile fraction, which is still included in or bound to, connected to or associated with the covering layer; and a second step (ii) wherein the substantially mobile fraction is detected or analyzed.

2. The method according to claim 1, wherein the digestive agent is an enzyme or a mixture of different digestive or cleavage agents.

3. The method according to claim 1 or 2, wherein different digestive or cleavage agents are used sequentially, or in parallel.

4. The method according to any one of the claims 1-3, further comprising an additional step wherein the device is contacted with at least one disruptive agent, which disrupt the structure of the covering layer carrying the remaining immobile fraction of the substance included in or bound to, connected to or associated with the covering layer, after which it is comprised of its basic elements, which are substantially mobile; and another step wherein the remaining fraction of the substance included in or bound to, connected to or associated with the covering layer, which is released from the covering layer, is detected or analyzed.

5. The method according to claim 4, wherein the disruptive agent is a detergent or organic solvent.

6. The method according to any one of the claims 1-5, wherein one of or both the steps of detection or analysis (ii and/or iv) is/are preceded by a separation step.

7. The method according to claim 6, wherein the separation is selected from Capillary Electrophoresis (CE) Liquid Chromatography (LC), gel-chromatography and gel-electrophoresis.

8. The method according to any one of the claims 1-7, wherein the detection or analysis is carried out by mass spectrometry, for example, MALDI MS, MALDI-TOF and MALDI-TOF-TOF, Electrospray Ionization (ESI MS-MS).

9. The method according to any one of the claims 1-7, wherein the detection or analysis is carried out by surface plasmon resonance (SPR), quartz crystal microbalance (QCM) or a fluorescence-based technique.

10. The method according to claim 1, wherein the device further comprises an aqueous layer trapped between the supporting solid surface and the covering layer.

11. The method according to claim 1, wherein the device further comprises an aqueous solution covering the covering layer.

12. The method according to claims 1, wherein the device further comprises a metal film on the supporting solid surface.

13. The method according to any one of the claims 1-9, wherein the membraneophilic region is a surface made of silicon dioxide, glass, metal oxides, for example, aluminum oxide, metal, for example, gold, Mica or a polymer, or any combinations thereof.

14. The method according to any one of the claims 1-13, wherein the covering layer is constituted by a surfactant membrane.

15. The method according to claim 14, wherein the surfactant membrane is selected from the group consisting of lipid bilayer membranes, cell membranes, intact cells, lipid vesicles, proteovesicles, lipid nanotubes, lipid monolayers, cell fragments, organelles and any combinations thereof.

16. The method according to any one of the claims 1-15, wherein the substance included in or bound to, connected to or associated with the covering layer is selected from the group consisting of peripheral and integral membrane proteins, glycosylphosphatidylinositol(GPI)-anchored proteins, phospholipids, sphingolipids, drugs, amphiphilic agents, lipophilic agents, sterols, sugars, oligonucleotides, polymers and DNA.

17. The method according to any one of the claims 1-15, wherein the device further comprises at least one inlet and at least one outlet connection for creation of a flow of the liquid or the aqueous solution covering the covering layer, said flow making an exchange of additional membrane, protein-lipid mixtures, washing solutions, staining solutions, blocking solutions, digestive solutions and other solutions or suspensions around the covering layer.

## Patentansprüche

1. Ein Verfahren zum Identifizieren einer Substanz, wobei die Substanz in einer Deckschicht eingeschlossen oder an diese gebunden, mit dieser verbunden oder mit dieser assoziiert in einer Vorrichtung vorliegt, wobei diese Vorrichtung umfasst:
wenigstens eine feste Trägeroberfläche, umfassend wenigstens eine membranophile Region,
eine Deckschicht, die wenigstens teilweise an der membranophilen Region anhaftet, wobei diese Deckschicht aus (i) einer Membran einer oberflächenaktiven Verbindung, (ii) einem ein Lipid nachahmenden Polymer, (iii) einem System einer oberflächenaktiven Verbindung oder einem Emulsionssystem, oder (iv) einem Flüssigkristall besteht; und
eine Substanz, die in der Deckschicht eingeschlossen, an diese gebunden, mit dieser verbunden oder mit dieser assoziiert vorliegt,
wobei das Verfahren einen ersten Schritt (i), worin die Vorrichtung mit wenigstens einem verdauungsaktiven Mittel oder Spaltungsmittel in Kontakt gebracht wird, das eine kontrollierte Abspaltung von Teilen der Substanz durchführt, wonach die Substanz aus einer im Wesentlichen mobilen Fraktion und einer immobilen Fraktion besteht, die immer noch in der Deckschicht eingeschlossen, an diese gebunden, mit dieser verbunden oder mit dieser assoziiert vorliegt; und einen zweiten Schritt (ii), in welchem die im Wesentlichen mobile Fraktion nachgewiesen oder analysiert wird, umfasst.

2. Das Verfahren gemäß Anspruch 1, wobei das verdauungsaktive Mittel ein Enzym ist, oder eine Mischung von verschiedenen verdauungsaktiven Mitteln oder Spaltungsmitteln.

3. Das Verfahren gemäß Anspruch 1 oder 2, wobei verschiedene verdauungsaktive Mittel oder Spaltungsmittel aufeinanderfolgend oder gleichzeitig verwendet werden.

4. Das Verfahren gemäß einem der Ansprüche 1-3, außerdem umfassend einen zusätzlichen Schritt, in welchem die Vorrichtung mit wenigstens einem zerstörenden Mittel in Kontakt gebracht wird, welches die Struktur der Deckschicht, die die verbliebene immobile Fraktion der Substanz in der Deckschicht eingeschlossen, an diese gebunden, mit dieser verbunden oder mit dieser assoziiert, trägt, zerstört, wonach sie aus ihren Grundbestandteilen besteht, die im Wesentlichen mobil sind; und einen weiteren Schritt, in dem die verbliebene Fraktion der Substanz, die in die Deckschicht eingeschlossen oder an diese gebunden, mit dieser verbunden oder mit dieser assoziiert vorliegt, die aus der Deckschicht freigesetzt wird, nachgewiesen oder analysiert wird.

5. Das Verfahren gemäß Anspruch 4, wobei das Zerstörungsmittel ein Detergens oder ein organisches Lösungsmittel ist.

6. Das Verfahren gemäß einem der Ansprüche 1-5, wobei einem oder beiden der Schritte zur Detektion oder Analyse (ii und/oder iv) ein Trennungsschritt vorausgeht.

7. Das Verfahren gemäß Anspruch 6, wobei die Trennung ausgewählt ist aus Kapillarelektrophorese (CE), Flüssigchromatographie (LC), Gelchromotographie und Gelelektrophorese.

8. Das Verfahren gemäß einem der Ansprüche 1-7, wobei der Nachweis oder die Analyse mittels Massenspektrometrie, zum Beispiel MALDI MS, MALDI-TOF und MALDI-TOF-TOF, oder Elektrospray-lonisierung (ESI MS-MS) durchgeführt wird.

9. Das Verfahren gemäß einem der Ansprüche 1-7, wobei der Nachweis oder die Analyse mittels Oberflächenplasmonresonanz (SPR), Quartzkristall-Mikrowaage (QCM) oder einer Technik auf Fluoreszenz-Basis durchgeführt wird.

10. Das Verfahren gemäß Anspruch 1, wobei die Vorrichtung außerdem eine wässrige Schicht umfasst, die zwischen der festen Trägeroberfläche und der Deckschicht eingeschlossen ist.

11. Das Verfahren gemäß Anspruch 1, wobei die Vorrichtung außerdem eine wässrige Lösung umfasst, die die Deckschicht bedeckt.

12. Das Verfahren gemäß Anspruch 1, wobei die Vorrichtung außerdem eine Metallfolie auf der festen Trägeroberfläche umfasst.

13. Das Verfahren gemäß einem der Ansprüche 1-9, wobei die membranophile Region eine Oberfläche darstellt, die aus Siliciumdioxid, Glas, Metalloxiden, zum Beispiel Aluminiumoxid, Metall, zum Beispiel Gold, Glimmer oder einem Polymer, oder einer beliebigen Kombination derselben hergestellt ist.

14. Das Verfahren gemäß einem der Ansprüche 1-13, wobei die Deckschicht aus einer Membran einer oberflächenaktiven Verbindung gebildet wird.

15. Das Verfahren gemäß Anspruch 14, wobei die Membran aus der oberflächenaktiven Verbindung aus der Gruppe, bestehend aus Lipid-Doppelschichtmembranen, Zellmembranen, intakten Zellen, Lipidvesikeln, Proteovesikeln, Lipid-Nanoröhrchen, Lipid-Monoschichten, Zellfragmenten, Organellen und einer beliebigen Kombination derselben ausgewählt ist.

16. Das Verfahren gemäß einem der Ansprüche 1-15, wobei die Substanz, die in der Deckschicht eingeschlossen oder an diese gebunden, mit dieser verbunden oder mit dieser assoziiert vorliegt, aus der Gruppe, bestehend aus peripheren und integralen Membranproteinen, Glykosylphosphatidyl-inositol(GPI)-verankerten Proteinen, Phospholipiden, Sphingolipiden, Medikamenten, amphiphilen Mitteln, lipophilen Mitteln, Sterolen, Zuckern, Oligonukleotiden, Polymeren und DNA ausgewählt ist.

17. Das Verfahren gemäß einen der Ansprüche 1-15, wobei die Vorrichtung außerdem wenigstens eine Einlass- und wenigstens eine Auslassverbindung zur Ausbildung eines Durchflusses der Flüssigkeit oder der wässrigen Lösung, die die Deckschicht bedeckt, umfasst, wobei dieser Durchfluss einen Austausch zusätzlicher Membran, von Protein-Lipid-Mischungen, Waschlösungen, Färbelösungen, Blockierungslösungen, verdauungsaktiven Lösungen oder anderen Lösungen oder Suspensionen um die Deckschicht herum bewirkt.

## Revendications

1. Procédé d'identification d'une substance, laquelle substance est incluse dans ou liée à, raccordée à ou associée à une couche de recouvrement dans un dispositif, ledit dispositif comprenant :
au moins une surface solide de support comprenant au moins une région membranéophile ;
une couche de recouvrement qui adhère au moins partiellement à la région membranéophile, ladite couche de recouvrement consistant en (i) une membrane de tensioactif, (ii) un polymère imitant un lipide, (iii) un système de tensioactif ou d'émulsion ou (iv) un cristal liquide ; et
une substance incluse dans ou liée à, raccordée à ou associée à la couche de recouvrement,
ledit procédé comprenant une première étape (i) dans laquelle le dispositif est mis en contact avec au moins un agent de digestion ou un agent de clivage, qui réalise un clivage contrôlé de parties de la substance, après quoi la substance consiste en une fraction sensiblement mobile et une fraction immobile, qui est toujours incluse dans ou liée à, raccordée à ou associée à la couche de recouvrement ; et une seconde étape (ii) dans laquelle la fraction sensiblement mobile est détectée ou analysée.

2. Procédé selon la revendication 1, dans lequel l'agent de digestion est une enzyme ou un mélange de différents agents de digestion ou de clivage.

3. Procédé selon la revendication 1 ou 2, dans lequel différents agents de digestion ou de clivage sont utilisés séquentiellement, ou en parallèle.

4. Procédé selon l'une quelconque des revendications 1 à 3, comprenant en outre une étape supplémentaire dans laquelle le dispositif est mis en contact avec au moins un agent perturbateur, qui perturbe la structure de la couche de recouvrement portant la fraction immobile restante de la substance incluse dans ou liée à, raccordée à ou associée à la couche de recouvrement, après quoi elle est composée de ses éléments de base, qui sont sensiblement mobiles ; et une autre étape dans laquelle la fraction restante de la substance incluse dans ou liée à, raccordée à ou associée à la couche de recouvrement, qui est libérée à partir de la couche de recouvrement, est détectée ou analysée.

5. Procédé selon la revendication 4, dans lequel l'agent perturbateur est un détergent ou un solvant organique.

6. Procédé selon l'une quelconque des revendications 1 à 5, dans lequel l'une des étapes de détection ou d'analyse (ii et/ou iv) ou les deux est/sont précédée(s) par une étape de séparation.

7. Procédé selon la revendication 6, dans lequel la séparation est choisie parmi l'électrophorèse capillaire (EC), la chromatographie liquide (CL), la chromatographie sur gel et l'électrophorèse sur gel.

8. Procédé selon l'une quelconque des revendications 1 à 7, dans lequel la détection ou l'analyse est réalisée au moyen de la spectrométrie de masse, par exemple, par MALDI MS, MALDI-TOF et MALDI-TOF-TOF, ionisation par électropulvérisation (ESI MS-MS).

9. Procédé selon l'une quelconque des revendications 1 à 7, dans lequel la détection ou l'analyse est réalisée par résonance plasmonique de surface (SPR), microbalance à cristal de quartz (QCM) ou une technique basée sur la fluorescence.

10. Procédé selon la revendication 1, dans lequel le dispositif comprend en outre une couche aqueuse piégée entre la surface solide de support et la couche de recouvrement.

11. Procédé selon la revendication 1, dans lequel le dispositif comprend en outre une solution aqueuse couvrant la couche de recouvrement.

12. Procédé selon la revendication 1, dans lequel le dispositif comprend en outre un film métallique sur la surface solide de support.

13. Procédé selon l'une quelconque des revendications 1 à 9, dans lequel la région membranéophile est une surface constituée de dioxyde de silicium, de verre, d'oxydes métalliques, par exemple d'oxyde d'aluminium, de métal, par exemple d'or, de mica ou d'un polymère, ou de l'une quelconque de leurs combinaisons.

14. Procédé selon l'une quelconque des revendications 1 à 13, dans lequel la couche de recouvrement est constituée d'une membrane de tensioactif.

15. Procédé selon la revendication 14, dans lequel la membrane de tensioactif est choisie dans le groupe constitué par des membranes bicouches lipidiques, des membranes cellulaires, des cellules intactes, des vésicules lipidiques, des protéovésicules, des nanotubes lipidiques, des monocouches lipidiques, des fragments cellulaires, des organelles et l'une quelconque de leurs combinaisons.

16. Procédé selon l'une quelconque des revendications 1 à 15, dans lequel la substance incluse dans ou liée à, raccordée à ou associée à la couche de recouvrement est choisie dans le groupe constitué par des protéines membranaires périphériques et intégrales, des protéines à ancrage glycosylphosphatidylinositol (GPI), des phospholipides, des sphingolipides, des médicaments, des agents amphiphiles, des agents lipophiles, des stérols, des sucres, des oligonucléotides, des polymères et de l'ADN.

17. Procédé selon l'une quelconque des revendications 1 à 15, dans lequel le dispositif comprend en outre au moins une admission et au moins un raccordement de refoulement permettant de créer un écoulement de liquide ou de la solution aqueuse couvrant la couche de recouvrement, ledit écoulement réalisant un échange de membrane supplémentaire, de mélanges de protéine-lipide, de solutions de lavage, de solutions de coloration, de solutions de blocage, de solutions digestives et d'autres solutions ou suspensions autour de la couche de recouvrement.
